(19) Europäisches Patentamt / European Patent Office / Office européen des brevets

(11) **EP 2 386 062 B1**

(12) **EUROPEAN PATENT SPECIFICATION**

(45) Date of publication and mention
of the grant of the patent:
**06.01.2016 Bulletin 2016/01**

(51) Int Cl.:
***G01N 33/574*** (2006.01)

(21) Application number: **10700589.4**

(86) International application number:
**PCT/GB2010/000030**

(22) Date of filing: **08.01.2010**

(87) International publication number:
**WO 2010/079339 (15.07.2010 Gazette 2010/28)**

(54) **FACTORS**

FAKTOREN

FACTEURS

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **09.01.2009 US 143640 P**

(43) Date of publication of application:
**16.11.2011 Bulletin 2011/46**

(73) Proprietor: **Oxford Biomedica (UK) Ltd**
**The Oxford Science Park**
**Oxford OX4 4GA (GB)**

(72) Inventor: **HARROP, Richard**
**Oxford OX4 4GA (GB)**

(74) Representative: **D Young & Co LLP**
**120 Holborn**
**London EC1N 2DY (GB)**

(56) References cited:
- PADRIK PEETER: "Prognostic factors of immunotherapy in metastatic renal cell carcinoma." MEDICAL ONCOLOGY (TOTOWA), vol. 20, no. 4, 2003, pages 325-334, XP002574216 ISSN: 1357-0560
- RICHARD HARROP ET AL: "Vaccination of colorectal cancer patients with TroVax given alongside chemotherapy (5-fluorouracil, leukovorin and irinotecan) is safe and induces potent immune responses" CANCER IMMUNOLOGY, IMMUNOTHERAPY, SPRINGER, BERLIN, DE, vol. 57, no. 7, 30 November 2007 (2007-11-30), pages 977-986, XP019624357 ISSN: 1432-0851
- Anonymous: "Oxford biomedica presents interim results from phase III trist study of trovax in renal cancer"[Online] 22 September 2009 (2009-09-22), - 22 September 2009 (2009-09-22) XP002574217 RNS News Service (Number: 4181Z) Retrieved from the Internet: URL:http://online.hemscottir.com/ir/oxb/ir.jsp?page=news-item&item=263210628287203> [retrieved on 2009-03-17]
- AMATO ROBERT J ET AL: "Vaccination of prostate cancer patients with modified vaccinia ankara delivering the tumor antigen 5T4 (TroVax): a phase 2 trial" JOURNAL OF IMMUNOTHERAPY, LIPPINCOTT WILLIAMS & WILKINS, HAGERSTOWN, MD, US, vol. 31, no. 6, 1 January 2008 (2008-01-01), pages 577-585, XP009123376 ISSN: 1524-9557
- ELKORD EYAD ET AL: "An MVA-based vaccine targeting the oncofetal antigen 5T4 in patients undergoing surgical resection of colorectal cancer liver metastases" JOURNAL OF IMMUNOTHERAPY,, vol. 31, no. 9, 1 November 2008 (2008-11-01), pages 820-829, XP009123377
- HARROP RICHARD ET AL: "Vaccination of colorectal cancer patients with modified vaccinia Ankara delivering the tumor antigen 5T4 (TroVax) induces immune responses which correlate with disease control: a phase I/II trial" CLINICAL CANCER RESEARCH, THE AMERICAN ASSOCIATION FOR CANCER RESEARCH, US, vol. 12, no. 11 Pt 1, 1 June 2006 (2006-06-01), pages 3416-3424, XP002548290 ISSN: 1078-0432

- **TYKODI SCOTT S ET AL: "Development of modified vaccinia Ankara-5T4 as specific immunotherapy for advanced human cancer." EXPERT OPINION ON BIOLOGICAL THERAPY DEC 2008, vol. 8, no. 12, December 2008 (2008-12), pages 1947-1953, XP002574218 ISSN: 1744-7682**

## Description

### Field of the Invention

The present invention relates to a method of cancer therapy which employs a factor or a set of factors to predict whether a patient will benefit from treatment with an immunotherapeutic agent.

In particular, the method predicts the clinical benefit to a potential patient of an MVA vector expressing a human 5T4 gene, such as Trovax®. More particularly, the method relates to those patients with renal, colorectal or prostate cancer.

### Background to the Invention

Tumor cells are notoriously poor immunogens despite the fact that many antigens that are over-expressed or unique to tumor cells (tumor-associated antigens) have been identified. The reasons for this apparent lack of immunogenicity may be that cancer antigens are generally not presented to the immune system in a micro-environment that favors the activation of immune cells which would lead to the killing of the tumor cells. Although no single known mechanism can explain poor tumor immunogenicity in all experimental models studied, the molecular basis can be separated conceptually into distinct groupings: i) lack of expression of co-stimulatory molecules essential for effective immune induction, ii) production of immuno-inhibitory substances and iii) variability in the expression of antigen by tumors.

Much progress has been made in the identification of tumor-associated antigens (TAA) that are potentially useful in the development of recombinant anti-cancer vaccines. TAAs can be divided into three major categories: i) non-self viral antigens e.g. E6/E7 from human papilloma virus (HPV), ii) altered self-antigens e.g. MUC-1 and iii) non-mutated self-antigens e.g. 5T4 and carcinoembryonic antigen (CEA).

Vaccinia virus (VV), a member of the poxvirus family, has been developed as a recombinant expression vector for the genetic delivery of antigens. Animals injected with a recombinant VV (rVV) have been shown to produce both antibody and CTL responses to the exogenous proteins. In contrast to tumor cells VV infection appears to create an optimal environment for the induction of an efficacious immune response Recombinant VV expressing murine homologues of TAA, which, in murine models, are classed as self-antigens, have also been shown to induce TAA specific immune responses in murine models, illustrating that such constructs are potentially able to overcome immune tolerance to self-antigens. In vivo models demonstrate that the immune responses generated are able to prevent tumor establishment and in some cases are able to actively treat established tumors. These data also indicate that it is possible to turn an anti-viral response into an anti-cancer response by presenting a TAA in the context of viral antigens.

Recombinant VV vectors expressing the self-antigen CEA have been constructed and have been evaluated for toxicity and to a lesser extent efficacy in late stage colorectal cancer. Such rVV vectors were well tolerated and both antibody and cell mediated immune responses to the self-antigen CEA were reported. Lack of tumor response data in these trials may be due to the patient population which had very advanced tumors and had already failed prior chemotherapy. To date over 700 people have been vaccinated with rVV and other poxviruses expressing TAAs in a spectrum of cancer immunotherapy clinical trials. There have been no reports of toxicity either from the virus itself or as a result of the immune response induced to the TAA beyond local injection site reactions and transient pyrexia.

Suitable methods and suitable clinical markers, however, that can guide such immunotherapeutic methods would be extremely beneficial.

Renal cell carcinoma (RCC) has been reported to be the tenth most common cancer in the US and studies suggest a continued rise in RCC incidence. Although most patients with early stage RCC can be cured surgically, approximately 33% of patients present with metastatic disease for which the treatment is usually not curative. In addition, approximately 50% of patients who undergo potentially curative surgery for less advanced disease can be expected to develop a recurrence with distant metastases. Five-year survival for patients with de novo metastatic or recurrent disease ranges between 0% and 20%.

Clinical factors associated with patients with metastatic RCC when they are treated with cytokines (interferon, and interleukin), chemotherapy or a variety of historic therapies have been reported to include tumour-, patient-, and disease-related factors, such as performance status (PS), time from diagnosis to therapy, number of metastatic sites, visceral metastasis, haemoglobin, calcium, lactate dehydrogenase, inflammation markers, and others.

Choueriri et al, Cancer (2007), 110(3): 543-550 reviewed the records of patients with metastatic renal cell carcinoma (RCC) who were treated with anti-VEGF agents - bevacizumab, sunitinib, sorafenib and axitinib - with a view to identifying patients who are more likely to benefit from these agents. The article reports that although many factors were associated individually with progression-free survival (PFS) on univariate analysis, only 5 factors were identified as independent predictors of a poor outcome on subsequent multivariate analysis. With the least favourable feature listed first, the following factors were identified: initial Eastern Cooperative Oncology Group performance status (ECOG PS) ≥1 vs 0, time from diagnosis to current treatment <2 years vs ≥2 years, abnormal baseline corrected serum calcium <8.5 mg/dL or >10 mg/dL vs 8.5-10 mg/dL, high platelet count >300 K/μL vs ≤ 300 K/μL, and higher absolute neutrophil count (ANC) >4.5 K/μL vs ≤4.5 K/μL.

Choueriri et al however only teaches that these factors were associated with PFS for patients with metastatic RCC who received four specific VEGF-targeted therapies. It does not teach a skilled worker what factors may or may not be important for other therapies and other cancers. It is unclear whether the same factors reported previously are relevant to patients who are treated with, for example, immunotherapies.

Colorectal carcinoma (CRC) is one of the most common cancers in Western societies, being second only to lung cancer as a cause of death from malignancy. It is the second most common cancer in England and Wales. Approximately 24,000 men and women develop the disease each year, and over half of these die from it

Fusek et al, World J Gastroenterol (2004), 10(13): 1890-1892 aimed to examine the calcium metabolism in patients with CRC and control patients. Seventy newly diagnosed CRC patients were included. The healthy control group was age and gender matched. They conclude that their results further strengthen the possibility that serum calcium might be a pathogenic and prognostic factor in the development of CRC. They say that their data draw attention to the possibility that by increasing calcium intake, the multi-levelled pathogenic process leading to tumorigenesis might be influenced. They go on to state that in order to prove this, further studies are necessary.

Fusek et al, however, does not indicate whether or not serum calcium might be a pathogenic and prognostic factor for any of the drug therapies used to treat CRC and other cancers.

Padrik *et al.* (Padrik, P. et al. (2003) Med. Oncol. 20: 325-34) relates to prognostic factors of immunotherapy in metastatic renal cell carcinoma.

Thus, there remains a need for suitable methods and suitable clinical markers that can guide immunotherapeutic methods.

## Summary

The invention provides materials and methods that address one or more needs in the fields of cancer therapy, immunotherapy, or related fields.

Some aspects of the invention relate to materials and methods for monitoring or determining the efficacy of an immunotherapy. Improved monitoring permits improvement of therapy for individual subjects; and more rapid determination of which subjects benefit from the therapy. Subjects obtaining less benefit can more quickly be given modifed or different therapeutic regimens.

## Statements of the Invention

In one aspect, the present invention provides a method for determining a prognosis for benefit for a cancer patient receiving immunotherapy treatment, wherein the immunotherapy treatment comprises use of 5T4, involving (a) measuring a level of platelets and haemoglobin in a sample from the cancer patient, and (b) comparing the level of platelets in the sample to a reference level of platelets and comparing the level of haemoglobin in the sample to a reference level of haemoglobin, wherein a lower level of platelets and higher level of haemoglobin in the sample correlates with increased benefit to the patient.

In another aspect, the present invention provides a method for determining a prognosis for benefit for a cancer patient receiving immunotherapy treatment wherein the immunotherapy treatment comprises use of 5T4, involving (a) measuring a level of platelets and haemoglobin in a sample from the cancer patient, and (b) classifying the patient as belonging to either a first or second group of patients, wherein the first group of patients having lower levels of platelets than a reference level of platelets and higher levels of haemoglobin than a reference level of haemoglobin is classified as having an increased likelihood of benefit than the second group of patients having higher levels of platelets and lower levels of haemoglobin.

In another aspect, the present invention provides a method of predicting the responsiveness of a patient or patient population with cancer to treatment with immunotherapy, or for selecting patients or patient populations that will respond to immunotherapy, comprising comparing the differential levels of platelets and differential levels of haemoglobin and wherein the immunotherapy treatment comprises use of 5T4.

## Baseline Platelet Counts

We have identified that immunotherapy performs better in cancer patients with a lower baseline platelet count.

Thus, we describe herein a method for determining a prognosis for benefit for a cancer patient receiving immunotherapy treatment involving (a) measuring a level of platelets in a sample from the cancer patient, and (b) comparing the level of platelets in the sample to a reference level of platelets, wherein a lower level of platelets in the sample correlates with increased benefit to the patient.

By "lower level" we include patients who have a level or count of baseline platelets below the median for a patient in need of immunotherapy or below or towards the bottom end of normal levels.

A further method for determining a prognosis for benefit for a cancer patient receiving immunotherapy treatment involves (a) measuring a level of platelets in a sample from the cancer patient, and (b) classifying the patient as belonging to either a first or second group of patients, wherein the first group of patients having low levels of platelets is classified as having an increased likelihood of benefit than the second group of patients having high levels of platelets.

We also describe a method for monitoring the effectiveness of a course of treatment for a patient with cancer. The method involves (a) determining a level of platelets in a sample from the cancer patient prior to immunotherapy treatment, and (b) determining the level of platelets in a sample from the patient after treatment, whereby comparison of the platelet level prior to treatment with the platelet level after treatment indicates the effectiveness of the treatment.

By "receiving immunotherapy" we also include patients who are being assessed for immunotherapy.

In other words we describe a method of predicting the responsiveness of a patient or patient population with cancer to treatment with immunotherapy, or for selecting patients or patient populations that may respond to immunotherapy, comprising comparing the differential levels of platelets wherein a platelet level below a reference level is associated with benefit.

By "lower level" we include a patient or patient population who have a level of platelets either below a reference level for a patient or patient population who have been diagnosed with cancer and are therefore need of treatment, such as immunotherapy; or below a reference level for a normal individual or population. By "reference level" we include a level which represents a level below which the administration of immunotherapy will confer a clinical benefit to the patient or patient population, such as improved overall survival, increased progression-free survival, decreased risk of tumour recurrence or spread.

In one embodiment the lower level will include a patient or patient population who have a level of platelets below the median for a patient or patient population who have been diagnosed with cancer and are therefore in need of treatment, such as immunotherapy, it will also include a patient or patient population who have a level of platelets which is below the median for a normal individual or patient population.

In one embodiment, the platelet level or count associated with a more favourable outcome is about $\leq 400 \times 10^9$ /L, about $\leq 350 \times 10^9$ /L, or about $\leq 300 \times 10^9$ /L. More particularly, the platelet level associated with a more favourable outcome is about $\leq 287 \times 10^9$ /L. In a preferred embodiment the baseline platelet count is about $\leq 281 \times 10^9$ /L, $\leq 281.5 \times 10^9$ /L, about $\leq 275.5 \times 10^9$ /L, about $\leq 273 \times 10^9$ /L, about $\leq 250 \times 10^9$ /L, $\leq 232 \times 10^9$ /L, about $\leq 225 \times 10^9$ /L, or about $\leq 215 \times 10^9$ /L. Especially preferred are platelet levels of $\leq 287 \times 10^9$ /L, $\leq 281 \times 10^9$ /L, or even more preferred $\leq 232 \times 10^9$ /L. These levels may be particularly associated with patients with RCC or CRC.

In one embodiment, the platelet level associated with a more favourable outcome in RCC is about $\leq 400 \times 10^9$ /L, about $\leq 350 \times 10^9$ /L, or about $\leq 300 \times 10^9$ /L. More particularly, the platelet level associated with a more favourable outcome in RCC is about $\leq 287 \times 10^8$ /L. In a preferred embodiment the baseline platelet count in RCC is about $\leq 281 \times 10^9$ /L, $\leq 281.5 \times 10^9$ /L, about $\leq 275.5 \times 10^9$ /L, about $\leq 273 \times 10^9$ /L, about $\leq 250 \times 10^9$ /L, $\leq 232 \times 10^9$ /L, about $\leq 225 \times 10^9$ /L, or about $\leq 215 \times 10^9$ /L. Especially preferred are platelet levels associated with RCC of $\leq 287 \times 10^9$ /L, $\leq 281 \times 10^9$ /L, or even more preferred $\leq 232 \times 10^9$ /L.

The above referenced platelet levels are reported in connection with a patient population diagnosed with RCC in which the platelet levels ranged from 114 to $1074 \times 10^9$ /L and the median platelet level is $281 \times 10^9$/L; an RCC patient population and a CRC patient population in which the median platelet levels were $273 \times 10^9$ /L, but the levels or their equivalents in relation to other patient populations may be generally applicable to the field of immunotherapy.

A normal platelet count in a healthy person (1 + year) is between 130,000 and 400,000 per $mm^3$ (microlitre) of blood ($130-400 \times 10^9$ /L).

Haemoglobin levels

We have identified that immunotherapy performs better in cancer patients with a higher baseline haemoglobin level.

Thus, we describe herein a method for determining a prognosis for benefit for a cancer patient receiving immunotherapy treatment involving (a) measuring level of haemoglobin in a sample from the cancer patient, and (b) comparing the level of haemoglobin in the sample to a reference level of haemoglobin, wherein a higher level of haemoglobin in the sample correlates with increased benefit to the patient.

By "higher level" we include patients who have a level of baseline haemoglobin above the median for a patient in need of immunotherapy or above or towards the upper end of normal levels.

A further method for determining a prognosis for survival for a cancer patient receiving immunotherapy treatment involves (a) measuring a level of haemoglobin in a sample from the cancer patient, and (b) classifying the patient as belonging to either a first or second group of patients, wherein the first group of patients having high levels of haemoglobin is classified as having an increased likelihood of survival than the second group of patients having low levels of haemoglobin.

We also describe a method for monitoring the effectiveness of a course of treatment for a patient with cancer. The method involves (a) determining a level of a haemoglobin in a sample from the cancer patient prior to immunotherapy treatment, and (b) determining the level of haemoglobin in a sample from the patient after treatment, whereby comparison of the haemoglobin level prior to treatment with the haemoglobin level after treatment indicates the effectiveness of the treatment

By "receiving immunotherapy" we also include patients who are being assessed for immunotherapy, i.e. patients who could potentially benefit from treatment for cancer.

In other words we describe a method of predicting the responsiveness of a patient or patient population with cancer to treatment with immunotherapy, or for selecting patients or patient populations that may respond to immunotherapy comprising comparing the differential levels of haemoglobin wherein a haemoglobin level above a reference level is associated with benefit.

By "higher lever" we include a patient or patient population who have a level of haemoglobin above a reference level for a patient or patient population who have been diagnosed with cancer and are therefore in need of treatment, such as immunotherapy; or above a reference level for a normal individual or population. By "reference level" we include a level which represents a level above which the administration of immunotherapy will confer a clinical benefit to the patient or patient population, such as improved overall survival increased progression-free survival, decreased risk of tumour recurrence or spread.

In one embodiment the higher level will include a patient or patient population who have a level of haemoglobin above the median for a patient or patient population who have been diagnosed with cancer and are therefore in need of treatment, such as immunotherapy; or above the median for a normal individual or patient population.

In one embodiment, the haemoglobin level associated with a more favourable outcome is about $\geq 100$ g/L, or more preferably about $\geq 125$ g/L, or even more preferably about $\geq 130$ $\mu$mol/L or about $\geq 132$ g/L. In one even more preferred embodiment, the haemoglobin level associated with a more favourable outcome is greater than about $\geq 140$ g/L or about $\geq 145$ g/L. In an especially preferred embodiment, the haemoglobin level associated with a more favourable outcome is about $\geq 153$ g/L. These levels may be particularly associated with patients with RCC or CRC.

In one embodiment, the haemoglobin level associated with a more favourable outcome in RCC is about $\geq 100$ g/L, or more preferably about $\geq 125$ g/L, or even more preferably about $\geq 130$ $\mu$mol/L or about $\geq 132$ g/L. In one even more preferred embodiment, the haemoglobin level associated with a more favourable outcome in RCC is greater than about $\geq 140$ g/L or about $\geq 145$ g/L. In an especially preferred embodiment, the haemoglobin level associated with a more favourable outcome in RCC is about $\geq 153$ g/L. These haemoglobin levels are reported in connection with an RCC patient population in which the haemoglobin levels ranged from 65-190 g/L and a median haemoglobin level of $\geq 132$ g/L, but the levels or their equivalents may be generally applicable in relation to other patient populations to the field of immunotherapy.

The haemoglobin level in a normal population has been reported as being in the range of 118-168 g/L. Haemoglobin levels have however been reported to vary according to gender and age. Thus, the reference range of 118-168 g/L has been reported as being the normal range for males of 65+ years. For males of 18-64 years the haemoglobin level in a normal population has been reported as being in the range of 138-172 g/L For females of 18-64 years the haemoglobin level in a normal population has been reported as being in the range of 120-156 g/L. For females of 65+ years the haemoglobin level in a normal population has been reported as being in the range of 111-155 gL.

Monocyte levels

We have identified that immunotherapy performs better in cancer patients with a lower baseline monocyte level.

Thus, we describe herein a method for determining a prognosis for benefit for a cancer patient receiving immunotherapy treatment involving (a) measuring a level of monocytes in a sample from the cancer patient, and (b) comparing the level of monocytes in the sample to a reference level of monocytes, wherein a lower level of monocytes in the sample correlates with increased benefit to the patient.

By "lower level" we include patients who have a level of baseline monocytes below the median for a patient in need of immunotherapy or below or towards the lower end of normal levels.

A further method for determining a prognosis for benefit for a cancer patient receiving immunotherapy treatment involves (a) measuring a level of monocytes in a sample from the cancer patient, and (b) classifying the patient as belonging to either a first or second group of patients, wherein the first group of patients having low levels of monocytes is classified as having an increased likelihood of benefit than the second group of patients having high levels of monocytes.

We also describe a method for monitoring the effectiveness of a course of treatment for a patient with cancer. The method involves (a) determining a level of monocytes in a sample from the cancer patient prior to immunotherapy treatment, and (b) determining the level of monocytes in a sample from the patient after treatment whereby comparison of the monocyte level prior to treatment with the monocyte level after treatment indicates the effectiveness of the treatment.

By "receiving immunotherapy" we also include patients who are being assessed for immunotherapy, i.e. patients who could potentially benefit from treatment for cancer.

In other words we describe a method of predicting the responsiveness of a patient or patient population with cancer to treatment with immunotherapy, or for selecting patients or patient populations that may respond to immunotherapy, comprising comparing the differential levels of monocytes wherein a monocyte level below a reference level is associated with benefit

By "lower level" we include a patient or patient population who have a level of monocytes either below a reference level for a patient or patient population who have been diagnosed with cancer and are therefore in need of treatment, such as immunotherapy, or below a reference level for a normal individual or population. By "reference level" we include a level which represents a level below which the administration of immunotherapy will confer a clinical benefit to the patient or patient population, such as improved overall survival, increased progression-free survival, decreased risk of tumour recurrence or spread.

In one embodiment the lower level will include a patient or patient population who have a level of monocytes below the median for a patient or patient population who have been diagnosed with cancer and are therefore in need of treatment, such as immunotherapy; it will also include a patient or patient population who have a level of monocytes which is below the median for a normal individual or patient population.

In one embodiment, the monocyte level associated with a more favourable outcome is about $\leq 1.10 \times 10^9$/L or about $\leq 1.0 \times 10^9$/L, or more preferably about $\leq 0.80 \times 10^9$/L, about $\leq 0.60 \times 10^9$/L, or about $\leq 0.40 \times 10^9$/L. Even more particularly, the monocyte level associated with a more favourable outcome is about $\leq 0.20 \times 10^9$/L. These levels may be particularly associated with patients with RCC.

Reference ranges for monocytes in a normal patient population have been reported as 0.20-1.10 GI/L (0.20-1.10 $\times 10^9$/L) (1+ years) absolute [monocytes % 0.0-12.0 (18+ years)].

White Blood Cell (WBC) levels

We have identified that immunotherapy performs better in cancer patients with a lower baseline white blood cell (WBC) level or count.

Thus, we describe herein a method for determining a prognosis for benefit for a cancer patient receiving immunotherapy treatment involving (a) measuring a level of WBCs in a sample from the cancer patient, and (b) comparing the level of WBCs in the sample to a reference level of WBCs, wherein a lower level of WBCs in the sample correlates with increased benefit to the patient.

By "lower level" we include patients who have a level of baseline WBCs below the median for a patient in need of immunotherapy or below or towards the bottom end of normal levels.

A further method for determining a prognosis for benefit for a cancer patient receiving immunotherapy treatment involves (a) measuring a level of WBCs in a sample from the cancer patient, and (b) classifying the patient as belonging to either a first or second group of patients, wherein the first group of patients having low levels of WBCs is classified as having an increased likelihood of benefit than the second group of patients having high levels of WBCs.

We also describe a method for monitoring the effectiveness of a course of treatment for a patient with cancer. The method involves (a) determining a level of WBCs in a sample from the cancer patient prior to immunotherapy treatment, and (b) determining the level of WBCs in a sample from the patient after treatment, whereby comparison of the WBC level prior to treatment with the WBC level after treatment indicates the effectiveness of the treatment.

By "receiving immunotherapy" we also include patients who are being assessed for immunotherapy, i.e. patients who could potentially benefit from treatment for cancer.

In other words we describe a method of predicting the responsiveness of a patient or patient population with cancer to treatments with immunotherapy, or for selecting patients or patient populations that may respond to immunotherapy, comprising comparing the differential levels of WBCs wherein a WBC level below a reference level is associated with benefit.

By "lower level" we include a patient or patient population who have a level of WBCs either below a reference level for a patient or patient population who have been diagnosed with cancer and are therefore in need of treatment, such as immunotherapy, or below a reference level for a normal individual or population. By "reference level" we include a level which represents a level below which the administration of immunotherapy will confer a clinical benefit to the patient or patient population, such as overall survival, increased progression-free survival, decreased risk of tumour recurrence or spread.

In one embodiment the lower level will include a patient or patient population who have a level of WBCs below the median for a patient or patient population who have been diagnosed with cancer and are therefore in need of treatment, such as immunotherapy; it will also include a patient or patient population who have a level of WBCs which is below the median for a normal individual or patient population.

In one embodiment, the WBC level associated with a more favourable outcome is about $\leq 10.8$ GI/L, about $\leq$

10.4 GI/L, about ≤ 100 GI/L, about ≤ 9.6 GI/L, about ≤ 9.2 GI/L, about ≤ 8.8 GI/L, about ≤ 8.4 GI/L, about ≤ 8.0 GI/L, about ≤ 7.6 GI/L, about ≤ 7.2 GI/L, about ≤ 6.8 GI/L, about ≤ 6.4 GI/L, about ≤ 5.0 GI/L, about ≤ 4.6 GI/L, about ≤ 4.2 GI/L, about ≤ 3.8 GI/L, about ≤ 3.4 GI/L, about ≤ 3.0 GI/L, about ≤ 2.6 GI/L, about ≤ 2.2 GI/L, about ≤ 1.8 GI/L, about ≤ 1.4 GI/L, about ≤ 1.0 GI/L, or about ≤ 0.8 GI/L In other embodiments the WBC level is about ≤ 3.0 GI/L or about ≤ 1.1 GI/L. In another embodiment, the WBC levels is about ≤ 5.0 GI/L, about ≤ 5.9 GI/L, about ≤ 7.2 GI/L, about ≤ 8.6 GI/L or about ≤ 10.4 GI/L. These levels may be particularly associated with patients with RCC.

These WBC levels are reported in connection with an RCC patient population, but the levels or their equivalents in relation to other patient populations may be generally applicable to the field of immunotherapy.

Reference ranges for WBCs in a normal patient population have been reported as 0.8-10.8 GI/L (18+ years).

Combinations

We have found that if a patient has two or more of the afore-mentioned favourable factors or biomarkers then the beneficial effect to the patient of immunotherapy is greater than if the patient only has one of the favourable factors.

Thus, we describe herein a further method for determining a prognosis for benefit for a cancer patient receiving immunotherapy involving (a) measuring levels of one or more, and preferably two or more of the following biomarkers: monocytes, platelets, haemoglobin and WBCs, and (b) comparing the level of the one or more selected biomarkers in the sample to a reference level of the one or more selected biomarkers, wherein a low level of monocytes, WBCs and/or platelets and a high level of any of haemoglobin (as appropriate), in said sample correlates with increased benefit to said patient

In a particularly preferred embodiment we have found that platelet, monocyte and haemoglobin levels impact the efficacy of treatment versus placebo. In particular, abnormal haematological levels, and particularly platelet, monocyte and haemoglobin levels are associated with reduced efficacy of treatment. In other words there is a survival advantage to treatment in patients with normal haematological levels.

We have found that in addition, to afore-mentioned factors or biomarkers, immunotherapy may form perform differently in cancer patients with additional baseline characteristics. The factors which may be beneficial to cancer treatment can be summarised as follows:

| High Levels | Low Levels |
|---|---|
| Chloride | Basophils |
| Creatinine | Corrected Calcium |
| Eosinophils | Serum Calcium |
| Haematocrit | Monocytes |
| Haemoglobin | Neutrophils |
| Sodium | Platelets |
| | White blood cells (WBCs) |

Thus, another method for determining a prognosis for benefit for a cancer patient receiving immunotherapy involving (a) measuring levels of platelets, haemoglobin, monocytes and/or WBCs and one or more biomarkers selected from the group consisting of : corrected calcium, creatinine, chloride, eosinophils, haematocrit, sodium, basophils, serum calcium, and neutrophils and (b) comparing the level of platelets, haemoglobin, monocytes and/or WBCs and the one or more selected biomarkers in the sample to a reference level of platelets, haemoglobin, monocytes and/or WBCs and the one or more selected biomarkers, wherein a low level of platelets, monocytes and/or WBCs, a high level of haemoglobin, and a high level of any of creatnine, chloride, eosinophils, haematocrit, sodium and/or a low level of any corrected calcium, basophils, serum calcium, and neutrophils in said sample correlates with increased benefit to said patient.

In particular in relation to corrected calcium we have found the following levels to be indicative of an effect:

Corrected calcium levels

We have identified that immunotherapy performs better in cancer patients with a lower baseline corrected calcium level.

Thus, we describe herein a method for determining a prognosis for benefit for a cancer patient receiving immunotherapy treatment involving (a) measuring a level of corrected calcium in a sample from the cancer patient, and (b) comparing the level of corrected calcium in the sample to a reference level of corrected calcium, wherein a lower level of corrected calcium in the sample correlates with increased benefit to the patient.

By "lower level" we include patients who have a level of baseline corrected calcium below the median for a patient in need of immunotherapy.

A further method for determining a prognosis for benefit for a cancer patient receiving immunotherapy treatment involves (a) measuring a level of corrected calcium in a sample from the cancer patient, and (b) classifying the patient as belonging to either a first or second group of patients, wherein the first group of patients having low levels of corrected calcium is classified as having an increased likelihood of benefit than the second group of patients having high levels of corrected calcium.

We also describe a method for monitoring the effectiveness of a course of treatment for a patient with cancer. The method involves (a) determining a level of a corrected calcium in a sample from the cancer patient prior to immunotherapy treatment, and (b) determining the level of corrected calcium in a sample from the patient after treatment whereby comparison of the corrected calcium level prior to treatment with the corrected calcium level after treatment indicates the effectiveness of the treatment

By "receiving immunotherapy" we also include patients who are being assessed for immunotherapy, i.e. patients who could potentially benefit from treatment for cancer.

In other words we describe a method of predicting the responsiveness of a patient or patient population with cancer to treatment with immunotherapy, or for selecting patients or patient populations that may respond to immunotherapy, comprising comparing the differential levels of corrected calcium wherein a corrected calcium level below a reference level is associated with benefit.

By "lower level" we include a patient or patient population who have a level of corrected calcium either below a reference level for a patient or patient population who have been diagnosed with cancer and are therefore in need of treatment, such as immunotherapy, or below a reference level for a normal individual or population. By "reference level" we include a level which represents a level below which the administration of immunotherapy will confer a clinical benefit to the patient or patient population, such as improved overall survival, increased progression-free survival, decreased risk of tumour recurrence or spread.

In one embodiment the lower level will include a patient or patient population who have a level of corrected calcium below the median for a patient or patient population who have been diagnosed with cancer and are therefore in need of treatment, such as immunotherapy; it will also include a patient or patient population who have a level of corrected calcium which is below the median for a normal individual or patient population.

In one embodiment, the corrected calcium level associated with a more favourable outcome is about ≤ 12.0 mg/dL or about s 11.0 mg/dL, or more preferably about s 10.0 mg/dL, about ≤ 9.6 mg/dL, or about s 9.4 mg/dL. Even more particularly, the corrected calcium level associated with a more favourable outcome is about ≤ 9.3 mg/dL. In other embodiments the corrected calcium level is about ≤ 9.2 mg/dL, about ≤ 9.1 mg/dL, about ≤ 9.0 mg/dL, about ≤ 8.8 mg/dL, about ≤ 8.7 mg/dL or about ≤ 8.6 mg/dL. Especially preferred are corrected calcium levels of about ≤ 9.3 mg/dL, about ≤ 9.2 mg/dL or about ≤ 9.1 mg/dL. These levels may be particularly associated with patients with RCC or CRC.

More particularly, in one embodiment, the corrected calcium level associated with a more favourable outcome in RCC is about ≤ 12.0 mg/dL or about s 11.0 mg/dL, or more preferably about ≤ 10.0 mg/dL about ≤ 9.6 mg/dL, or about ≤ 9.4 mg/dL. Even more particularly, the corrected calcium level associated with a more favourable outcome in RCC is about ≤ 9.3 mg/dL In other embodiments the corrected calcium level associated with RCC is about ≤ 9.2 mg/dL, about ≤ 9.1 mg/dL, about ≤ 9.0 mg/dL, about ≤ 8.8 mg/dL, about ≤ 8.7 mg/dL or about s 8.6 mg/dL Especially preferred are corrected calcium levels associated with RCC of about ≤ 9.3 mg/dL, about ≤ 9.2 mg/dL or about s 9.1 mg/dL These corrected calcium levels are reported in connection with an RCC patient population in which the corrected calcium levels ranged from 6.8 to 15.4 mg/dL and a median corrected calcium level of 9.3 mg/dL, but the levels or their equivalents in relation to other patient populations may be generally applicable to the field of immunotherapy.

In normal populations the serum level of calcium is closely regulated with a normal *total calcium* of 2.2-2.6 mmol/L (9-10.5 mg/dL) and a normal *ionized calcium* of 1.1-1.4 mmol/L (4.5-5.6 mg/dL).

The amount of total calcium varies with the level of serum albumin, a protein to which calcium is bound. The biologic effect of calcium is determined by the amount of *ionized calcium,* rather than the total calcium. Ionized calcium does not vary with the albumin level, and therefore it is useful to measure the ionized calcium level when the serum albumin is not within normal ranges, or when a calcium disorder is suspected despite a normal total calcium level.

One can derive a corrected calcium level when the albumin is abnormal. This is to correct for the change in total calcium due to the change in albumin-bound calcium, and gives an estimate of what the calcium level would be if the albumin were within normal ranges.

Corrected calcium (mg/dL) = measured total Ca (mg/dL) + 0.8 (4.0 - serum albumin [g/dL]), where 4.0 represents the average albumin level.

When there is hypoalbuminemia (a lower than normal albumin), the corrected calcium level is higher than the total calcium.

Reference ranges for corrected calcium in a normal patient population have been reported as 2.12- 2.56 mmol/L (3+ years) [8.48-10.24 mg/dL].

In respect of creatinine we have found the following levels to be associated with an effect:

Creatinine Levels

We have identified that immunotherapy performs better in cancer patients with a higher baseline creatinine level.

Thus, we describe herein a method for determining a prognosis for benefit for a cancer patient receiving immunotherapy treatment involving (a) measuring a level of creatinine in a sample from the cancer patient, and (b) comparing the level of creatinine in the sample to a reference level of creatinine, wherein a higher level of creatinine in the sample correlates with increased benefit to the patient.

By "higher level" we include patients who have a level of baseline creatinine above the median for a patient in need of immunotherapy.

A further method for determining a prognosis for benefit for a cancer patient receiving immunotherapy treatment involves (a) measuring a level of creatinine in a sample from the cancer patient, and (b) classifying the patient as belonging to either a first or second group of patients, wherein the first group of patients having high levels of creatinine is classified as having an increased likelihood of benefit than the second group of patients having low levels of creatinine.

We also describe a method for monitoring the effectiveness of a course of treatment for a patient with cancer. The method involves (a) determining a level of a creatinine in a sample from the cancer patient prior to immunotherapy treatment, and (b) determining the level of creatinine in a sample from the patient after treatment, whereby comparison of the creatinine level prior to treatment with the creatinine level after treatment indicates the effectiveness of the treatment.

By "receiving immunotherapy" we also include patients who are being assessed for immunotherapy, i.e. patients who could potentially benefit from treatment for cancer.

In other words we describe a method of predicting the responsiveness of a patient or patient population with cancer to treatment with immunotherapy, or for selecting patients or patient populations that may respond to immunotherapy comprising comparing the differential levels of creatinine wherein a creatinine level above a reference level is associated with benefit.

By "higher level" we include a patient or patient population who have a level of creatinine either above a reference level for a patient or patient population who have been diagnosed with cancer and are therefore in need of treatment, such as immunotherapy; or above a reference level for a normal individual or population. By "reference level" we include a level which represents a level above which the administration of immunotherapy will confer a clinical benefit to the patient or patient population such as overall survival, increased progression-free survival decreased risk of tumour recurrence or spread,.

In one embodiment the higher level will include a patient or patient population who have a level of creatinine above the median for a patient or patient population who have been diagnosed with cancer and are therefore in need of treatment, such as immunotherapy; or it will also include a patient or patient population who have a level of creatinine which is above the median for a normal individual or patient population.

In one embodiment, the creatinine level associated with a more favourable outcome is about $\geq 100$ $\mu$mol/L, or about $\geq 110$ $\mu$mol, more preferably about $\geq 120$ $\mu$mol/L. In one even more preferred embodiment, the creatinine level associated with a more favourable outcome is greater than about the normal level in a population. The normal range of creatinine level in a normal population has been reported as about 44 to 124 pmol /L. Thus in one even-more preferred embodiment the creatinine level is about $\geq 124$ $\mu$mol/L. In a particularly, preferred embodiment, the creatinine level associated with a more favourable outcome is about $\geq 130$ $\mu$mol/L, or about $\geq 135$ $\mu$mol/ L. In an especially preferred embodiment, the creatinine level associated with a more favourable outcome is about $\geq 145$ $\mu$mol/L. These levels may be particularly associated with patients with RCC or CRC.

In one embodiment, the creatinine level associated with a more favourable outcome in RCC is about $\geq 100$ $\mu$mol/L, or about $\geq 110$ $\mu$mol, more preferably about $\geq 120$ $\mu$mol/L. In one even more preferred embodiment, the creatinine level associated with a more favourable outcome in RCC is greater than about the normal level in a population. The normal range of creatinine level in a normal population has been reported as about 44 to 124 $\mu$mol /L. Thus in one even more preferred embodiment the creatinine level in RCC is about a 124 $\mu$mol/L. In a particularly, preferred embodiment, the creatinine level associated with a more favourable outcome in RCC is about $\geq 130$ $\mu$mol/L, or about $\geq 135$ $\mu$mol/L. In an especially preferred embodiment, the creatinine level associated with a more favourable outcome in RCC is about $\geq 145$ $\mu$mol/L. These creatinine levels are reported in connection with an RCC patient population in which the creatinine levels ranged from 58-268 $\mu$mol/ L and a median creatinine level of 111 $\mu$mol/ L, but the levels or their equivalents in other patient populations may be generally applicable to the field of immunotherapy.

Eosinophil Levels

In relation to eosinophils, the eosinophil level (ABS) in a normal patient population has been reported as 0.05-0.55 GI/L (0+ years), and also as 0.0-7.0% (18+ years), and references to a high or higher amount can be determined accordingly and with reference to the afore-mentioned definitions.

Haematocrit Levels

In relation to haematocrit, the haematocrit levels in a normal patient population has been reported (given as a percentage of 1.0) as 0.410-0.500 (males 18-64 years), 0.360-0.490 (males 65+ years), 0.350-0.460 (females 18-64 years), and 0.330-0.460 (65+ years), and references to a high or higher amount can be determined accordingly and with reference to the afore-mentioned definitions. In one embodiment for a male (18-64 years) the level should be $\geq 0.201$ and $\leq 0.599$.

Sodium Levels

In relation to sodium, the sodium levels in a normal patient population has been reported as 136 to 145 milliequivalents per liter (mEq/L) of blood. References to a high or higher amount can be determined accordingly and with reference to the afore-mentioned definitions.

Basophil Levels

In relation to basophils, the basophil level (ABS) in a normal patient population has been reported as 0.00-0.20 GI/L (1+ years), and also as 0.0-2.0% (0+ years), and references to a low or lower amount can be determined accordingly and with reference to the afore-mentioned definitions.

Neutrophil Levels

The primary criterion for classification of neutrophils as either segmented (mature) or non- segmented (younger stages including band neutrophils, metamyelocytes, and myelocytes) is the shape of the nucleus. In relation to neutrophils, the neutrophil level (Segmented) in a normal patient population has been reported as 40.0-75.0% (18+ years), and (ABS) as 1.80-8.00 GI/L (1+ years). The neutrophil level (Bands) in a normal patient population has been reported as 0.0-8.0% (18+ years), and (ABS) as 0.00-0.86 GI/L (18+ years). The neutrophil level (total) in a normal patient population has been reported as 40.0-75.0% (18+ years), and (ABS) as 1.80-8.00 GI/L (1+ years). References to a low or lower amount can be determined accordingly and with reference to the afore-mentioned definitions.

In addition to the foregoing, the invention includes, as an additional aspect, all embodiments of the invention narrower in scope in any way than the variations specifically mentioned above. For example, although aspects of the invention may have been described by reference to a genus or a range of values for brevity, it should be understood that each member of the genus and each value or sub-range within the range is intended as an aspect of the invention.

**Brief Description of the Drawings**

Fig 1: Shows the effect of baseline corrected calcium on patient survival in a renal cancer (TRIST) study, and in particular the survival of TRIST patients stratified by above median vs below median baseline corrected calcium.

Fig 2: Shows the effect of baseline corrected calcium on patient survival in a renal cancer (TRIST study, and in particular the survival of TRIST patients over a range of different corrected calcium levels.

Fig 3: Shows the effect of baseline corrected calcium on patient survival in a renal cancer (TRIST) study, and in particular the survival of TRIST patients over a range of different corrected calcium levels.

Fig 4: Shows the effect of baseline platelets on patient survival in a renal cancer (TRIST) study, and in particular the survival of TRIST patients stratified by above median vs below median baseline platelets.

Fig 5: Shows the effect of baseline platelets on patient survival in a renal cancer (TRIST) study, and in particular the survival of TRIST patients over a range of different platelet levels.

Fig 6: Shows the effect of baseline haemoglobin on patient survival in a renal cancer (TRIST) study, and in particular the survival of TRIST patients over a range of different haemoglobin levels.

Fig 7: Shows the effect of baseline creatinine on patient survival in a renal cancer (TRIST) study, and in particular the survival of TRIST patients over a range of different creatinine levels.

Fig 8: Shows the effect of particular baseline corrected calcium, creatinine, haemoglobin and platelets on patient survival in a renal cancer (TRIST) study.

Fig 9: Shows the effect of zero baseline inclusion factors vs one or more than one of the inclusion factors: corrected calcium, creatinine, haemoglobin and platelets on patient survival in a renal cancer (TRIST) study.

Fig 10: Shows the effect of particular baseline corrected calcium, creatinine, haemoglobin and platelets on patient survival in a renal cancer (TRIST) study, and in particular the more preferred baseline variables.

Fig 11: Shows the effect of baseline platelets on patient survival in four colorectal cancer studies, and, in particular the survival of CRC patients stratified by above median vs below median baseline platelets.

Fig 12: Shows the effect of baseline platelets on patient survival in four colorectal cancer studies, and in particular the survival of CRC patients stratified by above vs below a preferred baseline platelet level.

Fig 13: Shows the effect of baseline platelets on patient survival in four colorectal cancer and two renal cancer studies, and in particular the survival of the patients stratified by above median vs below median baseline platelets.

Fig 14: Shows the effect of baseline platelets on patient survival in renal cancer studies at a later stage than in Fig 13, and in particular the survival of the patients stratified by above normal, normal vs below normal baseline platelets.

Fig 15: Shows the effect of baseline haemoglobin on patient survival in four colorectal cancer studies, and in particular the survival of CRC patients stratified by above median vs below median baseline haemoglobin levels.

Fig 16: Shows the effect of baseline creatinine on patient survival in four colorectal cancer studies, and in particular the survival of CRC patients stratified by above median vs below median baseline creatinine levels.

Fig 17: Shows the effect of baseline monocytes on patient survival in renal cancer studies, and in particular the survival of the patients stratified by above normal, normal vs below normal baseline platelets.

Fig 18: Shows the effect of normal haematology on patient survival in renal cancer studies, and in particular the survival of the patients with normal haematology in terms of normal baseline levels of monocytes, platelets and haemoglobin associated with survival benefit trend.

Fig 19: Shows adjusted Hazard ratio ($\pm$ 95% CI) plotted as a function of the baseline platelet concentration. Baseline platelet levels have been segregated into percentiles and the respective platelet levels noted (x $10^9$/L). The number of patients falling into each category is noted along the x axis with the number of events in parentheses.

Fig 20: Shows adjusted Hazard ratio plotted as a function of the baseline monocyte concentration. Baseline monocyte levels have been segregated into percentiles and the respective monocyte levels noted (x $10^9$/L). The number of patients falling into each category is noted on the x axis along with the number of events in parentheses.

Fig 21: Shows adjusted Hazard ratio plotted as a function of the baseline white blood cell (WBC) concentration. Baseline WBC levels have been segregated into percentiles (bottom 10th, 25th and 50th percentile and top 50th, 25th and 10th percentile) and the respective WBC levels noted. The number of patients falling into each category is noted on the x axis along with the number of events in parentheses.

Fig 22: Shows adjusted Hazard ratio plotted as a function of the baseline haemoglobin concentration. Baseline haemoglobin levels have been segregated into percentiles (bottom 10th, 25th and 50th percentile and top 50th, 25th and 10th percentile) and the respective haemoglobin levels noted (g/dL). The number of patients falling into each category is noted on the x axis along with the number of events in parentheses.

**Detailed Description**

Immunotherapy

It is contemplated that the methods of the invention are suitable and applicable for all viral vectors and non-viral vectors adaptable for delivery of an exogenous gene to a mammalian cell for expression of the gene in the cell. A variety of viral vectors may be employed as disclosed herein. In some preferred aspects, viral vectors are replication deficient. Furthermore, as detailed above, a viral vector may preferably comprise poxvirus such as a vaccinia viral vector. A variety of vaccinia viral vectors are known in the art in certain aspects a vaccinia viral vector for use herein may be a modifed vaccinia Ankara (MVA) virus. Other exemplary immunotherapies include compositions that include the protein antigen itself; or fragments or epitopes of the antigen; or vectors for delivering a transgene that encodes the antigen (e.g., plasmid or liposomal vectors).

In other aspects the immunotherapy may comprise a viral vector which can be either the same as the original vector or a different viral vector such as a heterologous prime-boost vaccination regimen.

In some aspects immunotherapeutic methods concern a maintenance immunotherapy that does not comprise the viral vector. For instance, the maintenance immunotherapy comprises a composition comprising the antigen, or at least one epitope thereof, and an adjuvant or carrier. For example, a maintenance immunotherapy may comprise a plasmid that contains a nucleotide sequence that encodes the antigen, operably linked to an expression control sequence to permit expression of the antigen in cells of the mammalian subject. Such a method may, optionally, further comprise (d) immunizing the subject having a measurable immune response to the antigen with a maintenance immunotherapy that is free from the viral vector. Exemplary maintenance immunotherapies include compositions that include the protein antigen itself; or fragments or epitopes of the antigen; or vectors for delivering a transgene that encodes the antigen (e.g., plasmid or liposomal vectors).

In other aspects the maintenance immunotherapy may comprise a viral vector which can be either the same as the original vector or a different viral vector such as a heterologous prime-boost vaccination regimen.

Likewise, methods disclosed herein are applicable to immunotherapy utilizing a variety of antigens. In certain aspects, an antigen as defined herein comprises at least one tumor antigen such as the tumor antigens listed in the detailed description below. For example, the tumor antigen may comprise a 5T4 antigen. 5T4 antigen and viral vectors comprising 5T4 have been previously described for examples in U.S. Patent No. 7,148,035.

In one embodiment the invention is intended to be applicable to immunotherapies directed against malignancies. Thus, in some variations, the mammalian subject for immunotherapy is a subject having a cancer such as a cancer that expresses a least one tumor antigen (tumor associated antigen). Preferably, a subject having cancer comprises a cancer which expresses the same antigen that is comprised in the viral vector used for immunotherapy. In some cases a subject comprising a cancer may be a subject with a renal cell or a colorectal cancer. Preferably a mammalian subject is a human subject.

In addition to the immunotherapy methods described herein subjects may further be treated with one or more additional therapies such as a therapy considered the standard of care for a particular disease such as cancer. For example, the additional therapy or standard of care therapy may be chemotherapy, radiation therapy, surgery or cytokine therapy.

Generally, an immunotherapy for use herein will be formulated in a pharmaceutically acceptable carrier, and may additionally comprise preservatives, salts and/or adjuvants.

I. Tumor-associated antigens (TAAs)

In certain aspects the application concerns a tumor associated antigen. A suitable tumor associated antigen (TAA) or tumor antigens includes 5T4. As used herein the terms tumor associated antigen and tumor antigen are used interchangeably. Other suitable antigens include TAAs in the following classes: cancer testis antigens (e.g., HOM-MEL-40), differentiation antigens (e.g., HOM-MEL-55), overexpressed gene products (HOM-MD-21), mutated gene products (NY-COL-2), splice variants (HOM-MD-397), gene amplification products (HOM-NSCLC-11) and cancer related autoantigens (HOM-MEL-2.4) as reviewed in Cancer Vaccines and immunotherapy (2000) Eds Stern, Beverley and Carroll, Cambridge University Press, Cambridge. Further examples include, MART-1 (Melanoma Antigen Recognized by T-cells-1) MAGE-A (MAGE-A1, MAGE-A2, MAGE-A3, MAGE-A4, MAGE-A6, MAGE-A8, MAGE-A10, MAGE-A12), MAGE B (MAGE-B1- MAGE-B24), MAGE-C (MAGE-C1/CT7, CT10), GAGE (GAGE-1, GAGE-8, PAGE-1, PAGE-4, XAGE-1, XAGE-3), LAGE (LAGE-1a(1S), -1b(1L), NY-ESO-1), SSX (SSX1-SSX-5), BAGE, SCP-1, PRAME (MAPE), SART-1, SART-3, CTp11, TSP50, CT9/BRDT, gp100, MART-1, TRP-1, TRP-2, MELAN-A/MART-1, Carcinoembryonic antigen (CEA), prostate-specific antigen (PSA), MUCIN (MUC-1) and Tyrosinase. TAAs are reviewed in Cancer Immunology (2001) Kluwer Academic Publishers, The Netherlands. Additional tumor associated antigens include Her 2, survivin and TERT.

The term "antigen" refers to protein or peptide to be introduced into a subject. As described herein, an antigen may be provided through delivering a peptide or protein or through delivering a nucleic acid encoding a peptide or protein.

By "antigen" in the context of the present invention it is also meant to incorporate an antigenic peptide derived from an antigen. In particular, "tumor associated antigen" is intended to encompass a peptide derived from a tumor associated antigen.

An antigen such as a tumor associated antigen can be provided for use as a medicament in a number of different ways. It can be administered as part of a viral vector. A number of suitable viral vectors will be familiar to those skilled in the art and include a number of vectors described herein.

II. TroVax® Vaccine

TroVax® consists of a highly attenuated strain of vaccinia virus (VV), termed Modified Vaccinia Ankara, (MVA), and contains the human TAA 5T4 glycoprotein gene under regulatory control of a modified promoter, mH5. Thus, by "TroVax®" we include Modified Vaccinia Ankara, (MVA), that contains the human TAA 5T4 glycoprotein gene, and preferably under regulatory control of a modified promoter, mH5

MVA was developed as a safe vaccine for smallpox and MVA was derived from the VV Ankara strain by passaging in primary chick embryo fibroblasts (CEF), after which it was found to be replication defective in all mammalian cell lines tested, except Baby Hamster Kidney cells (BHK-21). Molecular genetic analysis of MVA has revealed substantial differences from the replication competent vaccinia virus which indicate that reversion of attenuation is highly unlikely. MVA is non-pathogenic in mammals including suckling mice, rabbits and primates. Importantly, no complications were reported when MVA was administered to over 120,000 subjects, many of whom were at risk from vaccine complications. Replication of competent strains of VV are handled in a Biosafety level II environment; however, MVA has been assigned Biosafety level I status by the National Institutes of Health Intramural Biosafety Committee in the US, the UK Health and Safety Executive and the biosafety authorities in Germany.

5T4 is a 72 kDa oncofoetal glycoprotein that is expressed on over 70% of carcinomas of the kidney, breast, gastrointestinal tract, colon and ovaries. Unlike other self-antigen TAAs such as CEA, 5T4 expression as detected by histochemical staining appears to be tumor specific with only low level sporadic staining observed in the gut and pituitary. However this level of staining is so low that it is difficult to determine if it is specific. 5T4-postitive tumors include invasive carcinoma of the Ampulla of Vater, breast, colon, endometrium, pancreas, or stomach; a squamous carcinoma of the bladder, cervix, lung or oesophagus; a tubulovillous adenoma of the colon; a malignant mixed Mullerian tumour of the endometirem; a clear cell carcinoma of the kidney; a lung cancer (large cell undifferentiated, giant cell carcinoma, broncho-alveolar carcinoma, metastatic leiomyosarcoma); an ovarian cancer (a Brenner tumour, cystadenocarcinoma, solid teratoma); a cancer of the testis (seminoma, mature cystic teratoma); a soft tissue fibrosarcoma,; a teratoma (anaplastic germ cell tumours); or a trophoblast cancer (choriocarcimoma (e.g. in uterus, lung or brain), tumour of placental site, hydatidiform mole). Immunohistochemical analysis indicates that 5T4 expression is an indicator of poor prognosis in colorectal cancer. Additionally, when tumor cells are transfected with the cDNA encoding 5T4, they display increased motility suggesting that expression of this molecule may induce metastatic properties in a tumor.

TroVax® is able to induce an anti-5T4 antibody response in mice. Additionally, such a response is able to prevent the establishment of syngeneic tumor cells expressing human 5T4 in two murine tumor models. To model more accurately the possible anti-tumor effects of TroVax® in humans, MVA recombinants were constructed expressing the murine homologue of 5T4 (m5T4). In this self-antigen model MVA-m5T4 induction of an m5T4 antibody response was observed. Furthermore such a response is able to retard or prevent the establishment of syngeneic tumor cells expressing m5T4. Mice have been vaccinated on four occasions with MVA-m5T4 and there have been no reports of toxicity. In addition a number of studies have explored the toxicological consequences of immunization with TroVax®. Mice have been immunized with up to 12 repeated administrations of TroVax®. There were no TroVax® related deaths or adverse effects on clinical signs, body weight, food consumption, organ weights or clinical pathology. There were no macroscopic or microscopic findings suggestive of systemic toxicity due to the test articles.

Because 5T4 is an oncofoetal antigen, mice, previously vaccinated with MVA-m5T4, were used for breeding. It was found that immunity to m5T4 did not have a detrimental effect on the ability of mice to become pregnant or give birth to healthy progeny. In a more detailed study, female mice were administered with approx 107 pfu of TroVax® or MVA-m5T4 or placebo at 21 and 14 days prior to pairing with untreated males and, for the pregnant females, on Day 6 of gestation. The pregnant females were maintained to Day 18 of gestation then the injected animals and their respective foetuses analysed macroscopically at necropsy. All clinical observations and necropsy findings were unremarkable. The pregnancy rate was slightly lower in the groups given both TroVax® and MVA-m5T4 compared to control. The toxicological significance of this finding is uncertain but may reflect a treatment impact on mating behavior. There was no adverse effect of treatment with either TroVax® or MVA-m5T4 on the uterine/implantation or foetal data. In summary, there was no female or maternal toxicity and no embryo-foetal toxicity in either group. Histological examination of the tissues from the MVA-m5T4 animals revealed no adverse microscopic findings.

It is apparent from pre-clinical studies that TroVax® has little potential to induce toxicity but is likely to induce an efficacious immune response to 5T4. In vivo studies suggest that such an immune response will have anti-tumor activity.

TroVax® has been administered to over 400 patients with metastatic colorectal or renal cancer. Over 3000 doses have been administered. No serious adverse event attributed to TroVax® by investigators or the sponsor has been reported. Mild transient injection site reactions are reported in the majority of patients together with mild transient pyrexia. No other notable, common or serious adverse events have been reported in studies using TroVax® as a single agent in heavily pretreated patients or in studies combining TroVax® with chemotherapy, (5FU and leucovorin combined with either oxaliplatin or irinotecan), interferon-α , IL-2 (high dose intravenous regimen or low dose subcutaneous injections) or with sunitinib.

Cancer treatment

The present invention is particularly suitable for use in predicting the response to the aforementioned immunotherapeutic agents in patients or patient population with a cancer. Such cancers include, for example, non-solid tumours such as leukaemia, multiple myeloma or lymphoma, and also solid tumours, for example bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, placental, uterine and vulval tumours.

The present invention is particularly suitable for identifying those patients with renal, colorectal, breast, prostate or ovarian cancer, more particularly renal or colorectal cancer that will respond to treatment with immunotherapeutic agents, such as TROVAX® as hereinbefore defined.

Sample

As used herein, the term "sample" is intended to mean any biological fluid, cell, tissue, organ or portion thereof. The term includes samples present in an individual as well as samples obtained or derived from the individual. For example, a sample can be a histologic section of a specimen obtained by biopsy, or samples that are placed in or adapted to tissue culture. Furthermore a sample can be a subcellular fraction or extract, or a crude or substantially pure protein preparation.

In the methods of the invention, a sample can be, for example, a cell or tissue obtained using a biopsy procedure or can be a fluid sample containing cells, such as blood, serum, semen, urine, or stool. Those skilled in the art will be able to determine an appropriate sample, which will depend on cancer type, and an appropriate method for obtaining a biopsy sample, if necessary. When possible, it can be preferable to obtain a sample from a patient using the least invasive collection means. For example, obtaining a fluid sample from a patient, such as blood, saliva, serum, semen, urine or stool, is less invasive than collecting a tissue sample.

Reference level

As used herein, the term "reference level" refers to a control level of expression of a factor or biomarker used to evaluate a test level of expression of a factor or biomarker in a sample of a patient. For example, when the level of corrected calcium in the patient is higher than the reference level of corrected calcium, the patient will be considered to have a high level of corrected calcium. Conversely, when the level of corrected calcium in the patient is lower than the reference level, the patient will be considered to have a low level of corrected calcium. In another example, when the level of platelets in the patient is higher than the reference level of platelets, the patient will be considered to have a high level of platelets. Conversely, when the level of platelets in the patient is lower than the reference level, the patient will be considered to have a low level of platelets. In a further example, when the level of haemoglobin in the patient is higher than the reference level of haemoglobin, the patient will be considered to have a high level of platelets. Conversely, when the level of haemoglobin in the patient is lower than the reference level, the patient will be considered to have a low level of haemoglobin. Similarly, when the level of creatinine in the patient is higher than the reference level of creatinine, the patient will be considered to have a high level of creatinine. Conversely, when the level of creatine in the patient is lower than the reference level, the patient will be considered to have a low level of creatinine. Similarly, when the level of monocytes in the patient is higher than the reference level of monocytes, the patient will be considered to have a high level of monocytes. Conversely, when the level of monocytes in the patient is lower than the reference level, the patient will be considered to have a low level of monocytes. Furthermore, when the level of WBCs in the patient is higher than the reference level of WBCs, the patient will be considered to have a high level of WBCs. Conversely, when the level of WBCs in the patient is lower than the reference level, the patient will be considered to have a low level of WBCs.

In some variations, the reference level may be a range or an average or median measurement of a biomarker calculated from a plurality mammalian subjects that are proposed for the immunotherapy or are not in need of immunotherapy. Where the reference is an average or median, a measurement for the biomarker level above the reference measurement is scored as elevated and a measurement below the reference measurement is scored as reduced. When the reference is a range a measurement for the biomarker around or above the top level for the reference measurement is scored as elevated and a measurement around or below the bottom level for the reference measurement is scored as reduced. In other variations, a measurement that statistically varies from the median or mean by a suitable significant amount (e.g., 1 or 1.5 or 2 standard deviations; or by a "p-value" or other statistical measure of significance) is scored as elevated or reduced.

In other variations, a reference level may be a baseline measurement or any other absolute measurement for a particular assay tool. In such variations, an elevated level or a reduced level may represent values that are a certain multiple or fraction of the reference value.

Some aspects of the invention involve screening for or determining the presence of a measurable difference from the reference level. Measurable may be defined as a level greater or lower than a baseline response, and more preferably at least about 2, 5, 10, 50, 100 or 1000 fold over or below a baseline response. In cases where there is no measurable baseline response, a baseline response may be defined as the lower detection limit of the assay used to measure the level.

The reference level can be determined by a plurality of methods, provided that the resulting reference level accurately provides a level of a biomarker above which exists a first group of patients having a different probability of survival than that of a second group of patients having levels of the biomarker below the reference level. The reference level can be determined by, for example, measuring the level of a biomarker in a non-tumorous sample as the sample of the patient to be tested. The reference level can also be a level of a biomarker of in vitro cultured cells which can be manipulated to simulate tumour cells, or can be manipulated in any other manner which yields levels of the biomarker which accurately determine the reference level.

The reference level can also be determined by comparison of the level of a biomarker, such as monocytes, WBCs, corrected calcium, platelet count, haemoglobin or creatinine, in populations of patients having the same cancer. This can be accomplished, for example, by histogram analysis, in which an entire cohort of patients are graphically presented, wherein a first axis represents the level of the biomarker, and a second axis represents the number of patients in the cohort whose samples contain the biomarker at a given level. Two or more separate groups of patients can be determined by identification of subsets populations of the cohort which have the same or similar levels of the biomarker. Determination of the reference level can then be made based on a level which best distinguishes these separate groups. A reference level also can represent the levels of two or more markers. Two or more markers can be represented, for example, by a ratio of values for levels of each biomarker.

The reference level can be a single number, equally applicable to every patient, or the reference level can vary, according to specific subpopulations of patients. For example, older men might have a different reference level than younger men for the same cancer, and women might have a different reference level than men for the same cancer. Furthermore, the reference level can be some level determined for each patient individually. For example, the reference level might be a certain ratio of a biomarker in the neoplastic cells of a patient relative to the biomarker levels in non-tumour cells within the same patient. Thus the reference level for each patient can be proscribed by a reference ratio of one or more biomarkers, such as corrected calcium or platelet count, wherein the reference ratio can be determined by any of the methods for determining the reference levels described herein.

The reference level may be determined by measuring the baseline level of a biomarker prior to the commencement of immunotherapy

A high level of a biomarker, such as corrected calcium, platelet levels haemoglobin or creatinine can be related to a level of the biomarker above a determined reference level. Thus, a reference or basal level of a biomarker, such as corrected calcium, platelet levels, haemoglobin or creatinine in a sample is identified as a "cutoff" value, above which there is a significant correlation between the presence of the biomarker and increased or decreased tumour recurrence or spread. Those of skill in the art will recognize that some "cutoff" values are not sharp in that clinical correlations are still significant over a range of values on either side of the cutoff; however, it is possible to select an optimal cutoff value (for example varying H-scores, and the like) of a level of a biomarker for a cancer cell type. It is understood that improvements in optimal cutoff values could be determined, depending on the sophistication of statistical methods used and on the number and source of samples used to determine reference or basal values.

Verification that the reference level distinguishes the likelihood of tumour recurrence or spread in cancer patients expressing below-reference biomarker levels versus cancer patients expressing above-reference biomarker levels can be carried out using single variable or multi-variable analysis. These methods determine the likelihood of a correlation between one or more variables and a given outcome. In the specific case, the methods will determine the likelihood of a correlation between a biomarker level, or the levels of more than one biomarker (or a biomarker level coupled with another variable) and disease-free or overall survival of cancer patients. Any one of a plurality of methods well known to those of ordinary skill in the art for carrying out these analyses can be used. Examples of single variable analysis are the Kaplan-Meir method or the log-rank test. An example of multi-variable analysis is the Cox proportional-hazards regression model.

The Kaplan-Meier estimator used in the Examples (also known as the product limit estimator) estimates the survival function from life-time data. In medical research, it might be used to measure the fraction of patients living for a certain amount of time after treatment. The survival function, also known as a *survivor function* or *reliability function,* is a property of any random variable that maps a set of events, usually associated with mortality or failure of some system, onto time. It captures the probability that the system will survive beyond a specified time. The term *reliability function* is common in engineering while the term *survival function* is used in a broader range of applications, including human mortality.

Population-based determination of reference levels, for example, by histogram analysis can be carried out using a cohort of patients sufficient in size in order to determine two or more separate groups of patients having different biomarker levels. Typically, such a cohort comprises at least 25 patients, such as at least 50 patients, including at least 75 patients, and at least 100 patients. Similarly, verification of determined reference levels can also comprise at least 25 patients, such as at least 50 patients, including at least 75 patients, and at least 100 patients.

Further, while a reference level can separate two groups of patients, it is within the scope of the invention that numerous reference values might exist which separate a plurality of populations. For example, two reference values can separate a first group of patients with high levels of a biomarker from a second group of patients with intermediate levels the biomarker, and from a third group of patients with low levels of the biomarker. The number of different reference levels can be sufficient to proscribe a curve, such as a continuous line, which describes the likelihood of disease-free or overall survival in a patient as a function of the biomarker level in that patient. Such a curve will constitute a "continuous" biomarker level, where the likelihood of disease-free or overall survival in a patient is proportional to the biomarker level in that patient. Two or more biomarker levels can also be represented by such a curve.

Further Combinations

The reference level of a biomarker, such as moncytes, platelet count, haemoglobin or WBCs, can further be used in conjunction with another variable found to be a statistically significant indicator of the likelihood of disease-free or overall survival for cancer. Such indicators include the presence or levels of known cancer markers, or can be clinical or pathological indicators (for example, age, tumour size, tumour histology, clinical stage, family history and the like). For example, clinical stage of the cancer is also a statistically significant indicator of disease-free or overall survival, wherein the reference level of a biomarker can vary according to the clinical stage of the cancer. For example, the level of a biomarker, such as a low level of monocytes, platelet count, and/or WBCs, and/or a high level of haemoglobin in conjunction with clinical stage II of a cancer for a given patient, together are indicators for increased likelihood of disease free or overall survival. Hence, the reference level of a biomarker can vary as a function of another statistically significant indicator of disease-free or overall survival for cancer.

The use of two or more biomarkers can provide increased confidence in prognostic outcome. For example, as disclosed herein, combinations of low monocytes and low platelet count were correlated with increased disease-free survival. Thus, combinations of biomarkers useful in the prognostic methods of the invention include, for example, monocytes, WBCs, platelet count, haemoglobin, and a multiplicity of other combinations with biomarkers such as corrected calcium, chloride, creatinine, eosinophils, haematocrit, sodium, basophils, serum calcium, and neutrophils and a variety of other general and tumour-specific biomarkers, such as commercially available diagnostic markers.

Survival

One aspect of the present invention is seeking to achieve improved patient survival including progression-free survival, disease free-survival and overall survival, and also reducing the risk of recurrence and/or metastases. As used herein, the term "disease-free survival" includes the lack of tumour recurrence and/or spread and the fate of a patient after diagnosis, for example, a patient who is alive without tumour recurrence. The phrase "overall survival" refers to the fate of the patient after diagnosis, regardless of whether the patient has a recurrence of the tumour.

As used herein, the term "risk of recurrence" refers to the probability of tumour recurrence or spread in a patient subsequent to diagnosis of cancer, wherein the probability is determined according to the process of the invention.

Tumour recurrence refers to further growth of neoplastic or cancerous cells after diagnosis of cancer. Particularly, recurrence can occur when further cancerous cell growth occurs in the cancerous tissue. Tumour spread refers to dissemination of cancer cells into local or distant tissues and organs, for example during tumour metastasis. Tumour recurrence, in particular, metastasis, is a significant cause of mortality among patients who have undergone surgical treatment for cancer. Therefore, tumour recurrence or spread is correlated with progression-free survival, disease-free and overall patient survival.

Progression-free survival denotes the chances of staying free of disease progression after a particular treatment.

Benefit

As used in the context of a course of treatment, "benefit" refers to the ability of the course of treatment to decrease the risk of tumour recurrence or spread and therefore to increase the likelihood of disease-free, progression-free, or overall survival of the patient.

The methods of the invention for determining a prognosis for survival for a cancer patient are applicable to patients at any stage of tumour progression, and further can be used to determine a stage of tumour progress. A stage of a tumour refers to the degree of progression of a tumour. Various stages of tumour development are well known to those of skill in the art, as exemplified in Markman, "Basic Cancer Medicine," Saunders, (ed. Zorab, R.) (1997). For example, cancers can be staged into three general stages-localized, regional spread, and distant spread. Cancers also can be staged using the TNM system, which considers the extent of direct spread within affected and nearby tissues, the extent of spread to nearby lymph nodes, and the extent of spread to distant organs. Based on these features, spread of cancers can be summarized by assigning Roman numerals from 0 through IV. Those skilled in the art can select an appropriate staging system for a particular type of cancer.

In particular, colon cancer can be staged using the Dukes, Astler-Coller and AJCC/TNM systems, which describe the spread of the cancer in relation to the layers of the wall of the colon or rectum, organs next to the colon and rectum, and other organs farther away. Dukes stage A is equivalent to AJCC/TNM stage I and Astler-Coller stage A, BI; Duke's stage B is equivalent to AJCC/TNM stage II and Astler-Coller stage B2, B3. Dukes stage C is equivalent to AJCC/TNM stage III and Astler-Coller stage C1, C2, C3. AJCC/TNM stages of colorectal cancer are as follow: Stage 0: the cancer has not grown beyond the inner layer (mucosa) of the colon or rectum. This stage is also known as carcinoma in situ or intramucosal carcinoma; Stage I: the cancer has grown through the mucosa into the submucosa, or can also have grown into the muscularis propria, but it has not spread outside the wall itself into nearby tissue such as lymph nodes; Stage II: the cancer has grown through the wall of the colon or rectum, into the outermost layers and may have invaded other nearby tissues, but has not yet spread to the nearby lymph nodes; Stage III: the cancer can be of any size, but has spread to 3 or fewer nearby lymph nodes, or has spread to 4 or more nodes but it has not spread to other parts of the body; Stage IV: the cancer has spread to distant organs such as the liver, lung, peritoneum or ovary.

The staging system for renal cell cancer is based on the degree of tumour spread beyond the kidney. Involvement of blood vessels may not be a poor prognostic sign if the tumour is otherwise confined to the substance of the kidney. Abnormal liver function test results may be due to a paraneoplastic syndrome that is reversible with tumour removal and do not necessarily represent metastatic disease. Except when computed tomography (CT) examination is equivocal or when iodinated contrast material is contraindicated, CT scanning is as good as or better than magnetic resonance imaging (MRI) for detecting renal masses.

The American Joint Committee on Cancer (AJCC) has designated staging by TNM classification.

TNM definitions
Primary tumour (T)

* TX: Primary tumour cannot be assessed
* T0: No evidence of primary tumour
* T1: Tumour 7 cm or less in greatest dimension limited to the kidney
* T2: Tumour more than 7 cm in greatest dimension limited to the kidney
* T3: Tumour extends into major veins or invades adrenal gland or perinephric tissues but not beyond Gerota's fascia
* T3a: Tumour invades adrenal gland or perinephric tissues but not beyond Gerota's fascia
* T3b: Tumour grossly extends into the renal vein(s) or vena cava below the diaphragm
* T3c: Tumour grossly extends into the renal vein(s) or vena cava above the diaphragm
* T4: Tumour invades beyond Gerota's fascia

Regional lymph nodes (N)

* NX: Regional lymph nodes cannot be assessed
* N0: No regional lymph node metastasis
* N1: Metastasis in a single regional lymph node
* N2: Metastasis in more than 1 regional lymph node
* [Note: Laterality does not affect the N classification.]

Distant metastasis (M)

* MX: Distant metastasis cannot be assessed
* M0: No distant metastasis
* M1: Distant metastasis

AJCC stage groupings

Stage I

* T1, N0, M0

Stage II

* T2, N0, M0

Stage III

* T1, N1, M0
* T2, N1, M0
* T3a, N0, M0
* T3a, N1, M0
* T3b, N0, M0
* T3b, N1, M0
* T3c, N0, M0
* T3c, N1, M0

Stage IV

* T4, N0, M0
* T4, N1, M0
* Any T, N2, M0
* Any T, Any N, M1

Early stages of tumour development shall be understood to refer to stages in tumour development in which the tumour has detectably spread no further than the lymph nodes local to the organ of the primary tumour. Typically, early stages will be considered to be stages I and II.

Analytical methods

The following analytical methods were used in the following Examples, and may be usefully used to determine the appropriate levels of the biomarkers in the present invention:

| ASSAY | METHOD/INSTRUMENT |
| --- | --- |
| Creatinine in Serum | Olympus AU640/2700/5400 |

Analytical principle:

This procedure employs a kinetic modification of the classical Jaffe method which was first described in 1886. It involves the reaction of creatinine and an alkaline picrate solution to produce a red tautometer of creatinine picrate of which the absorbance is measured bichromatically at 520 nm/660 nm. The following reaction occurs at 37°C:

Creatinine + Alkaline Picrate $\rightarrow$ Creatinine-Picrate

The method measures the absorbance of the sample at a primary wavelength of 520 nm and compares it to the absorbance produced by a known calibrator. The result is then printed out directly in mg/dL.

| ASSAY | METHOD/INSTRUMENT |
|---|---|
| COMPLETE BLOOD COUNT (includes Haemoglobin measurement and monocyte, WBC and platelet count) | BECKMAN Version: 7 COULTER GENS/LH750ILH780 |

The Coulter employs electronic counting and sizing of particles to quantitate and evaluate blood cells. Gen S/LH750/LH780 WBC Differential analysis and classification are based on simultaneous measuring of cell volume, high frequency conductivity and laser light scatter. Hemoglobin, released by hemolysis to either a stable cyanide containing pigment or oxyhemoglobin-based hemachromagen, is measured by photometric absorbance.

| ASSAY | METHOD/INSTRUMENT |
|---|---|
| Corrected calcium | calculated using the formula: **[(4 - serum albumin g/dL) * 0.8 + serum calcium mg/dL]** |

Serum albumin and calcium is measured.

| ASSAY | METHOD/INSTRUMENT |
|---|---|
| Albumin in Serum | Olympus AU640/2700/5400 |

Analytical principle:

This procedure is based on the dye-binding capability of albumin. Albumin reacts with bromocresol green at pH 4.0 to form a green color. The increase in color is measured bichromatically (600/800 nm) and is proportional to the amount of albumin present. The Olympus method measures the absorbance of the sample at a primary wavelength of 600 nm and compares it to the absorbance produced by known calibrators. The result is then printed out directly in g/dL.

| ASSAY | METHOD/INSTRUMENT |
|---|---|
| Calcium in Serum | Olympus AU640/2700/5400 |

Analytical principle:

This Olympus Calcium procedure is based on Calcium ions (Ca2+) reacting with Arsenazo III (2,2'-[1,8- Dihydroxy-3,6-disulphonaphthylene-2,7-bisazo]-bisbenzenear-sonic acid) to form an intense purple colored complex. Magnesium does not significantly interfere in calcium determination using Arsenazo III. In this method the absorbance of the Ca-Arsenazo III complex is measured bichromatically at 660/700 nm. The resulting increase in absorbance of the reaction mixture is directly proportional to the calcium concentration in the sample.

$$Ca^{2+} + \text{Arsenazo III} \xrightarrow{\text{Acidic Medium}} \text{Ca-Arsenazo III complex (purple)}$$

**Examples**

**Example 1: Study Details - TRIST**

The study was termed TRIST: TroVax® Renal Immunotherapy Survival Trial. An international Phase III, randomized, double blind, placebo controlled, parallel group study to investigate whether TroVax® added to first-line standard of care therapy, prolongs the survival of patients with locally advanced or metastatic renal clear cell adenocarcinoma.

The primary purpose of this trial is to demonstrate the effect of TroVax® on survival in patients with locally advanced or metastatic renal clear cell adenocarcinomas. Clear cell adenocarcinomas of the kidney uniformly express 5T4 at high concentrations (80-90% of tumors examined) and are therefore an obvious candidate for treatment with a 5T4 vaccine.

Reported median survival times for this indication vary between studies but are generally in the range of 6 to 18 months depending on patient's status at entry and to a lesser extent on treatment. Novel forms of treatment are urgently needed.

This study will assess the impact on survival of adding TroVax® to the first-line standard of care for renal cancer. The current standard of care varies between countries and institutions and is influenced by the patient's status, the national regulatory status of different treatments and local reimbursement considerations. Commonly accepted standards of care for renal cancer include IL-2, IFN$\alpha$, or a receptor tyrosine kinase inhibitor such as sunitinib. The use and availability of these treatments varies geographically.

High dose IL-2, although approved for the treatment of renal cancer is not included in this study as the high incidence of serious adverse events and need for intensive care limit its application and would complicate the safety evaluation of TroVax®.

The rationale for the potential concurrent use of IL-2 is that this compound is believed to act as an adjuvant. IL-2 is currently one of the standards of care regimens for the first line treatment of advanced and metastatic renal cancer. The dose schedule of IL-2 chosen is well recognised by the oncology community and has been validated in large scale phase III clinical trials. Over 30 patients treated with a combination of TroVax® and IL-2 (high dose intravenous or low dose subcutaneous regimens) have been assessed in phase II studies in patients with renal cancer. The combination was well tolerated. Compared with the historical adverse event profile of IL-2 alone the only additional adverse events reported were minor local reactions at the site of TroVax® injection and mild transient pyrexia. Humoral and/or cellular immune responses to 5T4 were induced in almost all patients and objective responses by RECIST have been reported.

Although it is not clear whether the biologic effects of IFN$\alpha$ occur entirely or in part via immunostimulation, there is evidence to show that it does have a modest clinical effect in renal cancer patients with an objective response rate of approximately 7.5-15%. Studies to determine whether IFN$\alpha$ increases survival in patients with renal cancer have produced inconsistent results. Given the immunological mechanism of action of IFN$\alpha$, it is reasonable to evaluate the effect of TroVax® on survival in patients receiving this common standard of care. An ongoing study has not indicated any untoward safety impact resulting from coadministration of IFN$\alpha$ and TroVax®.

Phase II studies including over 20 patients treated with a combination of TroVax® and IFN$\alpha$ (three times weekly subcutaneous regimen) are ongoing in patients with renal cancer. Developing data indicate the combination to be well tolerated. Compared with the historical adverse event profile of IFN$\alpha$ alone the only additional adverse events reported are minor local reactions at the site of TroVax® injection and mild transient pyrexia. The expected humoral and/or cellular immune responses to 5T4 will be confirmed. Interim study reports will be available for review by regulatory authorities, IRB / Ethics Committees and investigators as part of the approval process of this study.

Recently developed oral kinase inhibitors, such as sorafenib and sunitinib, are becoming increasingly important in the management of advanced or metastatic renal cell carcinoma. Safety and immunology data necessary to support coadministration of sorafenib and TroVax® are not available. In view of this and the higher overall response rate reported with sunitinib the latter will be included in this study as an example of a kinase inhibitor used in the treatment of renal cancer.

Therefore, in regions where this treatment is approved, sunitinib may be used as the standard of care alongside TroVax®/placebo in this study. A phase II study of patients treated with a combination of TroVax® and sunitinib (50mg oral dose taken once daily, on a schedule of 4 weeks on treatment followed by 2 weeks off) is ongoing in patients with renal cancer. Developing data indicate the combination to be well tolerated. Compared with the reported data on sunitinib alone the only additional adverse events reported are minor local reactions at the site of TroVax® injection and mild transient pyrexia. The expected humoral and/or cellular immunes response to 5T4 are to be confirmed. An interim study report will be available for review by regulatory authorities, IRB / Ethics Committees and investigators as part of the approval process of this study.

A cancer vaccine is intended to prolong survival by inducing an immune response to a tumor associated antigen. Preclinical models indicate that cancer vaccines may delay tumor growth and reduce the number of new metastases. It is not yet known whether a cancer vaccine must produce a high objective tumor response rate (by RECIST) in order to have clinically useful effect on prolonging survival. This will only be determined by a randomised survival study in patients receiving adequate vaccination to reliably induce an efficacious immune response. To date, both disease stabilization and late tumor responses have been reported with various cancer vaccines.

The maximum immunological response to TroVax® dose not usually occur until the patient has received a minimum of three injections and it is not yet established whether continuing TroVax® despite early progression will confer therapeutic benefit. Therefore, in this study, if tumor progression is observed but the patient is tolerating TroVax®/placebo and their performance status remains at a Karnofsky score >60%, they should be requested to continue on study receiving TroVax®/placebo until they have received a minimum of eight injections of the study preparation. Continuation on study beyond this point to receive all TroVax®/placebo injections is permitted for such patients but is at the discretion of the investigator or patient.

A randomized, parallel group, double blind design is standard in phase III efficacy studies. Interim statistical analyses conducted by an independent Data Safety Monitoring Board according to a pre-specified charter will be based on these interim analyses of safety and efficacy. The DSMB may recommend continuation of the study, stopping the study or stopping enrolment of patients of a specific treatment cell. The DSMB will also assess whether the frequency of events in the control arm matches the predictions used to determine the sample size of the study and may recommend changes to the number of events (deaths) triggering the final analysis.

TroVax® is a vaccine against a tumor-associated antigen. The assessment of such tumor vaccines for patients with solid tumors is complicated by a number of factors which influence the definition of the objectives, the route to achieving the objectives and the ongoing management of patients in the study.

**Special features of tumor vaccines that are relevant to the objectives are listed below:**

- Vaccine-mediated immunotherapy requires repeated administration and time for the patient to develop an immune response to the vaccine antigen. In previous phase II studies it was shown that at least three administrations of TroVax® were required to generate a significant immune response. This means that patients who are removed from the study medication before receiving three injections of TroVax® due to death or rapidly progressive renal cancer, do not allow assessment of the potential of a TroVax®-induced immune response to provide benefit to patients treated for a longer period.

- Cancer vaccines such as TroVax® may exert beneficial effects in delaying tumor growth and metastasis that do not manifest as RECIST responses but may prolong survival. This has implications for the management of patients because patients with a RECIST classification of progressive disease may still benefit from continuing with TroVax.

- It is not yet known whether tumor shrinkage predicts survival advantage. This means that the definitive efficacy endpoint is survival.

Objectives

Primary efficacy objective

To assess whether the addition of TroVax® to first line standard of care, will prolong survival of patients with locally advanced or metastatic clear cell renal adenocarcinoma when compared to placebo.

Analysis will occur after a predetermined number of deaths have occurred necessary to trigger the primary endpoint analysis or when specified by an independent Data Safety Monitoring Board based on analyses of interim data.

The analysis will be based on the Intent to Treat (ITT) population, composed of all patients.

Primary safety objective

To assess whether the addition of TroVax® to first line standard of care alters the profile of serious and non-serious adverse events, when compared to placebo, in patients with locally advanced or metastatic clear cell renal adenocarcinoma. This will be assessed in the Intent to Treat (ITT) population.

Secondary efficacy objectives

- To compare the proportion of patients with progression free survival at 26 weeks (+/-1 week) in the TroVax® versus placebo arms based on radiological data. Data will be analysed using the ITT population and adjudicated (blinded peer review) baseline and week 26 radiological data.

- To compare the tumor response rates, time to response and duration of response between patients treated with TroVax® versus placebo. This will be analysed in the Intent to Treat (ITT) population.

To assess whether the addition of a minimum of three doses of TroVax® to first line standard of care, will prolong survival of patients with locally advanced or metastatic clear cell renal adenocarcinoma when compared to placebo. This will be an exploratory analysis in the Modified Intent to Treat (MITT) population.

To assess whether TroVax® has an impact on the quality of life as measured by QLQ30 and EuroQOL questionnaires when compared to placebo. This will be analysed in the Intent to Treat (ITT) population.

Endpoints

Primary efficacy endpoint

The survival event rate ratio in the TroVax® arm versus the placebo in the Intent to Treat (ITT) population based on the log of the hazard ratio derived from the Cox Proportional Hazards regression model. A frequentist monitoring approach will be used for evaluating the event ratio.

The key objective of this study is to determine whether TroVax® is able to prolong survival in patients receiving first line standard of care.

Analysis is triggered by a predetermined number of deaths in the study population or when specified by an independent Data Safety Monitoring Board based on analyses of interim data.

Primary safety endpoints

The number of adverse events (serious and non-serious) in the Intent to Treat population in the TroVax® versus the placebo arm.

The laboratory variables (complete blood count and chemistry panel) in the Intent to Treat (ITT) population in the TroVax® versus the placebo arm.

Secondary efficacy endpoints

The proportion of patients in the TroVax® versus placebo arms in the Intent to Treat (ITT) population with progression free survival at 26 weeks based on a comparison of baseline and week 26 (+/- 1 week) radiological data and using RECIST criteria. Data will be adjudicated (blinded peer review).

Tumor response rates according to the investigator's reported interpretation of the radiological reports based on RECIST criteria observed in the Intent to Treat (ITT) population.

The survival event rate ratio in the TroVax® arm versus the placebo in the Modified Intent to Treat (MITT) population based on the log of the hazard ratio derived from the Cox Proportional Hazards regression model. A frequentist monitoring approach will be used for evaluating the event ratio.

The quality of Life score for TroVax® versus placebo as measured by QLQ30 and EuroQOL questionnaires in the Intent to Treat (ITT) and Per Protocol populations.

Immunology endpoint

Anti-5T4 antibody levels (additional measures of immune response including specific measures of cellular response will be investigated at some centres. Each will be the subject of a separate related protocol and informed consent for specific study sites and will be conditional upon regulatory and IRB/ethics committee approval before implementation.)

Metastatic renal cancer has a poor prognosis. The median survival overall has been reported to be as low as 6 months and five year survival is <5%. Conventional systemic cytotoxic chemotherapeutic agents and hormonal therapies have little impact on survival and response rates are usually <10%. The wide variations in the natural history of the disease and spontaneous regression rates of up to 6% have led to the investigation of immune mechanisms as a factor influencing responses and outcomes. Biological and immunologic therapies have demonstrated the best response rates with some impact on overall survival. However the management of metastatic renal cancer remains a therapeutic challenge.

Interferon alpha (IFNα) has demonstrated response rates of 8-26% with median survivals of 13 months. Interleukin-2 (IL-2) induces responses in 7-23% of patients with a median survival of 12 months. The benefit of biologic agents has been confirmed by randomised controlled trials, which have shown modest survival benefits with IFNα compared with medroxprogesterone or vinblastine. Motzer (2004) "Prognostic factors for survival of patients with stage IV renal cell carcinoma: Memorial Sloan-Kettering Cancer Center experience." Clin Cancer Res 10 (18 Pt 2): 6302S-3S, in a retrospective analysis of 670 patients in 24 trials of systemic chemotherapy or cytokine therapies, demonstrated longer survival times with cytokine therapy. In the group who were long term survivors, 70% were in trials that involved IFNα and /or IL-2 and 30% had been treated with hormonal or cytotoxic agents.

The initial studies with IL-2 used protocols based on the principles of chemotherapy, using maximum tolerated doses. This was associated with significant renal, cardiac, pulmonary and haemodynamic toxicity, often requiring admission to intensive care wards and limiting utility to a selected subsection of the patient group. Subsequent studies of IL-2 have demonstrated similar efficacy, but with significantly less toxicity, using lower doses administered subcutaneously on an outpatient basis. In a study, comparing high and low-dose IL-2, there was a higher response rate with high dose treatment but this did not translate into survival benefit.

Negrier et al. "Recombinant human interleukin-2, recombinant human interferon alfa-2a, or both, in metastatic renal-cell carcinoma. Groupe Francaise d'Immunotherapie. N Engl J Med 1998; 338; 1272-8 assessed the use of these biologic agents as single agent therapy or combination therapy. They demonstrated response rates of 6.5%, 7.5% and 18.6% for IFNα, IL-2 or the combination, respectively. Although there was a difference in progression free survival, this did not translate into a survival advantage. The rationale for the combination of these agents is that, in vitro, IFNα enhances cell membrane expression of major histocompatibility antigens to which IL-2 activated T-cells can respond.

There is well-documented evidence to suggest that selection and prognostic factors significantly influence outcomes and responses to cytokine therapies. Motzer has assessed the prognostic value of a number of variables in patients with advanced or metatstic renal cell carcinoma. In these patients, low Karnofsky performance status, low haemoglobin level and high corrected serum calcium level indicated a poor prognosis. The median time to death in patients with zero risk factors was 22 months. The median survival in patients with one of these risk factors was 11.9 months and patients with 2-3 risk factors had a median survival of 5.4 months.

Two new drugs have recently been developed for the management of renal cancer sunitinib and sorafenib. Both function by inhibiting multiple receptor kinases. Overall (complete and partial) response rates reported with sunitinib are substantially higher (25.5-36.5%) than reported with sorafenib (2%) though information on time to tumor progression and survival is still maturing.

Safety and immunology data necessary to support coadministration of sorafenib and TroVax® are not available. In view of this and the higher overall response rate reported with sunitinib the latter will be included in this study as an example of a receptor tyrosine kinase inhibitor used in the treatment of renal cancer.

Sunitinib malate is a small molecule that inhibits multiple receptor tyrosine kinase (RTKs), some of which are implicated in tumor growth, pathologic angiogenesis, and metastatic progression of cancer. Sunitinib was evaluated for its inhibitory activity against a variety of kinases (>80 kinases) and was identified as an inhibitor of platelet-derived growth factor receptors (PDGFRα and PDGFRβ), vascular endothelial growth factor receptors (VEGFR1, VEGFR2 and VEGFR3), stem cell factor receptor (KIT), Fms-like tyrosine kinase-3 (FLT3), colony stimulating factor receptor Type 1 (CSF-1 R), and the glial cell-line derived neurotrophic factor receptor (RET). Sunitinib inhibition of the activity of these receptor tyrosine kinase (RTKs) has been demonstrated in biochemical and cellular assays, and inhibition of function has been demonstrated in cell proliferation assays. The primary metabolite exhibits similar potency to sunitinib when compared in biochemical and cellular assays.

The use of single agent sunitinib in the treatment of cytokine-refractory MRCC was investigated in two single-arm, multi-centre studies. All patients enrolled into these studies experienced failure of prior cytokine-based therapy. The primary endpoint for both studies was overall response rate (ORR). Duration of response (DR) was also evaluated.

One hundred and six patients were enrolled into Study 1, and 63 patients were enrolled into Study 2. Across the two studies, 95% of the pooled population of patients had at least some component of clear-cell histology. Patients received 50 mg sunitinib in cycles with 4 weeks on and 2 weeks off. Therapy was continued until the patients met withdrawal criteria or had progressive disease. There were 27 PRs in Study 1 as assessed by a core radiology laboratory for an ORR of 25.5% (95% CI 17.5, 34.9). There were 23 PRs in Study 2 as assessed by the investigators for an ORR of 36.5% (95% CI 24.7-49.6). The majority (>90%) of objective disease responses were observed during the first four cycles; the latest reported response was observed in cycle 10. DR data from Study 1 is premature as only 4 of 27 patients (15%) responding to treatment had experienced disease progression. At the time of the data cut-off, Study 1 was ongoing with 44 of 106 patients (41.5%) continuing treatment, and 11 of the 63 patients (17.5%) enrolled on Study 2 continued to receive sunitinib on continuation protocols.

As of March 2006 no data are available to determine whether sunitinib (or sorafenib) prolongs survival in patients with renal cancer.

Despite recent development of the kinase inhibitors, stage IV renal cell carcinoma is an area of high unmet medical need. The use of vaccines in this area is novel but capitalises on the accepted opinion that immunologic mechanisms may have a part to play in the treatment of this disease.

Primary efficacy objective

- To assess whether the addition of TroVax® to first line standard of care, will prolong survival of patients with locally advanced or metastatic clear cell renal adenocarcinoma when compared to placebo. This will be assessed in the Intent to Treat (ITT) population.

Primary safety objective

- To assess whether the addition of TroVax® to first line standard of care alters the profile of serious and non-serious adverse events, when compared to placebo, in patients with locally advanced or metastatic clear cell renal adenocarcinoma. This will be assessed in the Intent to Treat (ITT) population.

Secondary efficacy objectives

- To compare the proportion of patients with progression free survival at 26 weeks in the TroVax® versus placebo arms. This will be assessed in the Intent to Treat,(ITT) population.

- To compare the tumor response rates, time to response and duration of response between patients treated with TroVax® versus placebo. This will be analysed in the Intent to Treat (ITT) population.

- To assess whether the addition of a minimum of three doses of TroVax® to first line standard of care will prolong survival of patients with locally advanced or metastatic clear cell renal adenocarcinoma when compared to placebo. This will be an exploratory analysis in the Modified Intent to Treat (MITT) population.

- To assess whether TroVax® has an impact on the quality of life as measured by QLQ30 and EuroQOL questionnaires when compared to placebo. This will be analysed in the Intent to Treat (ITT) population.

Study Endpoints

Primary efficacy endpoint

- The survival event rate ratio in the TroVax® arm versus the placebo in the Intent to Treat (ITT) population based on the log of the hazard ratio derived from the Cox Proportional Hazards regression model. A frequentist monitoring approach will be used for evaluating the event ratio.

Primary safety endpoints

- The number of adverse events (serious and non-serious) in the Intent to Treat population in the TroVax® versus the placebo arm.

- The laboratory variables (complete blood count and chemistry panel) in the Intent to Treat (ITT) population in the TroVax® versus the placebo arm.

Secondary efficacy endpoints

- The proportion of patients in the TroVax® versus placebo arms in the Intent to Treat (ITT) population with progression free survival at 26 weeks based on a comparison of baseline and week 26 (+/- 1 week) radiological data and using RECIST criteria. Data will be adjudicated (blinded peer review).

- Tumor response rates according to the investigator's reported interpretation of the radiological reports based on RECIST criteria observed in the Intent to Treat (ITT) population.

- The survival event rate ratio in the TroVax® arm versus the placebo in the Modified Intent to Treat (MITT) population based on the log of the hazard ratio derived from the Cox Proportional Hazards regression model. A frequentist monitoring approach will be used for evaluating the event ratio.

- The Quality of Life score for TroVax® versus placebo as measured by QLQ30 and EuroQOL questionnaires in the Intent to Treat (ITT) and Per Protocol populations.

Immunology endpoint

- Anti-5T4 antibody levels (additional measures of immune response including specific measures of cellular response will be investigated at some centers. Each will be the subject of a separate related protocol and informed consent for specific study sites and will be conditional upon regulatory and IRB/ethics committee approval before implementation.)

Study population

Patients of any ethnic group with histologically proven clear cell renal adenocarcinoma who have had their primary tumor surgically removed and require treatment for locally advanced or metastatic disease. The intent is to include 700 patients split equally between the TroVax® and placebo arms.

Study design

This is an international, randomized, double blind, placebo controlled, parallel group study to investigate whether a minimum of three doses of TroVax® added to first-line standard of care therapy, prolongs the survival of patients with locally advanced or metastatic renal clear cell adenocarcinoma.
The primary endpoint is survival. The study is designed to be pragmatic, limiting additional study related investigations to a minimum. Protocol mandated scans and X-rays are limited to two time points (baseline and week 26) to permit comparison of the percentage of patients with progressive disease at 6 months as a secondary efficacy endpoint. Six months was selected based on review of published literature indicating that progressive disease was commonly observed by 26 weeks in patients with renal cancer. Endpoints such as tumor response by RECIST are considered of secondary importance to survival and will be determined by radiological examinations ordered at the discretion of the investigator based on the clinical status of the patient and will be based the interpretation of the patient's care-team (investigator and local radiologist).
Study enrolment will only commence at each centre once ethics and regulatory approval have been obtained from the relevant authorities.
After signing the study informed consent form and meeting the baseline enrolment criteria patients will be assigned by the investigator (their physician) to one of the following defined first-line standard of care regimens based on what is best for the patient and consistent with local practice:

1. subcutaneous low dose IL-2

2. interferon alpha (excluding pegylated IFNalpha)

3. sunitinib

Only after the standard of care therapy has been decided should the investigator telephone the Interactive Voice Recognition Service (IVRS). Randomization to TroVax® or placebo will be stratified based on the standard of care chosen by the investigator, study prognostic indicators (Motzer score) and geography.
TroVax® is administered at a dose of $1 \times 10^9$ TCID50/ml in 1ml by injection into the deltoid muscle of the upper arm at regular intervals up to 8 weeks apart up to a maximum of 13 doses.
An independent Data Safety Monitoring Board will be responsible for preparing the formal monitoring rules for this study. This parallel-designed study contains a series of planned interim assessments for futility, and to ensure that the planning elements relative to attrition and the primary endpoint remain consistent. A frequentist monitoring approach will be used for evaluating the event rate ratio to ensure that the assumptions are accurate and the sample size continues to be appropriate for assessing superiority. The DSMB may recommend changes to the enrollment target if pretrial assumptions prove inaccurate. These DSMB reviews will be conducted confidentially. Data analysis will not be shared with the sponsor, investigators or any other participant in the study.

Study design

Type of study

This is an international, randomised, double blind, placebo controlled, parallel group study designed to assess whether, when added to first-line standard of care, TroVax® prolongs survival in patients with locally advanced or metastatic renal carcinoma.

The primary endpoint is survival. The study is designed to be pragmatic, limiting additional study related investigations to a minimum. Protocol mandated scans and X-rays are limited to two time points (baseline and week 26) to permit comparison of the percentage of patients with progressive disease at 6 months as a secondary efficacy endpoint. Six months was selected based on review of published literature indicating that progressive disease was commonly observed by 26 weeks in patients with renal cancer. Endpoints such as tumor response by RECIST are considered of secondary importance to survival and will be determined by radiological examinations ordered at the discretion of the investigator based on the clinical status of the patient and will be based the interpretation of the patient's care-team (investigator and local radiologist).

Study enrolment of 700 patients will only commence once ethics and regulatory approval has been obtained from the relevant authorities.

After signing the study informed consent form and meeting the baseline enrolment criteria patients will be assigned by the investigator (their physician) to one of the following defined standard of care regimens based on what is best for the patient and consistent with local practice:

1. subcutaneous low dose IL-2

2. interferon-$\alpha$ (excluding pegylated IFN$\alpha$)

3. sunitinib

Only after the standard of care therapy has been decided should the investigator telephone the Interactive Voice Randomisation Service (IVRS). Randomisation to TroVax® or placebo will be stratified based on the standard of care chosen by the investigator, the study site and prognostic indicators.

An independent Data Safety Monitoring Board will periodically review emerging data. These reviews will be conducted confidentially. Data analysis will not be shared with the sponsor, investigators or any other participant in the study. A frequentist monitoring approach will be used for evaluating the event rate ratio to ensure that the assumptions are accurate and the sample size continues to be appropriate for assessing superiority. The DSMB may recommend changes to the enrollment target if pretrial assumptions prove inaccurate.

Rationale for study design

A randomised, parallel group, double blind design is standard in phase III efficacy studies. Interim statistical analyses conducted by an independent Data Safety Monitoring Board will ensure that the trial can be closed if shown to be futile or resized if it turns out that the assumptions made about the primary endpoint in the control group are inaccurate.

Study sites, duration and recruitment rates

This is an international trial with recruitment across approximately 100 sites. The recruitment rates are estimated to be approximately 0.5 to 4 patients per site per month. Since this is a survival study patients are expected to be on study for a median time of 12 months.

Justification of the proposed dosing regimen

In the TroVax® phase I study four dose levels were studied ($1 \times 10^8$ TCID50/ml, $2 \times 10^8$TCID50/ml, $5 \times 10^8$ TCID50/ml, and $1 \times 10^9$ TCID50/ml) and two different routes of administration, intramuscular and intradermal, were compared. There was no clinically or statistically significant difference in peak immune response though the highest dose produced a slightly earlier antibody response. No difference was observed between the routes of administration in terms of antibody response. All doses and routes were well tolerated with only local injection site reactions which were of similar frequency. In view of a trend to an earlier antibody response the dose of $1 \times 10^9$ TCID50/ml was selected.

In subsequent phase II studies involving >70 patients, a dose level of $1 \times 10^9$TCID50/ml was used and safety, tolerability and immunogenicity were confirmed.

In this study, TroVax®/placebo is administered at weeks 1, 3, 6, 9, 13, 17, 21, 25, 33, 41, 49, 57 and 65. This frequency is influenced by experience gained in phase II studies in patients with renal or colorectal cancer where TroVax® was co-administered with either combination chemotherapy, IL-2 or IFNα.

Study Population

Patient Recruitment

A total of 700 patients with clear cell renal carcinoma will be enrolled in the study. Eligible patients will have had the primary tumor surgically removed.

Patients will receive one of the following defined standards of care:

subcutaneous low dose IL-2

interferon alpha (excluding pegylated IFNα)

sunitinib

The choice of first-line standard of care for each patient will be made by the patient's physician based on normal clinical criteria, local standard of care, and local regulatory and reimbursement status or economic availability. Once treatment is selected, patients will be randomised to TroVax® or placebo.

Patients will be recruited internationally. Patients of all ethnic groups are eligible for the study.

Entry Criteria

Patients who meet the following inclusion criteria and none of the exclusion criteria will be included in this study.

Inclusion criteria

Signed informed consent. The patient must be competent to give written informed consent and comply with the protocol requirements.

Locally advanced or metastatic, histologically proven clear cell renal carcinoma.

Primary tumor surgically removed (some residual advanced primary tumor may remain).

At least four weeks post surgery or radiotherapy (defined from time of randomisation.)

First-line. No prior therapy for renal cancer except surgery or radiotherapy.

Measurable disease.

Aged 18 years or more.

Patient expected to survive a minimum of 12 weeks (i.e. in the opinion of the investigator there is a >90% probability that the patient will survive >12 weeks if treated with the selected standard of care).

Free of clinically apparent autoimmune disease (including no prior confirmed diagnosis or treatment for autoimmune disease including Systemic Lupus Erythematosis, Grave's disease, Hashimoto's thyroiditis, multiple sclerosis, insulin dependant diabetes mellitus or systemic (non-joint) manifestations of rheumatoid disease).

Total white cell count $\geq$ 3 x 109/L and lymphocyte count $\geq$1 x 109/L.

Serum creatinine $\leq$1.5 times the upper limit of normal.

Bilirubin $\leq$ 2 times the upper limit of normal and an SGPT of $\leq$ 4 times the upper limit of normal.

Women must be either post menopausal, or rendered surgically sterile or, if of child bearing potential, must have been practising a reliable form of contraception (oral contraception + a barrier method) for at least three months prior to the first dose of TroVax® and must continue while they are being treated with TroVax®. Men must practise a reliable form of contraception (barrier or vasectomy) while they are being treated with TroVax®.

No acute changes on 12-lead ECG.

Ejection fraction documented as not less than 45% or no clinical suspicion that cardiac ejection fraction is less than 45%.(If clinical suspicion exists the ejection fraction should be measured according to local site procedures).

Karnofsky performance status of $\geq$ 80%.

Exclusion criteria

Cerebral metastases. (Known from previous investigations or clinically detectable).

Previous exposure to TroVax®.

Serious infections within the 28 days prior to entry to the trial.

Known to test positive for HIV or hepatitis B or C.

Life threatening illness unrelated to cancer.

History of allergic response to previous vaccinia vaccinations.

Known allergy to egg proteins.

Known hypersensitivity to neomycin.

Participation in any other clinical trial of a licensed or unlicensed drug within the previous 30 days or during the course of this trial.

Previous malignancies within the last 10 years other than successfully treated squamous carcinoma of the skin or in situ carcinoma of the cervix treated with cone biopsy.

Previous history of major psychiatric disorder requiring hospitalization or any current psychiatric disorder that would impede the patient's ability to provide informed consent or to comply with the protocol.

Oral corticosteroid use unless prescribed as replacement therapy in the case of adrenal insufficiency.

Ongoing use of agents listed in locally approved prescribing information as causing immunosuppression.

Prior history of organ transplantation.

Pregnancy or lactation.

Withdrawal Criteria

In accordance with applicable regulations, a patient has the right to withdraw from the study at any time and for any reason without prejudice to his or her future medical care by the physician or at the institution.

If a patient is withdrawn from treatment with TroVax®/placebo because of an adverse event (AE), the event will be followed up until it has resolved or has stabilized. Because this is a survival study patients should continue to be followed until death to document subsequent treatment and survival status

In addition to AEs, other reasons for removal of patients from the study would be the patient's withdrawal of consent. Should this happen, since this is a survival study, the patient's physician must request consent from the patient for survival follow up.

Withdrawal from the study, and reason for withdrawal, must be documented in the CRF.

Because the primary endpoint of this study is survival and all randomised patients will be included in the primary or secondary endpoint analysis patients who wish to withdraw from all other study related procedures for any reason should be asked whether they would consent to follow up limited to documenting their subsequent management and survival status. If they agree, a new informed consent form should be used to document consent to such follow up treatment plan and methods. The Study Schedule is set out below:

EP 2 386 062 B1

| | Baseline | Wk 1 | Wk 2 | Wk 3 | Wk 4 | Wk 5 | Wk 6 | Wk 7 | Wk 8 | Wk 9 | Wk 10 | Wk 11 | Wk 12 | Wk 13 | Wk 14 | Wk 15 | Wk 16 | Wk 17 | Wk 18 |
|---|---|---|---|---|---|---|---|---|---|---|---|---|---|---|---|---|---|---|---|
| | TroVax®/Placebo day 1/wk | | X | | X | | X | | | X | | | X | | | | X | | | X |
| Patients only receive one of these treatments | Patients receive only one of the following treatments | | | | | | | | | | | | | | | | | | | |
| | IL-2 Treatment days 1-5 each wk | | X | X | X | X | X | X | Continue with 6 weeks subcutaneous IL-2 followed by 2 weeks without IL-2 every 8 weeks until tumour progression or week 46 (whichever is first) | | | | | | | | | | | |
| | OR | | | | | | | | | | | | | | | | | | | |
| | IFNα | | Subcutaneous IFNα day 1, 3 and 5 of each week until tumour progression (refer to nationally approved prescribing information or institutional guidelines of use of IFNα for renal cancer). | | | | | | | | | | | | | | | | | |
| | OR | | | | | | | | | | | | | | | | | | | |
| | sunitinib | | X | X | X | X | | | Continue with 4 weeks on sunitinib then 2 weeks without sunitinib every 6 weeks until tumour progression. (see nationally approved sunitinib prescribing information) | | | | | | | | | | | |

**Patients receive all the following procedures**

| Procedure | | | | | | | | | | |
|---|---|---|---|---|---|---|---|---|---|---|
| Consent form | X | | | | | | | | | |
| Randomisation | | X | | | | | | | | |
| Medical History | X | | | | | | | | | |
| Physical examination | X | X | | X | | X | | | | |
| Blood for immuno (10ml) | X | | X | | X | | | | | |
| Weight, BP, Pulse, Temp. | X | X | X | X | X | X | X | X | | |
| CBC/Diff/Plts | X | X | X | X | X | X | X | X | | |
| Chemistry Panel | X | X | X | X | X | X | X | X | | |
| CT or MRI Chest, Abd, Pelvis | X | | | | | | | | | |
| 12 lead ECG | X | | | | | | | | | |
| Echocardiogram* | X | | | | | | | | | |
| Karnofsky | X | | X | X | X | X | X | X | | |
| Tumour histopathology | X | | | | | | | | | |
| Pregnancy Test (If... — Prior to TroVax®/placebo if any possibility of pregnancy) | X | | | | | | | | | |

| | | | | | | | | | | | | | | | | |
|---|---|---|---|---|---|---|---|---|---|---|---|---|---|---|---|---|
| applicable) | | | | | | | | | | | | | | | | |
| QOL | X | | | | | X | | | X | | | X | | | X | |
| Concomitant Therapy | Record on each visit that patient receives TroVax®/placebo | | | | | | | | | | | | | | | |
| AEs | Throughout the study while patient receiving TroVax®/placebo and 30 days after | | | | | | | | | | | | | | | |
| Subsequent renal cancer Rx | Record other renal cancer treatment once patient is not receiving TroVax®/placebo | | | | | | | | | | | | | | | |
| Survival status / date of death | Record on each visit that patient receives TroVax®/placebo and every 12 weeks thereafter. If patient does not return to clinic seek survival status and date of death as permitted by patient consent | | | | | | | | | | | | | | | |

If clinically indicated * including LDH at baseline

Timing of all TroVax® injections +/- 3 days. Timing of all laboratory and clinical observations must remain the same relative to TroVax®. Week 26 scan may vary by +/- 7 days.

| | Wk 19 | Wk 20 | Wk 21 | Wk 22 | Wk 23 | Wk 24 | Wk 25 | Wk 26 | Wk 27 | Wk 28 | Wk 29 | Wk 30 | Wk 31 | Wk 32 | Wk 33 | Wk 34 | Wk 35 | Wk 36 | Wk 37 |
|---|---|---|---|---|---|---|---|---|---|---|---|---|---|---|---|---|---|---|---|
| TroVax®/Placebo day 1 | | | X | | | | X | | | | | | | X | | | | | |
| Patient | Patients receive only one of the following treatments | | | | | | | | | | | | | | | | | | |

EP 2 386 062 B1

| | | |
|---|---|---|
| IL-2 Treatment days 1-5 each wk | Continue with 6 weeks subcutaneous IL-2 followed by 2 weeks without IL-2 every 8 weeks until tumour progression or week 46 (whichever is first) | |
| OR | | |
| IFNα | Subcutaneous IFNα day 1, 3 and 5 of each week until tumour progression (refer to nationally approved prescribing information or institutional guidelines of use of IFNα for renal cancer). | |
| OR | | |
| sunitinib | Continue with 4 weeks on sunitinib then 2 weeks without sunitinib every 6 weeks until tumour progression. (see nationally approved sunitinib prescribing information) | |

**Patients receive all the following procedures**

| Procedure | Week markings |
|---|---|
| Physical examination | X (≈wk 30), X (≈wk 10) |
| Weight, BP, Pulse, Temp | X (≈wk 50), X (≈wk 30), X (≈wk 20), X (≈wk 10) |
| CBC/Diff/Plts | X (≈wk 50), X (≈wk 30), X (≈wk 20), X (≈wk 10) |
| Chemistry Panel | X (≈wk 50), X (≈wk 30), X (≈wk 20), X (≈wk 10) |
| CT or MRI Chest, Abd, Pelvis | X (≈wk 30) |
| Karnofsky | X (≈wk 50), X (≈wk 30), X (≈wk 20), X (≈wk 10) |

| QOL | | | X | | | X | | | X | | | X | | | |
|---|---|---|---|---|---|---|---|---|---|---|---|---|---|---|---|
| Pregnancy Test (if applicable) | Prior to TroVax®/placebo if any possibility of pregnancy | | | | | | | | | | | | | | |
| Concomitant Therapy | Record on each visit that patient receives TroVax®/placebo | | | | | | | | | | | | | | |
| AEs | Throughout the study while patient receiving TroVax®/placebo and 30 days after | | | | | | | | | | | | | | |
| Subsequent renal cancer Rx | Record other renal cancer treatment once patient is not receiving TroVax®/placebo | | | | | | | | | | | | | | |
| Survival status / date of death | Record on each visit that patient receives TroVax®/placebo and every 12 weeks thereafter. If patient does not return to clinic seek survival status and date of death as permitted by patient consent | | | | | | | | | | | | | | |

Timing of all TroVax® injections +/- 3 days. Timing of all laboratory and clinical observations must remain the same relative to TroVax®. Week 26 scan may vary by +/- 7 days.

EP 2 386 062 B1

| | wk 38 | wk 39 | wk 40 | wk 41 | wk 42 | wk 43 | wk 44 | wk 45 | wk 46 | wk 47 | wk 48 | wk 49 | wk 50 | wk 51 | wk 52 | wk 53 | wk 54 | wk 55 | wk 56 |
|---|---|---|---|---|---|---|---|---|---|---|---|---|---|---|---|---|---|---|---|
| TroVax®/Placebo day 1 | | | | X | | | | | | | X | | | | | | | | |
| Patients receive only one of the following treatments | | | | | | | | | | | | | | | | | | | |
| IL-2 Treatment days 1-5 each wk | Continue with 6 weeks subcutaneous IL-2 followed by 2 weeks without IL-2 every 8 weeks until tumour progression or week 46 (whichever is first). | | | | | | | | | No further IL-2 | | | | | | | | | |
| OR | | | | | | | | | | | | | | | | | | | |
| IFNα | Subcutaneous IFNα day 1, 3 and 5 of each week until tumour progression (refer to nationally approved prescribing information or institutional guidelines of use of IFNα for renal cancer). | | | | | | | | | | | | | | | | | | |
| OR | | | | | | | | | | | | | | | | | | | |
| sunitinib | Continue with 4 weeks on sunitinib then 2 weeks off sunitinib every six weeks until tumour progression. (see nationally approved sunitinib prescribing information) | | | | | | | | | | | | | | | | | | |
| Patients receive all the following procedures | | | | | | | | | | | | | | | | | | | |
| Physical examination | | | | X | | | | | | | X | | | | | | | | |

Patients only receive one of these treatments

| | | | | X | | | | | | X | | | | | | | |
|---|---|---|---|---|---|---|---|---|---|---|---|---|---|---|---|---|---|
| Weight, BP, Pulse, Temp | | | | X | | | | | | X | | | | | | | |
| CBC/Diff/Plts | | | | X | | | | | | X | | | | | | | |
| Chemistry Panel | | | | X | | | | | | X | | | | | | | |
| Karnofsky | | | | X | | | | | | X | | | | | | | |
| QOL | | | | X | | | | | | X | | | | | | | |
| Pregnancy Test (if applicable) | Prior to TroVax®/placebo if any possibility of pregnancy |
| Concomitant Therapy | Record on each visit that patient receives TroVax®/placebo |
| AEs | Throughout the study while patient receiving TroVax®/placebo and 30 days after |
| Subsequent renal cancer Rx | Record other renal cancer treatment once patient is not receiving TroVax®/placebo |
| Survival status / date of death | Record on each visit that patient receives TroVax®/placebo and every 12 weeks thereafter. If patient does not return to clinic seek survival status and date of death as permitted by patient consent |

Timing of all TroVax® injections +/- 3 days. Timing of all laboratory and clinical observations must remain the same relative to TroVax®. Week 26 scan may vary by +/- 7 days.

| | Wk 57 | Wk 58 | Wk 59 | Wk 60 | Wk 61 | Wk 62 | Wk 63 | Wk 64 | Wk 65 | Wk 66 | Subsequent weeks |
|---|---|---|---|---|---|---|---|---|---|---|---|
| TroVax®/Placebo day 1 | X | | | | | | | | X | | |
| **Patients receive only one of the following treatments** | | | | | | | | | | | |
| IL-2 Treatment days 1-5 each wk | No further IL2 | | | | | | | | | | |
| **OR** | | | | | | | | | | | |
| IFNα | Subcutaneous IFNα day 1, 3 and 5 of each week until tumour progression (refer to nationally approved prescribing information or institutional guidelines for use of IFNα for renal cancer). | | | | | | | | | | |
| **OR** | | | | | | | | | | | |
| sunitinib | Continue with 4 weeks on sunitinib then 2 weeks off sunitinib every six weeks until tumour progression. (see nationally approved sunitinib prescribing information) | | | | | | | | | | |
| **Patients receive all the following procedures** | | | | | | | | | | | |
| Physical examination | X | | | | | | | X | | | Continue follow-up for survival |

| | | | | | | | | | | |
|---|---|---|---|---|---|---|---|---|---|---|
| **Weight, BP, Pulse, Temp** | X | | | | . | | | | X | | Record subsequent therapy for renal cancer |
| **CBC/Diff/Plts** | X | | | | | | | | X | | |
| **Chemistry Panel** | X | | | | | | | | X | | |
| **Karnofsky** | X | | | | | | | | X | | |
| **QOL** | X | | | | | | | | X | | |
| **Pregnancy Test (if applicable)** | Prior to TroVax®/placebo if any possibility of pregnancy | | | | | | | | | | |
| **Concomitant therapy** | Record on each visit that patient receives TroVax®/placebo | | | | | | | | | | |
| **AEs** | Throughout the study while patient receiving TroVax®/placebo and 30 days after | | | | | | | | | | |
| **Subsequent renal cancer Rx** | Record other renal cancer treatment once patient is not receiving TroVax®/placebo | | | | | | | | | | |
| **Survival status / date of death** | Record on each visit that patient receives TroVax®/placebo and every 12 weeks thereafter. If patient does not return to clinic seek survival status and date of death as permitted by patient consent | | | | | | | | | | |

Timing of all TroVax® injections +/- 3 days. Timing of all laboratory and clinical observations must remain the same relative to TroVax®. Week 26 scan may vary by +/- 7 days.

EP 2 386 062 B1

Allocation of Treatments And Randomisation Procedures

Treatment (TroVax® or placebo) will be allocated based on stratified randomisation. The primary objective of stratification will be to ensure that the distribution of first-line standard of care treatment is balanced between the two study arms. Secondary objectives of stratification will be to establish balance between the treatment arms with regard to a prognostic index (Motzer score) and geography.

Motzer et al demonstrated in a series of 670 patients with advanced renal cell carcinoma that survival correlated with five prognostic factors: Karnofsky performance status (<80%), high lactate dehydrogenase (LDH) level (>1.5 times the upper limit of normal), low haemoglobin level (less than the lower limit of the gender normal), high corrected serum calcium level (>10 mg/dL), and absence of nephrectomy. The higher the number of positive factors the worse the prognosis. Inclusion criteria for this study require a baseline Karnofsky performance status ≥80% and prior excision of the primary tumor. During the randomisation procedure the patient's haemoglobin level (plus gender), LDH and serum calcium will be requested to ensure that the treatment arms are balanced with regard to these prognostic variables.

A telephone based interactive voice responsive system will be used. Patients will be registered into the study using an Interactive Voice Responsive System (IVRS). Treatment allocation (TroVax® or placebo) and patient registration will only occur after the Investigator has registered the standard of care therapy allocated to the patients and confirmed that the patient meets all inclusion / exclusion criteria. All randomised patients will be included in Intent to Treat (ITT) analyses.

Instruction on access and use of the IVRS service including local telephone access number, script of the randomisation questions in local language and help desk numbers will be issued separate from the protocol.

Study Medication Administration

Patients included in this trial should receive TroVax® or placebo plus one of the following first-line standards of care treatment options: IL-2 (low dose), interferon $\alpha$ or sunitinib. No other form of immunotherapy, chemotherapy, or radiotherapy should be administered between entering the study and tumor progression. Other concurrent medication may be used as detailed in "Other Concurrent Treatments" below. Following tumor progression patients may receive whatever chemotherapy, radiotherapy, cytokine therapy or other therapy is indicated for further management or palliation of the tumor. All such therapy should be recorded on the patient's case report form as the patient continues to be followed for survival.

Administration of TroVax®/Placebo

Prior to administering the vaccine, obtain the prospective patient's vaccination history and determine whether the individual had any previous reactions to any vaccine including TroVax®.

All immunisations of TroVax®/placebo will be given by intramuscular injection into the deltoid muscle of the upper arm.

All patients will receive the treatment in a side-room away from contact with other patients. The formulation will be delivered to this side-room. TroVax®/ Placebo are presented as lyophilised material. Detailed instructions will be provided to the pharmacist for reconstitution. TroVax® must be re-suspended by adding 1.2 mL of water for injection. The resulting solution will appear opalescent. One mL volume of the solution is then withdrawn into a syringe and injected into the patient. The injection will either be drawn up at the bedside by the person administering the dose, or in the pharmacy and delivered to the bedside in a syringe depending upon local circumstances. Prior to injection the check number of the dose must be confirmed, using IVRS, by either the pharmacist or another responsible individual.

Under no circumstances must the reconstituted material be allowed to stand for more than two hours at room temperature. If this does occur, the material must be rejected and IVRS notified.

The skin will be swabbed with ethanol and the injection will be given intramuscularly. Following this, the injection site will be covered with an occlusive bandage. This bandage will be removed before the patient is discharged from hospital.

Please note: The maximum immunological response to TroVax® dose not usually occur until the patient has received at least three injections. Disease stabilisation or late tumor responses have been reported with various cancer vaccines. It is not established whether continuing TroVax® despite early progression will confer therapeutic benefit. If tumor progression is observed but the patient is tolerating TroVax®/placebo and their performance status remains at a Karnofsky score >60% they should be requested to continue receiving TroVax®/placebo until they have received a minimum of eight injections. Continuation beyond this point is permitted at the discretion of the investigator and patient.

Patients should remain under medical observation for one hour following injection with TroVax®/placebo.

Adequate treatment provisions, including epinephrine injection (1:1000), should be available for immediate use should an anaphylactic reaction occur.

All healthcare staff handling TroVax® or materials contaminated by it must wear an apron, gloves, mask, and protective goggles. All materials potentially contaminated with TroVax® e.g. syringes, swabs, bandages, must be destroyed by incineration, or local equivalent, in accordance with hospital policy on genetically modified materials. Certificates of Destruction, or equivalent, must be completed for the used and unused vials, and copies maintained in the Trial File.

Administration of IL-2

IL-2 (Chiron or locally approved manufacturer) will be given by subcutaneous injection. The lyophilised material (22 million units) must be reconstituted in 1.2 mL of diluent after which it will have a shelf life of 48 hours when kept refrigerated at 2-8°C. The dosage schedule will be an initial dose of 250,000U/Kg/dose (with an upper limit of 22 million units/dose) for 5 days out of 7 in week 1 of each cycle followed by 125,000U/kg/dose (with an upper limit of 11 million units/dose) for 5 days in each of weeks 2-6 of each cycle. There will then be a two week recovery period before the next cycle of IL-2 commences. Once reconstituted a vial may be used for two injections when these are given on consecutive days. The dose used should be recorded in the Case Report Form.

Administration of IFNα

IFNα will be administered once a day as a subcutaneous injection three times per week on days 1, 3 and 5 of each week. (Note: Pegylated IFNα is not included as a standard of care option in this protocol. No safety or immunological activity data are currently available on the concomitant use of TroVax® and pegylated IFNα).

Unless tumor progression is noted the patient should be treated for a minimum of 12 weeks. Treatment may be continued until tumor progression at the discretion of the investigator.

Doses of IFNα used by different treatment centres depend on local Regulatory Authority approved label text, and manufacturer. The dose used in this study should reflect local standard of care but should be targeted between 9 million International Units (IU) and 18 million IU three times per week. Lower doses should be used during the first (and depending on final target dose) the second week. The actual schedule used will be recorded on the Case Report Form.

For further information on IFNα please refer to the nationally approved Package Insert or Summary of Product Characteristics produced by the local license holder.

For evaluation of patients for clinical benefit from the treatment please see study schedule. Patients who are benefiting from treatment are eligible for further treatment Thereafter, therapy will continue until criteria for progressive disease are met or up to an additional 12 months.

Administration of sunitinib

Sunitinib capsules are supplied as printed hard shell capsules containing sunitinib malate equivalent to 12.5 mg, 25 mg or 50 mg of sunitinib and should be handled according to the manufacturers instructions. The recommended dose of sunitinib for advanced Renal Cell Cancer is one 50 mg oral dose taken once daily, on a schedule of 4 weeks on treatment followed by 2 weeks off. Sunitinib may be taken with or without food. The schedule used should be recorded in the Case Report Form.

Treatment should continue until tumor progression or until unacceptable toxicity occurs.

Administration of other concurrent treatments

All other concurrent medications will be recorded in detail in the CRF during the treatment. This information may be used to assist interpretation of any report adverse events. If a patient has discontinued TroVax®/placebo and other renal cancer treatments are used, then a simple checklist in the CRF will be used to record the type of treatment; this information may be used to assist interpretation of survival data and management of the patient following the selected standard of care therapy.

Medication intended to relieve symptoms will be prescribed at the discretion of the Investigator and recorded in the Case Report Form (CRF). Medications prescribed by the patient's family practitioner will also be noted in the CRF. The patients should also keep a record of any over the counter medicines consumed and these should be noted in the CRF.

Therapies considered necessary for the subject's well being may be administered at the discretion of the investigator. These will be recorded in the Case Report Form.

Supportive care to mitigate known adverse events or complications of concomitant standard of care may be administered at the physician's discretion including antipyretics, non-steroidal anti-inflammatories, anti-emetics etc. Oral, intramuscular or intravenous steroids should not be used except where required to manage life threatening emergencies. Supportive care will be reported in the Case Report Form.

Management of Disease Progression

If disease progression is noted during the study, and other anticancer medications are required, the IL-2, IFNα, or sunitinib should be stopped. The selection of subsequent antitumor therapy is not specified by this protocol and is at the discretion of the patient and his or her physician.

In the event of tumor progression the patients should remain within the study (unless they request to withdraw). This is for two reasons:

This is a survival study and patients need to be followed for survival data.

The maximum immunological response to TroVax® does not usually occur until the patient has received at least three injections. Disease stabilisation or late tumor responses have been reported with various cancer vaccines. It is not established whether continuing TroVax® despite early progression will confer therapeutic benefit. Therefore if tumor progression is observed but the patient is tolerating TroVax®/placebo and their performance status remains at a Karnofsky score >60% they should be requested to continue receiving TroVax®/placebo until they have received a minimum of eight injections of the study preparation. Continuation on study beyond this point to receive all TroVax®/placebo injections is permitted at the discretion of the investigator or patient.

Specific Procedures

Screening and selection procedures

A screening log must be maintained for all patients screened for entry to the study including, if applicable, the reason for not entering the study.

Inclusion/exclusion criteria are listed in the section titled Entry Criteria (above) and the study schedule.

Imaging/diagnostic

Within 2 weeks of screening, and prior to receiving study drug metastases will be documented using chest, abdominal and pelvic CT scans according to defined guidelines contained in a Site Operations Manual. This will enable a possible independent review at a later time. An MRI or CT scan of the brain will also be obtained if there is a clinical suspicion of cerebral metastases.

Clinical and laboratory/diagnostic

For screening, these are required within 14 days before the first TroVax®/Placebo injection:

- History and physical examination, including height, weight, and vital signs.

- Karnofsky performance status.

- Quality of life (QLG130,EuroQOL) will be evaluated.

- 12 lead EGC (for all patients) and Echocardiogram only if clinically indicated

- Clinical pathology tests (Full blood count with differential white cell and platelet counts, urea and electrolytes, liver function tests (total bilirubin, AST, ALT, alkaline phosphatase), serum proteins, calcium, phosphate, uric acid and creatinine). In addition, at baseline LDH must be measured.

- Pregnancy test (for women of reproductive potential - including those whose last menstrual period was within the last two years). At screening this will be a serum test but at all other time points this will be a urine test.

- If available, tumor tissue from earlier biopsies will be obtained. (To be batched and tested at a later date for the presence of tumor antigens.)

All clinical laboratory tests will be conducted by a suitably qualified central laboratory.

Samples for immunology

10 mL blood samples will be required. These samples are to be placed in a heparinised blood collection tube and are to be processed immediately by a suitably qualified central laboratory. The samples will then be analysed by Oxford BioMedica, or designee, according to their SOPs.

Study materials

TroVax® /Placebo

TroVax® /Placebo will be supplied by Oxford BioMedica Ltd.

Packaging and labelling and additional information. Packaging and labelling will be in accordance with Good Manufacturing Practice (GMP) for clinical trials. Each vial will bear a label conforming to national regulations for an Investigational Medicinal Product. The outer carton labelling will also bear a label conforming to national regulations for an Investigational Medicinal Product.

Investigators and pharmacists should note that the clinical trial supplies may only be used for the clinical trial for which they are indicated. They must not be employed for any other trial, whether of TroVax® or not, or for any other clinical use.

Additional information may be found in the current version of the Investigators Brochure.

Storage and disposition of study medications

TroVax®/placebo must be stored in a locked fridge between 2°C to 8°C (36°F to 46°F).in the hospital pharmacy, or other comparable secure location. It must be stored in such a way that it cannot be mixed up or confused with other medications, be they clinical trial supplies or medicines for routine clinical use.

Dispensing will be documented by completing a log with the date of dispensing and the patient details. Used vials should be stored in labelled biohazard bags or containers prior to reconciliation by the trial monitor.

At each visit, the clinical trial monitor will review the drug-dispensing log and reconcile it with the unused vials (if available due to local procedures). All unused vials will be destroyed on site in accordance with procedures for destruction of genetically modified waste and destruction will be documented appropriately. A copy of the Certificate of Destruction will be lodged in the site Trial File.

Precautions/overdose

TroVax® is contraindicated in patients who have previously had hypersensitive reactions to TroVax®, vaccinia vaccinations, egg proteins or neomycin. Patients should remain under medical observation for one hour following injection with TroVax. Adequate treatment provisions, including epinephrine injection (1:1000), should be available for immediate use should an anaphylactic reaction occur. TroVax® is also contraindicated in patients who are pregnant or lactating.

Although highly unlikely, it is possible that an autoimmune response against the pituitary or gut might occur since these organs showed sporadic low level staining for 5T4 in *in vitro* experiments. Studies in over 100 patients receiving approximately 450 doses of TroVax® have not indicated any laboratory or clinical signs or symptoms suggestive of compromised pituitary function. However, the Investigators should be aware of the preclinical finding.

All healthcare staff handling TroVax® or materials contaminated by it must wear an apron, gloves, a mask and protective goggles. Pregnant healthcare staff must not handle either TroVax® or materials contaminated with TroVax®.

No cases of TroVax® overdose have been reported. No active medical intervention is know to be required in the event of overdose. The patient should be observed for as long as is considered appropriate by the investigator/physician based on the patient's clinical condition and supportive care given if required.

IL-2

II-2 is available commercially from Chiron or a local manufacturer. The lyophilised material (22 million units) must be reconstituted in 1.2 mL of diluent after which it will have a shelf life of 48 hours when kept refrigerated.

Current prescribing information should be reviewed prior to administering IL-2.

IFNα

IFNα is available commercially from a number of manufactures. Only commercially available material approved by the competent national regulatory authority should be used in this study

IFNα may be supplied in single use prefilled syringes or in multiuse prefilled "pens". Patients will be instructed to self administer the IFNα in accord with approved package insert and patient information leaflet by appropriately qualified medical, nursing or pharmacy staff. Reconstitution is not required.

IFNα should be stored at 2° to 8°C (36°F to 46°F).

Current prescribing information should be reviewed prior to administering IFNα.

Sunitinib

Sunitinib is supplied as 12.5mg, 25mg and 50mg capsules which should be administered according to the manufacturer's instructions (Pfizer).

Other Study Supplies

Case report forms (CRFs) will be used in this study (see Data Collection section below). Quality of life questionnaires EuroQOL and QLQ30 and laboratory kits will also be supplied. The Principal Clinical Investigator and Co-Investigators must keep all CRF supplies, both completed and blank, in a secure place.

Adverse Events

Adverse Event Definition

An adverse event is any untoward medical occurrence in a patient or clinical investigation subject administered a pharmaceutical product and which does not necessarily have to have a causal relationship with the treatment. All adverse events must be described in the appropriate section of the CRF and their severity and putative relationship to the study medication noted. Definitions of severity are as follows:

Mild: does not interfere with the conduct of the study, resolves spontaneously, does not need medication or any other therapy.

Moderate: requires treatment, interferes temporarily with the conduct of the study.

Severe: forces withdrawal from the study

Serious: death, life threatening, requires or prolongs hospitalisation, results in persistent or significant disability/incapacity, overdose, or is a congenital anomaly/birth defect

Definitions of relationship to study medication are as follows:

Unrelated: bears no relation to timing of medication, similar to symptoms or signs expected in the disease process, does not recur on re-challenge.

Possibly: bears relation to timing of medication, similar to symptoms or signs expected in the disease process, does not recur on re-challenge.

Probably: bears clear relation to timing of medication, distinct from symptoms or signs expected in the disease process, does not recur on re-challenge.

Definitely: bears clear relation to timing of medication, distinct from symptoms or signs expected in the disease process, recurs on re-challenge.

Adverse events may also be expected or unexpected. Adverse events are to be considered expected if listed in the Investigator Brochure.

Serious Adverse Event (SAE) and Serious Adverse Reaction (SAR) Definition

Investigators are required to notify Oxford BioMedica's pharmacovigilance service provider (PAREXEL) immediately if a patient has a reportable serious adverse event. A serious adverse event (SAE) is defined by ICH-GCP as:

Death (death due to progressive renal cancer is the primary endpoint of this study and should not be reported as an adverse event unless in the opinion of the investigator the study medication (TroVax®/placebo) may possibly, probably or definitely have contributed to or hastened death)

Life threatening

Requires or prolongs hospitalisation

Results in persistent or significant disability/incapacity

Congenital anomaly/birth defect

Other medically important condition starting or worsening during the study.

The investigator must also complete as much as possible of the serious adverse event form in the Case Report Form (CRF) and transfer it to Oxford BioMedica's pharmacvigilance service provider (PAREXEL) not later than 24 hours after the even becomes known to the investigator or his/her staff.

As further information or follow up information becomes available the investigator should document this and amend any previous report if appropriate. This information should be transferred to Oxford BioMedica's pharmacovigilance service provider (PAREXEL) using the serious adverse event form in the CRF.

PAREXEL will report all serious, related, and unexpected adverse events to all relevant Regulatory Authorities in accordance with local regulations.

Further instructions on the documentation and transfer of information to permit full compliance with national and international pharmacovigilance requirements and Good Clinical Practice together with training for investigator staff will be provided separate to this protocol.

General Requirements

This study will utilize the Common Terminology Criteria for Adverse Events Version 3 to determine the severity of the reaction for adverse event reporting.

Reporting requirements and procedures depend upon:

whether agents are suspected of causing the adverse event,

whether the possibility of such an adverse event was reported in the protocol, consent form, or manufacturer's literature (expected or unexpected adverse event),

the severity or grade of the adverse event.

Withdrawals due to Adverse Events

If a patient is withdrawn from treatment because of an adverse event (AE), the patient will be followed up until the AE is resolved or has stabilised. Because the primary endpoint of this study is survival the patient will continue to be followed for survival status even if trial therapy was withdrawn.

Withdrawal from the study, and reason for withdrawal, must be documented in the CRF.

Since the primary endpoint of this study is survival and all randomised patients will be included in the analysis of the primary endpoint. Patients who wish to withdraw from all other study related procedures should be asked whether they would consent to allow follow up limited to establishing their survival status. If they agree, a new consent form to document this consent but withdrawal from all other study procedures should be completed.

Pregnancy

Patients should be advised that they or their partner should avoid becoming pregnant during the study.

Patients of reproductive potential should be taking contraceptive measures as required by the relevant inclusion criterion (as stated above).

If a patient does become pregnant she should immediately inform the investigator who should document this on the adverse events page of the CRF. The Investigator should provide necessary counselling for the patient. The Investigator should follow the pregnancy to its conclusion. Spontaneous abortion or foetal abnormality or abnormal birth should be reported as serious adverse events as described above.

Management of Toxicity

The NCI Common Terminology Criteria for Adverse Events v3.0 (CTCAE) will be utilized (see Appendix A). Toxicity will be evaluated on every patient visit.

All toxic events should be managed with optimal supportive care, including transfer to the Intensive Care Unit if appropriate.

TroVax®/Placebo management of toxicity

No dose reductions of TroVax®/placebo are permitted. Paracetamol/acetaminophen may be used to manage transient pyrexia or local discomfort following injection If the patient is unable to tolerate TroVax®/placebo at the protocol dose TroVax®/placebo should be discontinued but the patient should continue to be followed for survival data.

Standard of care management of toxicity

Toxicity associated with standard of care therapy should be managed according the nationally approved Package Insert or Summary of Product Characteristics and accepted medical practice. Dosage may be reduced or withdrawn at the discretion of the Investigator.

Data Management and Statistical Analysis

Overview of the Study Design

The DSMB will be responsible for preparing the formal monitoring rules for this study; a general overview of the monitoring program is described in this section of the protocol. Oxford BioMedica will provide guidance to the DSMB, however the Board is an independent body and will be charged with preparing the formal monitoring and stopping rules for the study. This parallel-designed study contains a series of planned interim assessments for futility, and to ensure the planning elements relative to attrition and the primary endpoint remain consistent. The initial interim assessment will take place after 50 patients (25 patients per arm or ~7% of the target population) have been randomised and followed for 8 weeks when the blood sampling for 5T4 antibodies following the third dose of TroVax® is scheduled to be performed. The intra-treatment group adverse event profiles, rates of attrition, and antibody response will be evaluated by the DSMB. Sample size estimates for this study are predicated on a one year survival.

Sample Size Estimates

Estimates were prepared to detect an absolute difference of ~11% in survival at 1-year (base proportions: 50% to 61 %); estimates are presented below in Table A.

Table A: Estimates Based on Overall Survival

| Power | Total Sample Size (N) | Total Required Events | Alpha | Beta | Proportio n Surv. (S1) | Proportio n Surv. (S2) | Hazard Ratio |
|---|---|---|---|---|---|---|---|
| 0.80 | 691 | 309 | 0.05 | 0.2 | 0.500 | 0.605 | 0.725 |

A total sample size of ~700 patients (split equally between the two groups), or 309 events, achieves 80% power to detect a hazard rate of 0.725 when the proportions surviving in each group are 0.500 and 0.605 at a significance level of 0.05 using a two-sided test. These estimates represent the initial framework for monitoring based on the log of the hazard ratio from the Cox Proportional Hazards regression model without adjusting for covariates.

Report Definitions

Power is the probability of rejecting a false null hypothesis.
Events are the number of deaths (from whatever cause) that must occur in each group.
Alpha is the probability of rejecting a true null hypothesis.
Beta is the probability of accepting a false null hypothesis.
S1 is the proportion surviving in group 1, S2 is the proportion surviving in group 2.

HR is the hazard ratio. It is calculated using Log(S2)/Log(S1).

This sample size would also be appropriate for detecting a minimum difference in median survival of ~11.3 weeks, based on exponential survival times (Table B). Details used in preparing this estimate are presented below.

Table B: Comparing Median Survival (H0: Theta1 = Theta2. Ha: Theta1 <> Theta2)

| Power | N1 | N2 | Allocation Ratio | Alpha | Beta | Theta1 | Theta 2 | Theta1/ Theta2 |
|---|---|---|---|---|---|---|---|---|
| 0.80000 | 350 | 350 | 1.00000 | 0.05000 | 0.20000 | 48.0 | 59.3 | 0.80902 |

Report Definitions

Power is the probability of rejecting a false null hypothesis.

N1 is the number of failures needed in Group 1, N2 is the number of failures needed in Group 2.

Alpha is the probability of rejecting a true null hypothesis.

Beta is the probability of accepting a false null hypothesis.

Theta1 is the Mean Life in Group 1, Theta2 is the Mean Life in Group 2.

Patient Populations

The Intent to Treat (ITT) population will include all patients who are randomised.

The Modified Intent to Treat (MITT) population, will include all patients who receive three or more injections, or experience an adverse event directly attributable to the study medication resulting in discontinuation, prior to the third injection. Patients who fail to successfully receive three injections for reasons not directly associated with the study medication will not be included in this population.

The Per Protocol (PP) population includes only patients who met the inclusion and exclusion criteria and were treated in accord with the protocol requirements.

The primary efficacy analysis will be carried out using the ITT population. However, an exploratory analysis of the primary efficacy parameter will also be carried out using MITT population and the PP population.. All safety analyses will be carried out using the Intent to Treat population.

Monitoring of the Primary Endpoint

The DSMB may recommend stopping the trial early if presented with overwhelming evidence of efficacy.

Evidence would be deemed "overwhelming" if the one-sided P-value in favour of the active treatment derived from the Cox Proportional Hazards time-to-death model is less than 0.01%. The overall effect of treatment must also be considered clinically plausible by the DSMB.

P-values will be adjusted to maintain an overall one-sided P-value of 2.5% using the alpha-spending approach of Lan and Demets (Lan KKG and DeMets DL (1983) Discrete sequential boundaries for clinical trials. Biometrika 70: 659-663).

The DSMB will review at each meeting the number of patients lost to follow-up. If the number of patient lost to follow-up is high enough to compromise the objectives of the study the DSMB may either recommend terminating the study on the grounds that it will not effectively address its objective or alternatively resizing the study to permit the objective of the study to be appropriately address.

The DSMB may also recommend stopping the trial early if presented with evidence of futility. At each interim analysis the conditional power will be calculated. If, taking into account the whole clinical context, the DSMB considers the prospect of achieving a Statistical significant result within a reasonable sample size to be unacceptably low, then the DSMB may recommend stopping the trial.

The methodology for study re-sizing will follow that of Li, Shih, Xie and Lu (Li G, Shih WJ, Xie T and Lu J (2002) A sample size adjustment procedure for clinical trials based on conditional power. Biostatistics 3: 277-287).

Statistical Analyses

Unless otherwise stated, all statistical tests will be performed using 2-sided tests at the 5% significance level. Baseline is defined as the last observation before the initiation of the study related treatment. Continuous demographic parameters, such as the patient's age at the time of enrolment, will be summarised for the ITT population using descriptive statistics (N, mean, median, standard deviation, minimum and maximum value, and 95% 2-sided confidence limits) and compared between groups using a 2-sample t-test. Categorical parameters will be summarised as a proportion of the ITT population and compared using a 2-tailed Fishers Exact test. Co-morbid risk factors will be summarised for the ITT population by treatment assignment and according to the type of variable (categorical, continuous) and compared between groups. Kaplan-Meier estimates for the time to death will be prepared based on the ITT population. Event rates at 12- and 24-months will be derived from the Kaplan-Meier estimates. The number and proportion of patients alive after each treatment cycle will be tabulated and summarised using 95% confidence intervals. Separate tables containing patient counts, percentages, and 95% binomial confidence intervals will be prepared based on risk factors. No data will be imputed for patients who withdrew prematurely from the study, or have missing values for specific parameters.

Univariate analyses will be prepared for each laboratory parameter and compared between groups using a 2-sample t-test. The proportion of patients found to have abnormal values considered clinically significant will be compared between treatment groups using a 2-tailed Fisher's Exact test. Laboratory shift tables containing patient counts and percentages will be prepared by treatment assignment, laboratory parameter, and time.

Demography

Patient demographic data will be summarised by type of variable; categorical data by counts and percentages and continuous variable by means, standard deviations, medians, minimum, maximum and numbers of patients.

Analysis of Efficacy Data

The standard covariates for the efficacy analyses are:

Geographical region (three groups: USA, European Union, Eastern Europe excluding European Union).

First line of standard care (three groups: IL-2, interferon-a, sunitinib)

Prognostic index (Motzer score). (Motzer score classifies patients into three prognostic groups: "favorable", "intermediate" and "poor" based on an algorithm which considers pretreatment performance status, LDH, hemoglobin, and corrected serum calcium. The inclusion and exclusion criteria preclude enrolment of the "poor" prognostic group. All eligible patients will be covered in the remaining two groups)

Primary

The primary endpoint is time to death. Time to death will be analyzed in the ITT population using a Cox Proportional Hazards regression model with terms for treatment and the standard efficacy covariates.

Secondary

The secondary efficacy endpoints will be analysed following the statistical procedures presented below.

Endpoint: The proportion of patients with progression free survival at 26 weeks (+/- 1 week) based on radiological data in the ITT population.

The proportion of patients with progression free survival at 26 weeks (+/- 1 week) relative to baseline will be analysed using a logistic regression model with terms for treatment and the standard efficacy covariates. Data will be analysed using the ITT population and adjudicated (blinded peer review).

Endpoint: Tumor response rates based on RECIST according to the investigator's reported interpretation of the radiological reports observed in the ITT population.

Both the rate and duration of tumor response will be compared between treatment groups. Response rates will be compared between treatment groups and analysed using a logistic regression model with terms for treatment and the standard efficacy covariates. The duration of response will be analysed using a Cox Proportional Hazards regression model with terms for treatment and the standard efficacy covariates.

Endpoint: The survival event rate ratio in the TroVax® arm versus the placebo arm in the MITT population, based on the log of the hazard ratio.

Time to death will be analysed using a Cox Proportional Hazards regression model with terms for treatment and the standard efficacy covariates. Survival curves for the proportion of patients remaining event-free will be estimated using the Kaplan-Meier method

Endpoint: Anti-5T4 serum antibody levels (additional measures of immune response including specific measures of cellular response will be investigated at some centres).

Qualitative antibody response to 5T4 within the active treatment group will be analysed as a main effect using a logistic regression model with terms for the standard efficacy covariates.

The analysis of the Quality of Life (QOL) parameters is discussed below.

Analysis of adverse event data

Safety will be assessed using the Intent to Treat population. Adverse events will be coded using the MedDRA classification to give a preferred term and organ class for each event. Proportions of patients with adverse events will be presented. Tables of adverse events will be presented by organ class and also by organ class and preferred term. These tables will also include overall totals for adverse events within each body system and organ class. The number of patients with an event in each classification of severity and relationship to treatment within each treatment group will be tabulated. Serious adverse events and adverse events leading to withdrawal will be listed separately.

Treatment emergent and non-emergent events will be presented separately. Treatment emergent adverse events are defined as adverse events that had an onset day on or after the day of the first dose of study medication. Adverse events that have missing onset dates will be considered to be treatment emergent.

Adverse events will be listed by patient within groups showing time of onset, period of event, severity, relationship to disease and outcome.

QOL Parameters

Results from the QOL questionnaire (EuroQoL and QLQ30) will be presented for the ITT and Per-Protocol populations. Results from the QOL questionnaire will be analyzed using a generalised linear modelling approach based on maximum likelihood, treating patients as a random effect in the model. Terms will be included for the standard efficacy covariates.

Concomitant Medication

Concomitant medication will be listed by patient, treatment assignment, and study visit.

Vital Signs

Vital signs to be collected throughout the course of the study include systolic and diastolic blood pressures (mmHg), heart rate (bpm), body temperature (oC/°F), and weight (kg). Vital signs will be summarised using univariate statistics (N, arithmetic average, standard deviation, median, and range) for each clinical assessment and presented for the cohort of patients who have data at the initial baseline visit and at least one the specific follow-up visits. In addition to the univariate statistics, the changes from baseline to each follow-up assessment visit will be analysed using a paired-difference t-test for the within-group mean change from baseline. Additionally, 95% confidence interval limits for the mean change from baseline will also be reported.

The incidence rates of clinically notable vital sign changes, including the criteria for clinically notable, will be summarized and presented in a Patient Data Listing. Vital signs and body weight abnormalities of potential clinical significance will be defined as follows:

- Systolic blood pressure change $\geq 20$ mmHg and a systolic blood pressure value that was $\geq 180$ or $\leq 90$ mmHg

- Diastolic blood pressure change $\geq 15$ mmHg and a diastolic blood pressure value that was $\geq 105$ or $\leq 50$ mmHg

- Pulse change of $\geq 15$ bpm and a pulse value that was $\geq 120$ or $\leq 50$ bpm

- Temperature change of $\geq 1$ °C/2°F and a temperature value that was $\geq 38°C/101°F$

- Body weight decrease $\geq 5\%$

- Clinically significant abnormal vital signs will be flagged and presented using counts by study visit.

An additional listing will be provided for those patients who have clinically significant vital sign abnormalities.

Other Safety Parameters

All other safety parameters will be listed by patient, treatment assignment, and study treatment period.

Laboratory Parameters

Haematology, biochemistry and other laboratory data will be listed at each time point by treatment group and, for appropriate values, will be flagged using the signed laboratory ranges as High/Low/Within laboratory normal range (H, L).

Changes from baseline will also be listed and abnormal changes from baseline will be flagged.

An additional listing will be provided for those patients who have laboratory values that are abnormal and considered to be clinically significant.

Withdrawals

The number (%) of patients who withdraw from the study over time, along with their reasons for withdrawal, will be tabulated.

Deaths

All deaths occurring during the treatment period of study and its follow up period will be listed.

Determination of Treatment Group Comparability

Patient demographics and disease histories will be summarised for each treatment group and compared between treatment groups.

Treatment Assignment

Patients will be randomised using a stratfied central randomisation scheme. Given the initial target enrolment and the proposed number of clinical sites, attempting to balance the enrolment on an intra-centre basis was not considered feasible using a deterministic randomisation scheme. For example, if patients were to be randomised intra-centre using randomised blocks of 4, and 50% of the sites failed to fill a complete block, an enrolment imbalance could develop between the 2 groups resulting in a loss of statistical power. To eliminate this potential imbalance, a central randomisation scheme will be used, balancing on blocks of 4 within geographical areas (usually countries) involving multiple sites.

Stratification

Patients will be stratified by selected standard of care, prognostic indicator (Motzer score), geographical area, and institution. The stratification will be performed by IVRS.

**Results**

Following the announced ending of the trial, immunological and clinical response data were un-blinded. An exploratory analysis was undertaken with the primary aim of identifying potential correlates between parameters and enhanced patient survival.

The analyses have focused solely on the biomarkers detected in patients known to have received TroVax®. The magnitude of the biomarker level has been analyzed. Each patient was then categorized into "above median" or "below median" category or low or high level category. The survival of patients in each category was compared by plotting Kaplan-Meier curves.

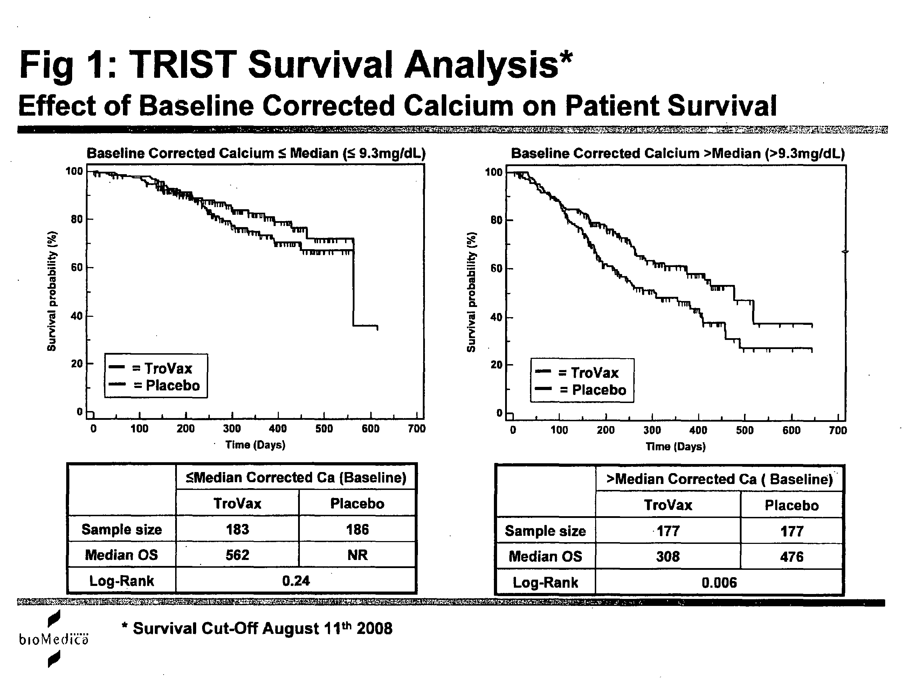

Figure 1 illustrates "above median" and "below median" corrected calcium effects on patient survival in TroVax®-treated TRIST patients.

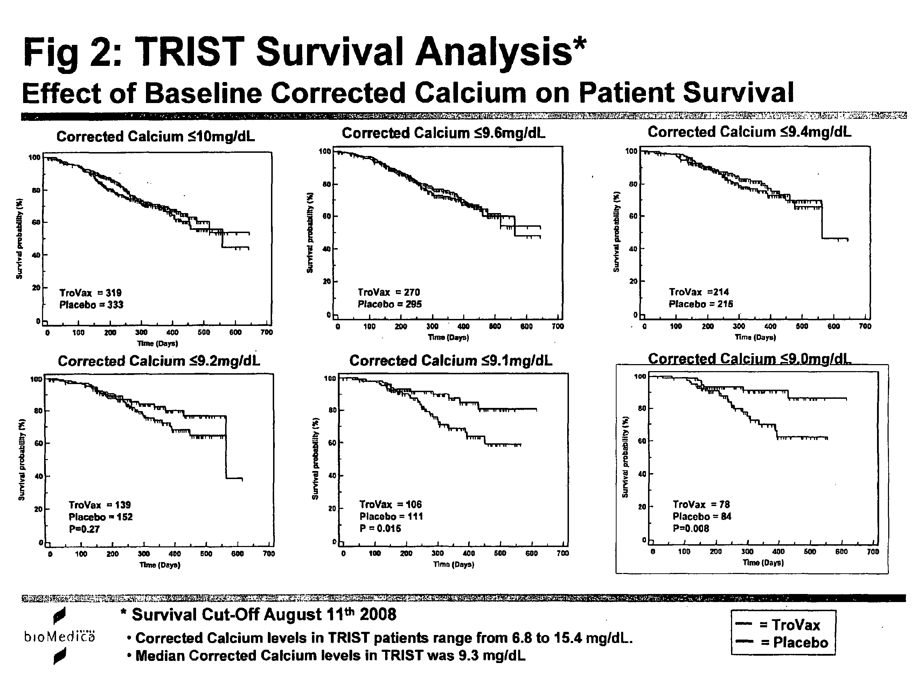

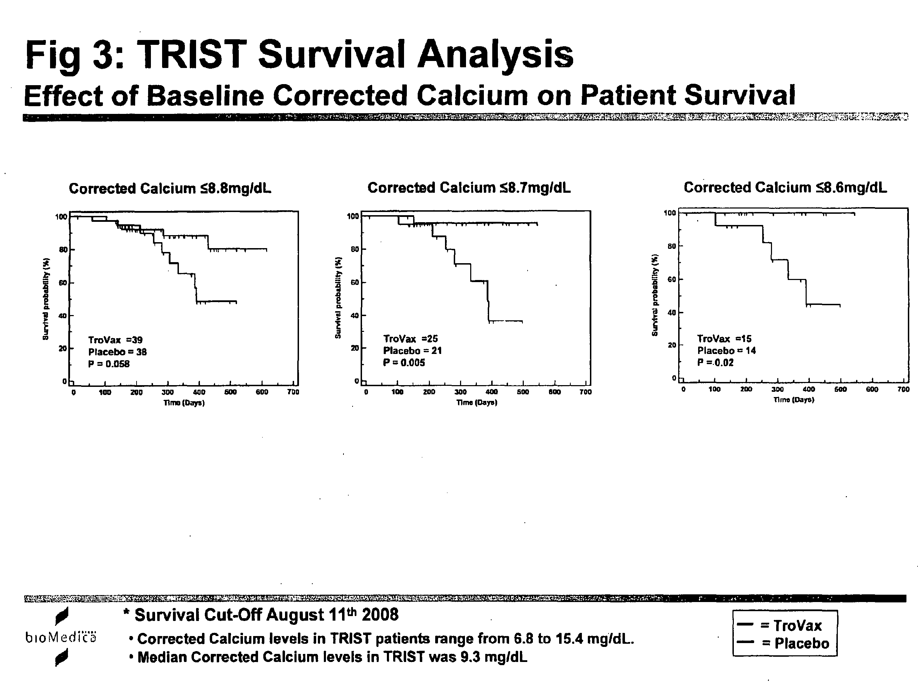

The data summarized in Figure 1 suggest that patients with lower (below median) corrected calcium levels survive for longer than those with above median corrected calcium levels. Figures 2 and 3 confirm that this effect and in particular that it continues as the corrected calcium level is further reduced below median.

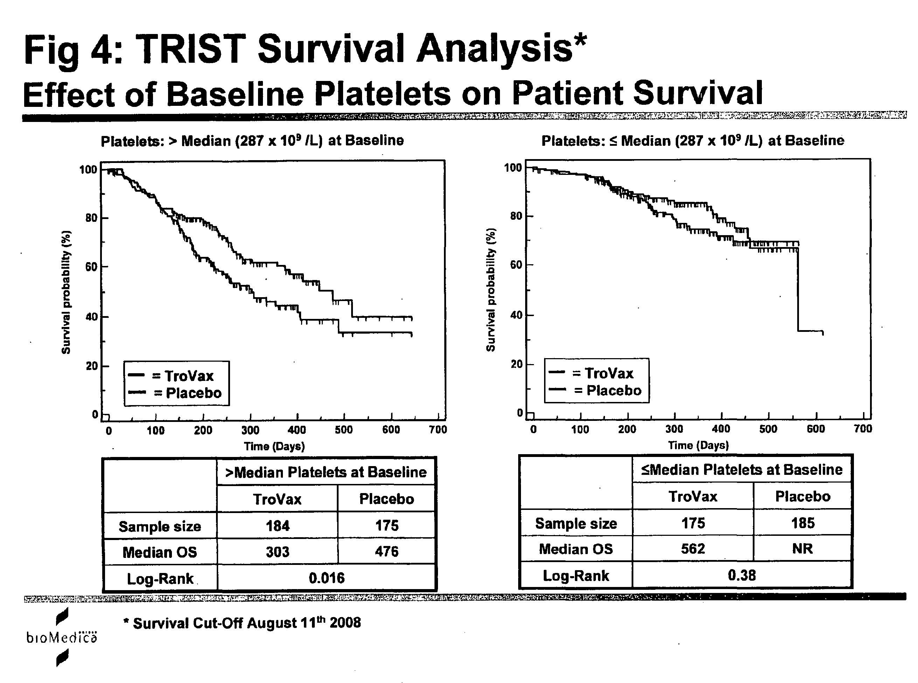

Figure 4 illustrates "above median" and "below median" platelet level effects on patient survival in TroVax®-treated TRIST patients.

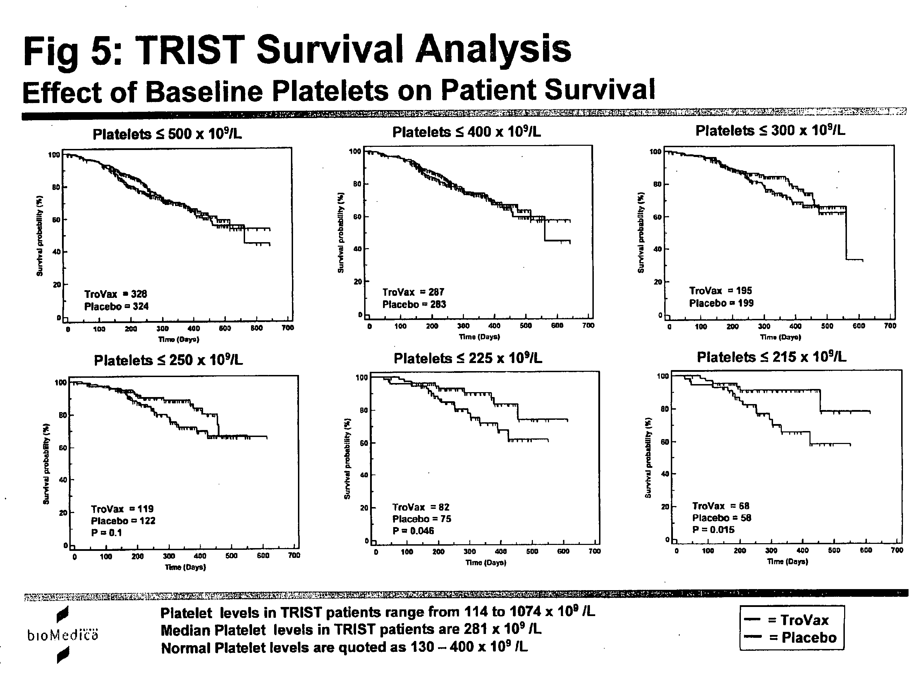

The data summarized in Figure 4 suggest that patients with lower (below median) platelet levels survive for longer than those with above median platelet levels. Figure 5 confirms that this effect continues as the platelet level is further reduced below median.

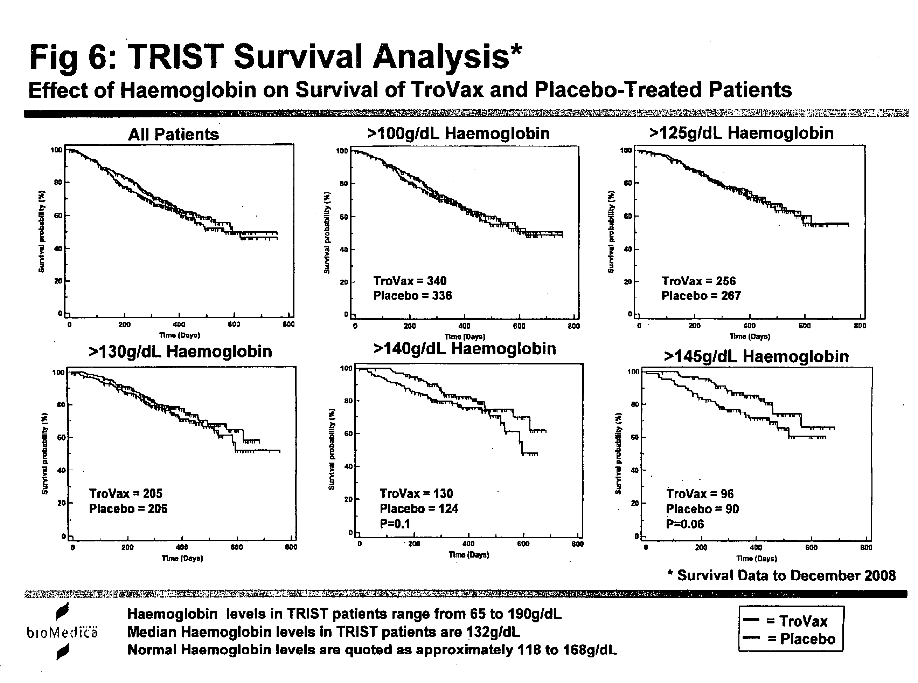

Figure 6 illustrates haemoglobin level effects on patient survival in TroVax®-treated TRIST patients. The data summarized in Figure 6 suggest that patients with higher haemoglobin levels survive for longer than those with lower haemoglobin levels.

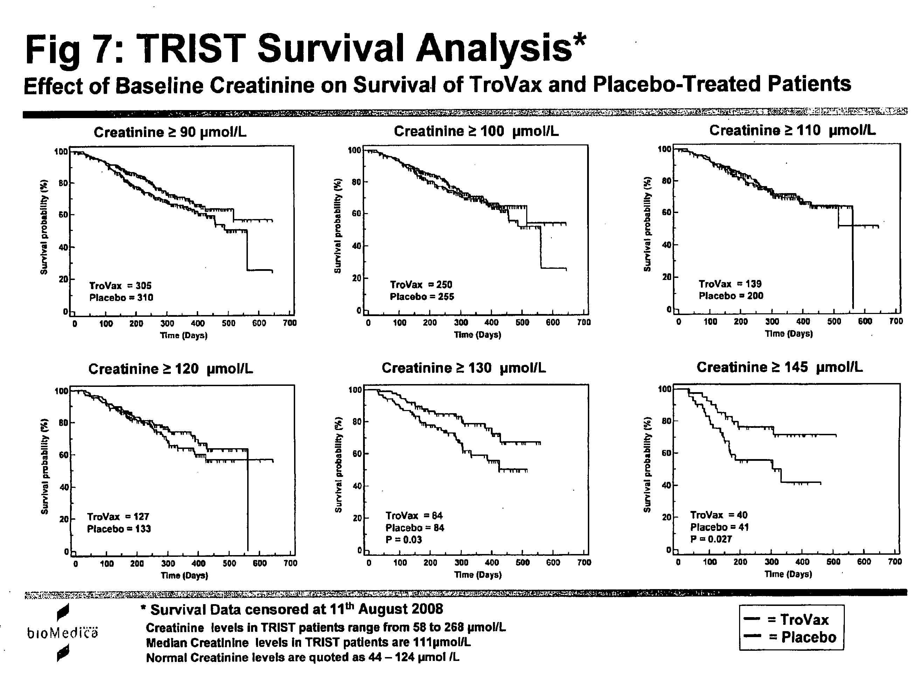

Figure 7 illustrates creatinine level effects on patient survival in TroVax®-treated TRIST patients. The data summarized in Figure 7 suggest that patients with higher creatinine levels. survive for longer than those with lower creatinine levels.

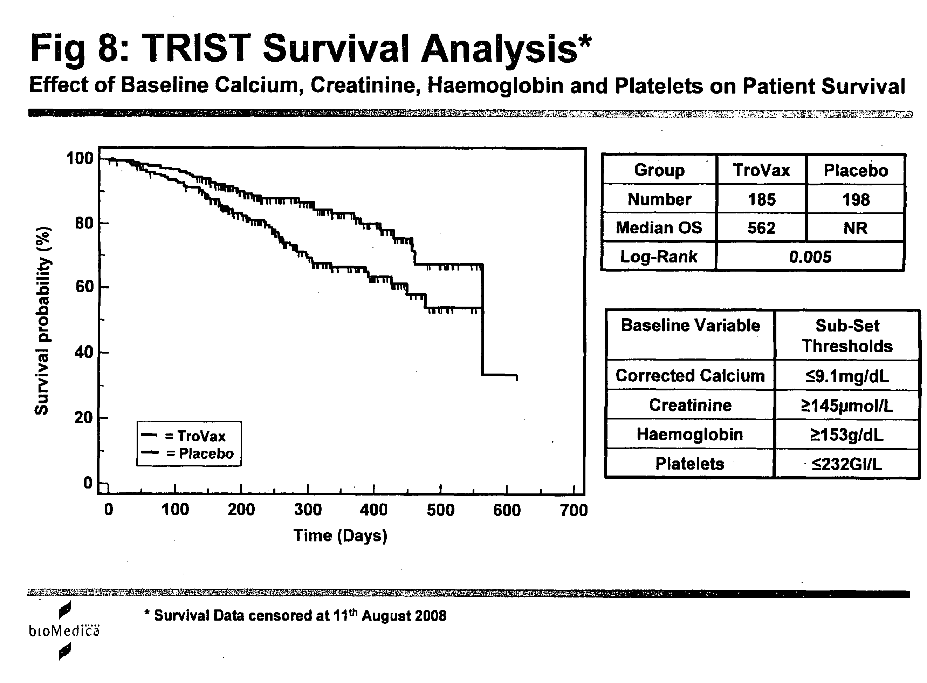

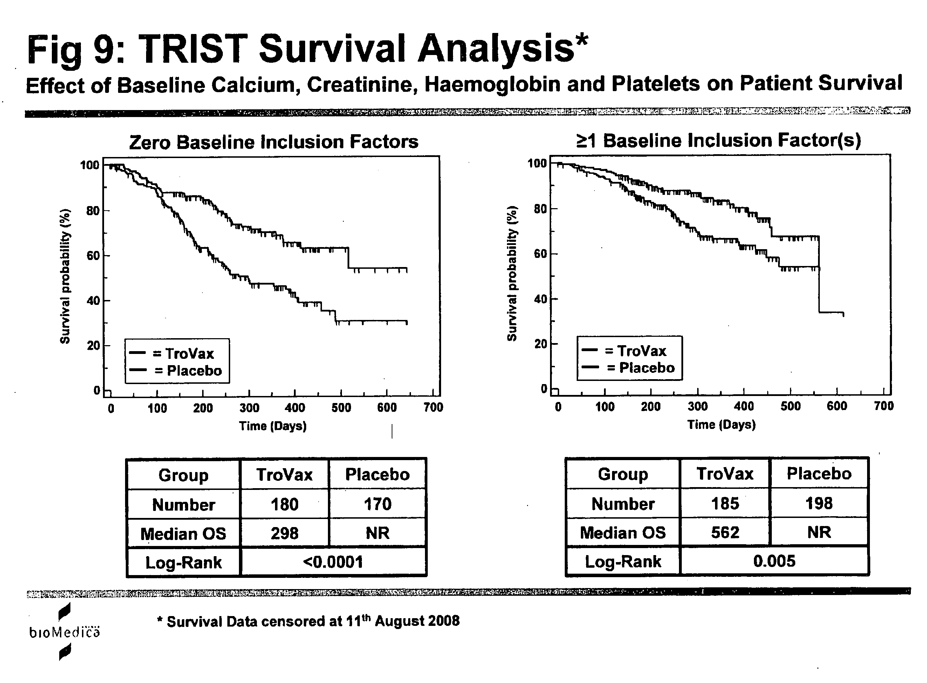

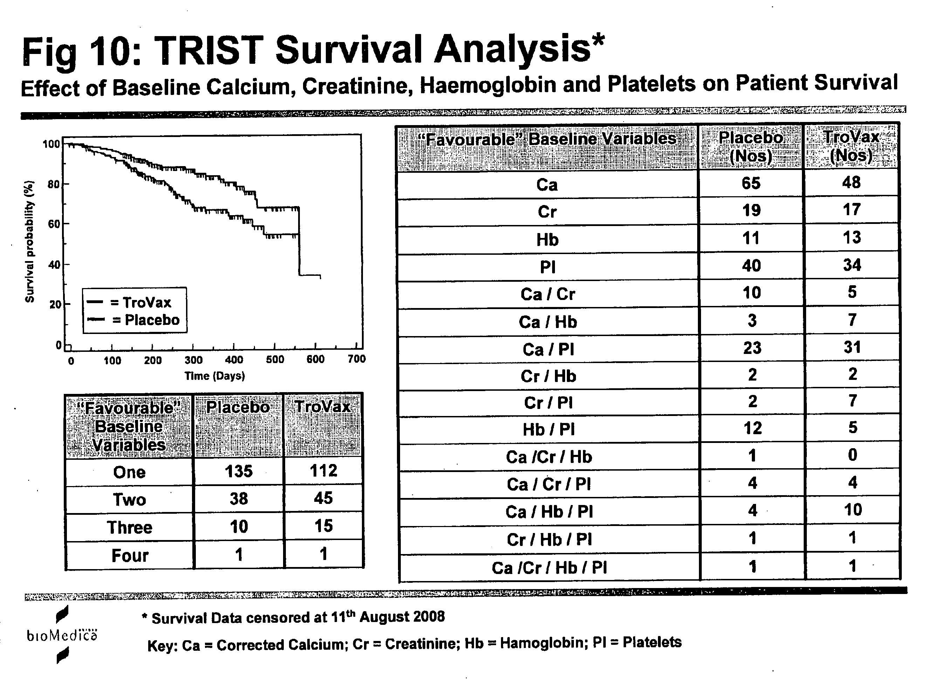

The overall effect of baseline corrected calcium, creatinine, haemoglobin and platelets on patient survival in TroVax®-treated TRIST patients is summarised in Figures 8 to 10.

**Example 2 - Phase II Survival Analysis (CRC) Patients - Effect of factors on Patient Survival**

The results of the trials described in the following papers were analysed:

Vaccination of colorectal cancer patients with modified vaccinia Ankara delivering the tumor antigen 5T4 (TroVax) induces immune responses which correlate with disease control: a phase I/II trial. Harrop R, Connolly N, Redchenko I, Valle J, Saunders M, Ryan MG, Myers KA, Drury N, Kingsman SM, Hawkins RE, Carroll MW. Clin Cancer Res. 2006 Jun 1;12(11 Pt 1):3416-24.

An MVA-based vaccine targeting the oncofetal antigen 5T4 in patients undergoing surgical resection of colorectal cancer liver metastases. Elkord E, Dangoor A, Drury NL, Harrop R, Burt DJ, Drijfhout JW, Hamer C, Andrews D, Naylor S, Sherlock D, Hawkins RE, Stem PL. J Immunother. 2008 Nov-Dec;31(9):820-9..

Vaccination of colorectal cancer patients with TroVax given alongside chemotherapy (5-fluorouracil, leukovorin and irinotecan) is safe and induces potent immune responses. Harrop R, Drury N, Shingler VV, Chikoti P, Redchenko I, Carroll MW, Kingsman SM, Naylor S, Griffiths R, Steven N, Hawkins RE. Cancer Immunol Immunother. 2008 Jul;57(7):977-86.

Vaccination of colorectal cancer patients with modified vaccinia ankara encoding the tumor antigen 5T4 (TroVax) given alongside chemotherapy induces potent immune responses. Harrop R, Drury N, Shingler VV, Chikoti P, Redchenko I, Carroll MW, Kingsman SM, Naylor S, Melcher A, Nicholls J, Wassan H, Habib N, Anthoney A. Clin Cancer Res. 2007 Aug 1;13(15 Pt 1):4487-94.

An exploratory analysis was undertaken with the primary aim of identifying potential correlates between parameters and enhanced patient survival.

The analyses have focused solely on the biomarkers detected in patients known to have received TroVax®. The magnitude of the biomarker level has been analyzed. Each patient was then categorized into "above median" or "below median" category or low or high level category. The survival of patients in each category was compared by plotting Kaplan-Meier curves.

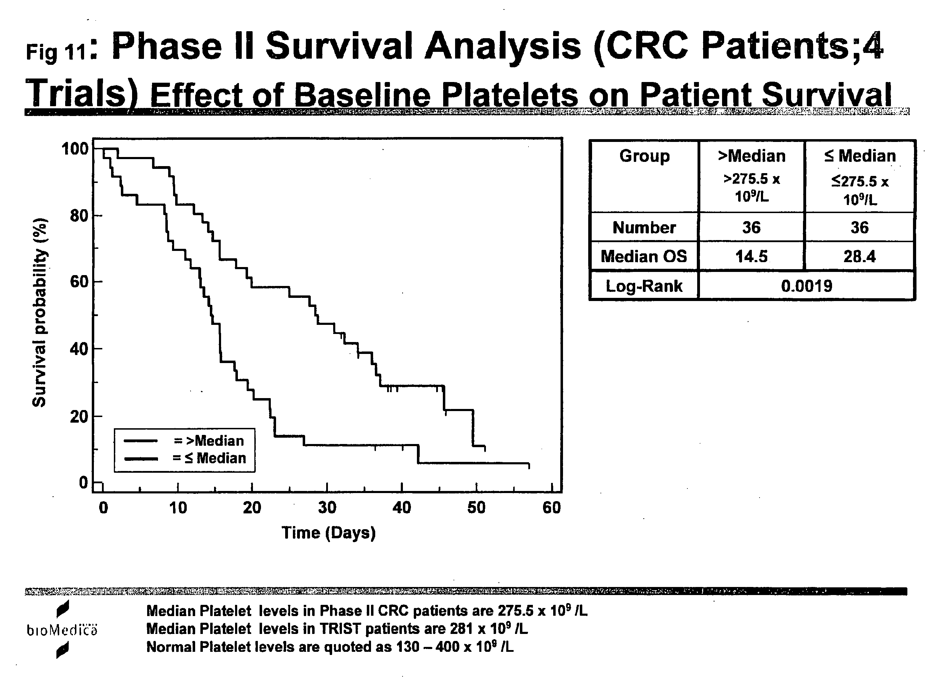

Figure 11 illustrates "above median" and "below median" platelet level effects on patient survival in TroVax®-treated CRC patients.

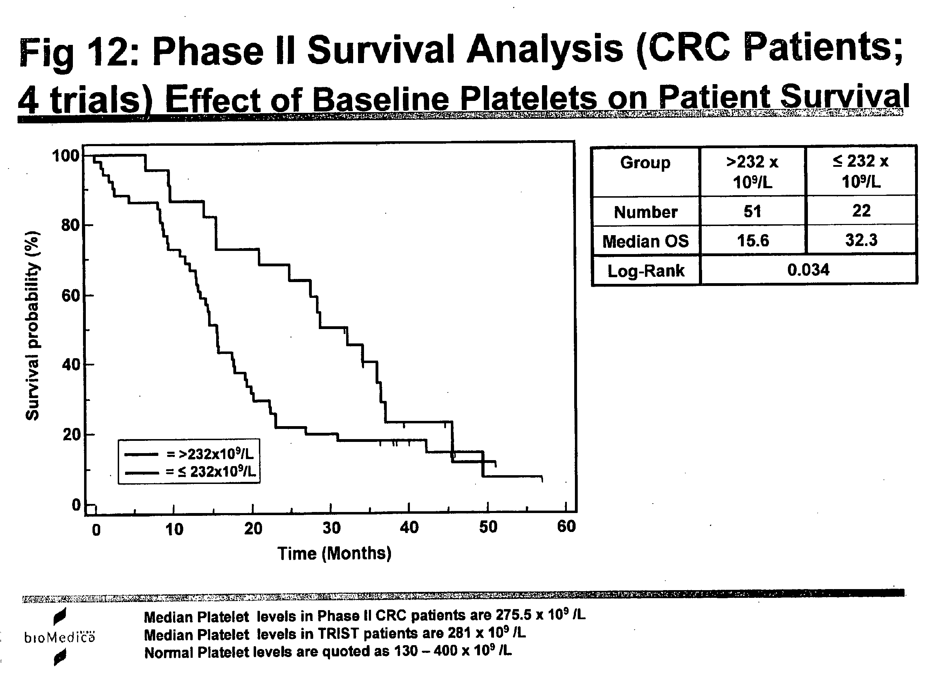

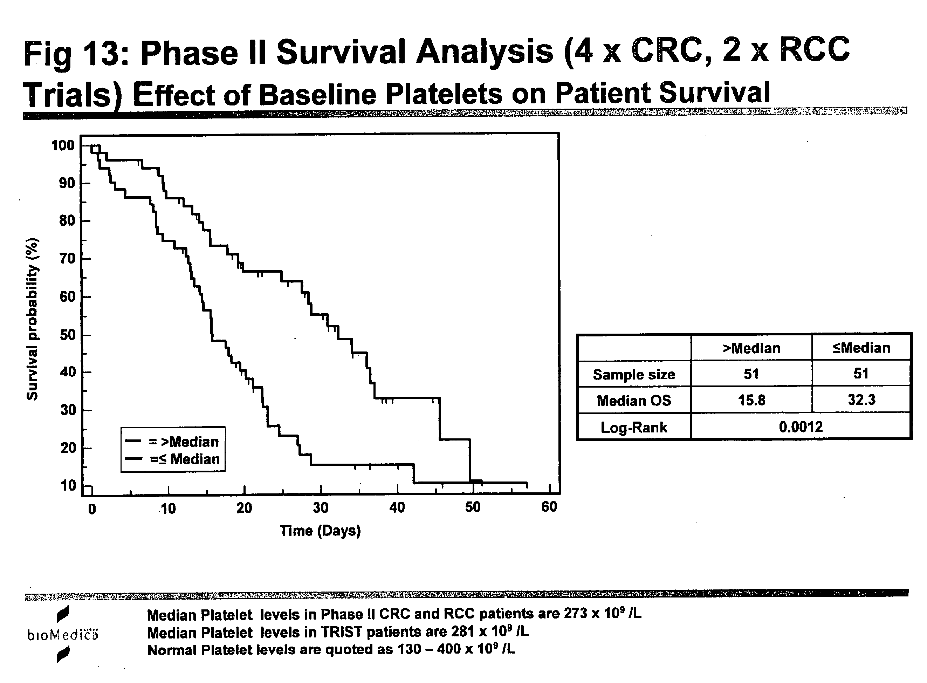

The data summarized in Figure 12 suggest that patients with lower platelet levels survive for longer than those with higher platelet levels. This effect is confirmed in Figure 13 which also includes the results of two RRC trials, namely:

- Clinical trial phase II (single centre study) A preliminary study of the safety, immunogencity and clinical efficacy of TroVax® given in conjunction with interleukin 2 (IL-2) in the treatment of stage IV renal cell cancer, IND Number 11433; and

- A phase I/II feasibility trial to assess the safety, immunological activity and efficacy of TroVax® plus interferon α (IFN-α) in patients with advanced or metastatic renal cell cancer. EudraCT Number: 2006-000753-22. GTAC Number: 117.

Details of these trials can be found on www.clinicaltrials.gov.

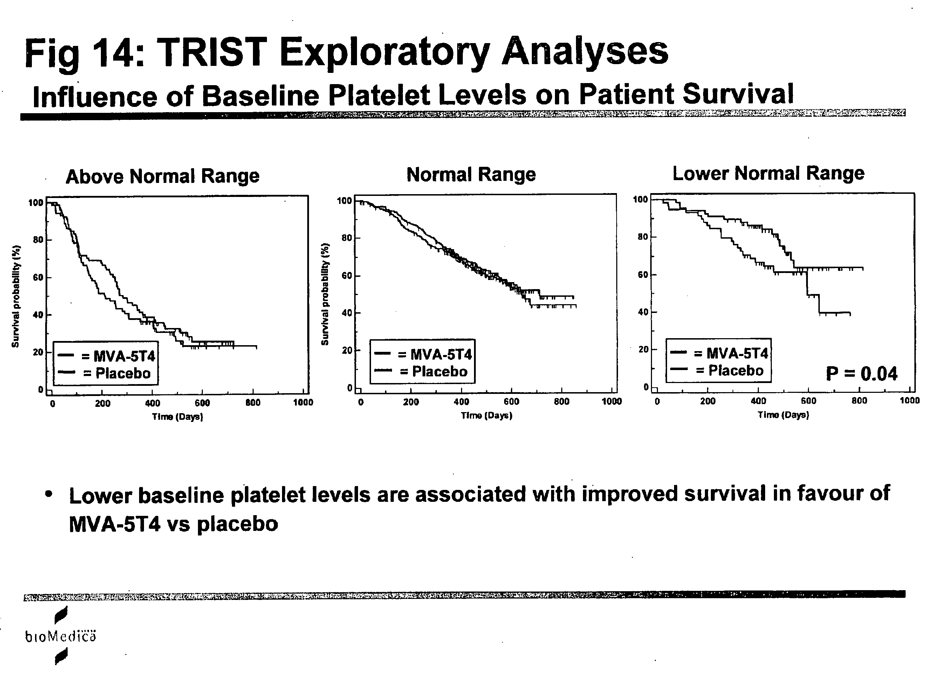

Figure 14 shows the influence of baseline platelet levels on patient survival at a later stage than Figure 13. It can be seen that lower normal range platelet levels are associated with improved survival in favour of TroVax® versus placebo.

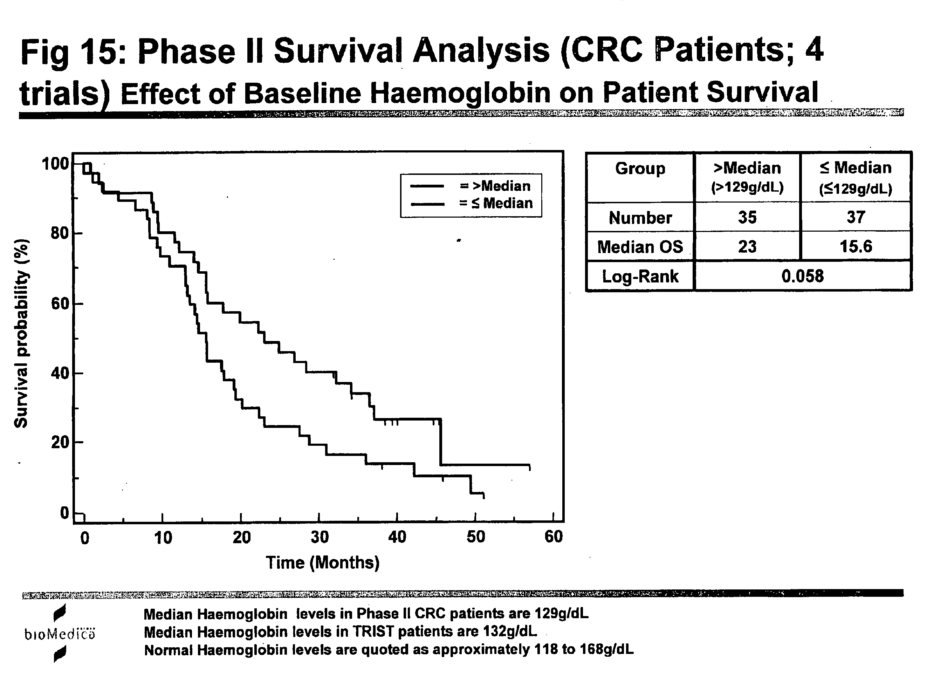

Figure 15 illustrates "above median" and "below median" haemoglobin level effects on patient survival in TroVax®-treated CRC patients in the four above-mentioned CRC trials. The data summarized in Figure 15 suggest that patients with higher haemoglobin levels survive for longer than those with lower haemoglobin levels.

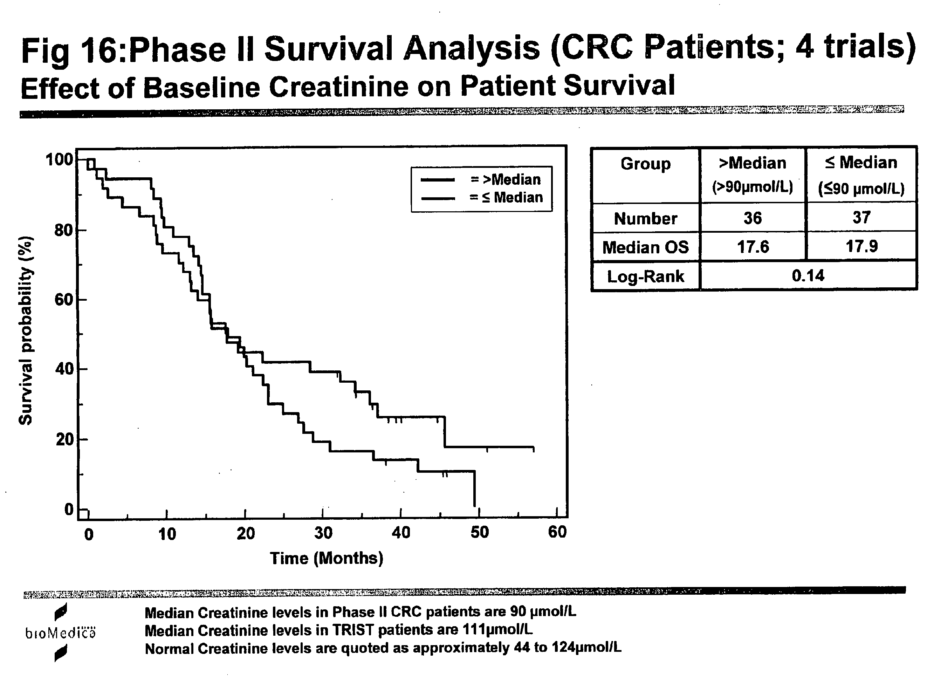

Figure 16 illustrates "above median" and "below median" creatinine level effects on patient survival in TroVax®-treated CRC patients in the four above-mentioned CRC trials. The data summarized in Figure 16 suggest that patients with higher creatinine levels survive for longer than those with lower creatinine levels.

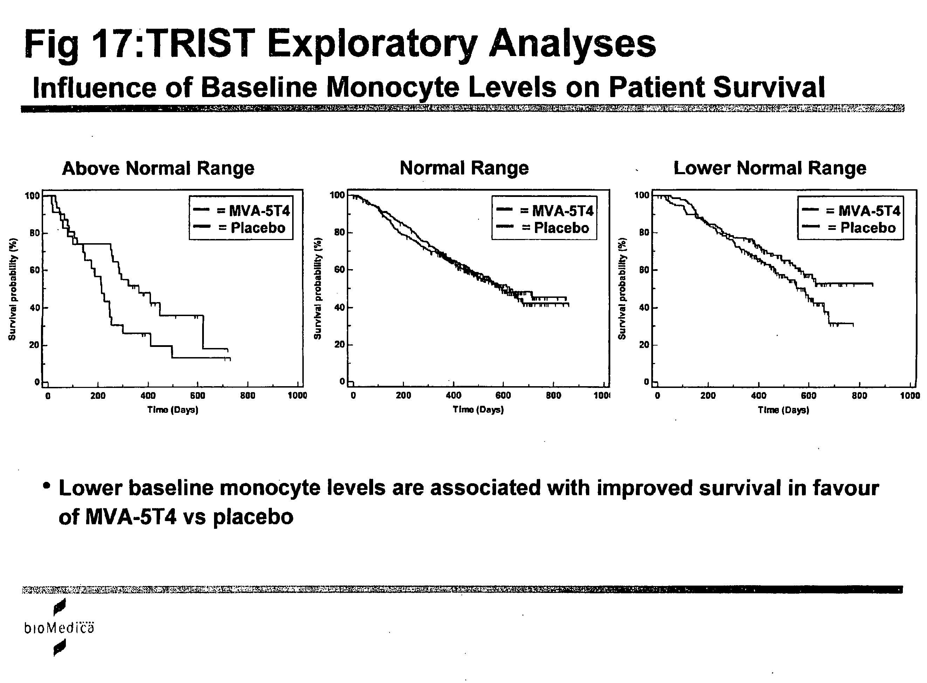

Figure 17 shows the influence of baseline monocyte levels on patient survival at the same stage as Figure 14. It can be seen that lower normal range platelet levels are associated with improved survival in favour of TroVax® versus placebo.

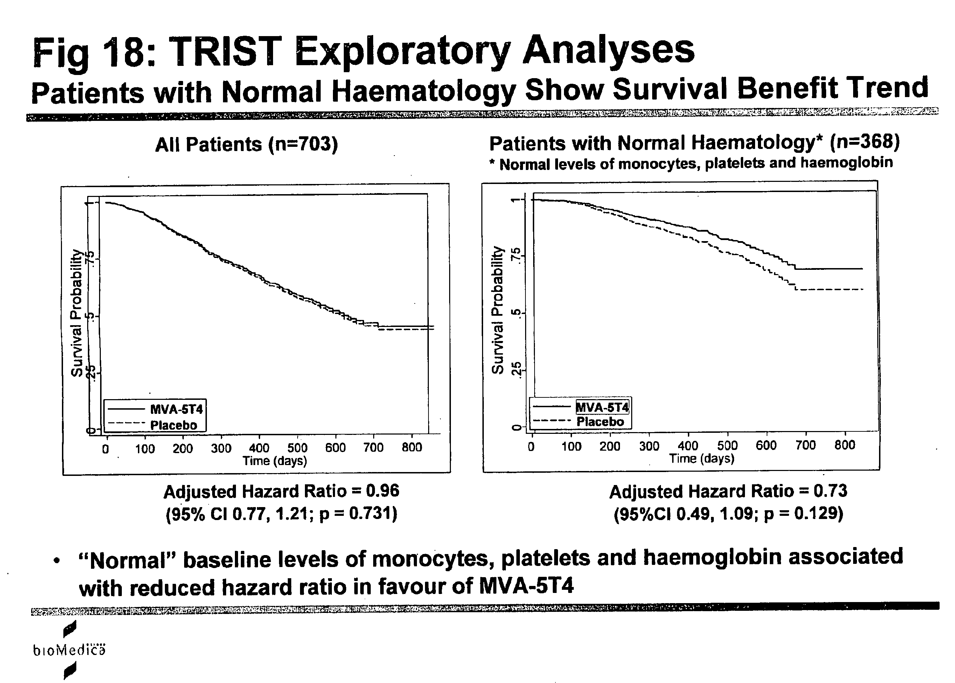

Figure 18 shows that patients with a normal haematology show a survival benefit rend. Thus, baseline levels of monocytes, platelets and haemoglobin within normal baseline levels are associated with a reduced hazard ratio in favour of TroVax®

## Example 3 - Survival analysis

From previous analysis reported above, four variables (platelets, monocytes, white blood cells (WBC) and haemoglobin) were selected to be examined in more detail. In this analysis, the change in treatment effect across subsets of patients defined by baseline values of platelets, monocytes, WBC and haemoglobin was examined.

### Objective & Methods

Objective

To estimate and plot the adjusted hazard ratio across changing baseline levels of platelets, monocytes, white blood cells and haemoglobin.

Methods

Subsets of patient variables were created using cut points defined by the 10th, 25th, 50th, 75th and 90th percentile for each of the four factors. For each subset, the adjusted hazard ratio (which adjusted for imbalances in prognostic factors between the two treatment arms) was estimated using a Cox proportional hazards model. As there were a limited number of patients and events in the subsets, it was not possible to include all known prognostic variables, as a consequence, prognostic variables to be included in the model were selected using a backwards selection procedure with a 10% selection criterion. The adjusted hazard ratio and 95% confidence intervals were plotted by deceasing and increasing values of baseline concentration.

This analysis was performed on mature survival data censored 13th March 2009 (downloaded on 1st June 2009)

Table 1 lists the prognostic variables included in the baseline model prior to the selection procedure. The specific factor being investigated was not included in the model as a confounder (for example when estimating the hazard ratio for subsets defined by baseline levels of monocytes, monocytes was not included as a confounder in the model). When the subsets were defined by WBC, then in addition to WBC not being included in the model, monocytes, neutrophils and lymphocytes were also not being included.

**Table 1: Known prognostic factors identified from the literature included in the baseline model**

| prognostic factors * |
| --- |
| **Patient variables** |
| Age |
| Gender |
| Body Mass Index (BMI) |
| Karnofsky score |
| |
| **Blood biochemistry variables** |
| Alkaline Phosphatase |
| Corrected Calcium |
| Haemoglobin |
| LDH |
| Sodium |
| Lymphocytes |
| Monocytes |
| Neutrophils |
| Platelets |
| **Tumour variables** |
| Total number of metastatic sites |
| Number of bone lesions |

(continued)

| prognostic factors * |
| --- |
| Number of liver lesions |
| Number of lymph node lesions |

\* Model also included Standard of care (INF-alpha, IL-2, sutent), and treatment arm (TroVax, placebo). Variables listed are known prognostic factors in renal cell carcinoma obtained from a literature search.

## Results

### Platelets

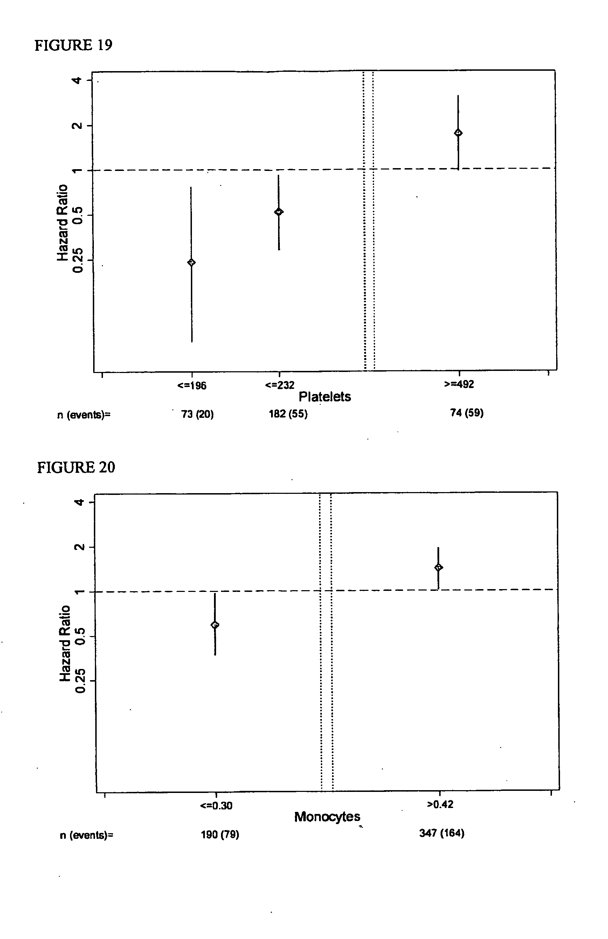

It can be seen from Table 2 and Figure 19, that the hazard ratio differed across subsets of patients define by platelet concentration. In patients with platelets ≤ 232, the hazard ratio was 0.52 (95% CI 0.29, 0.93), so the hazard of dying was approximately 50% less in TroVax compared to placebo. Conversely, it was found that the hazard was higher in Trovax compared to placebo for patients who had platelets >288, however this result was not significant at the 5% level.

**Table 2: Adjusted hazard ratio and 95% CI for subsets defined by baseline levels of platelets**

| percentile | platelet level | n (events) | Hazard ratio | 95% CI | p-value |
| --- | --- | --- | --- | --- | --- |
| 10% | ≤196 | 73 (20) | 0.24 | (0.07,0.77) | 0.017 |
| 25% | ≤232 | 182 (55) | 0.52 | (0.29,0.93) | 0.032 |
| 90% | ≥492 | 74 (59) | 1.74 | (0.98,3.09) | 0.058 |

### Monocytes

It can be seen from Table 3 and Figure 20, that the hazard ratio differed across subsets of patients define by monocyte level. In patients with monocytes ≤ 0.30, the hazard ratio was 0.59 (95% CI 0.37, 0.97), so the hazard of dying was approximately 40% less in TroVax compared to placebo. Conversely, in patients with monocytes >0.42, the hazard ratio was 1.41 (95% CI 1.02, 1.94).

**Table 3: Adjusted hazard ratio and 95% CI for subsets defined by baseline levels of monocytes**

| percentile | monocyte level | n (events) | Hazard ratio | 95% CI | p-value |
| --- | --- | --- | --- | --- | --- |
| 25% | ≤0.30 | 190 (79) | 0.59 | (0.37,0.97) | 0.038 |
| 50% | >0.42 | 347 (164) | 1.41 | (1.02,1.94) | 0.035 |

### White Blood Cells (WBC)

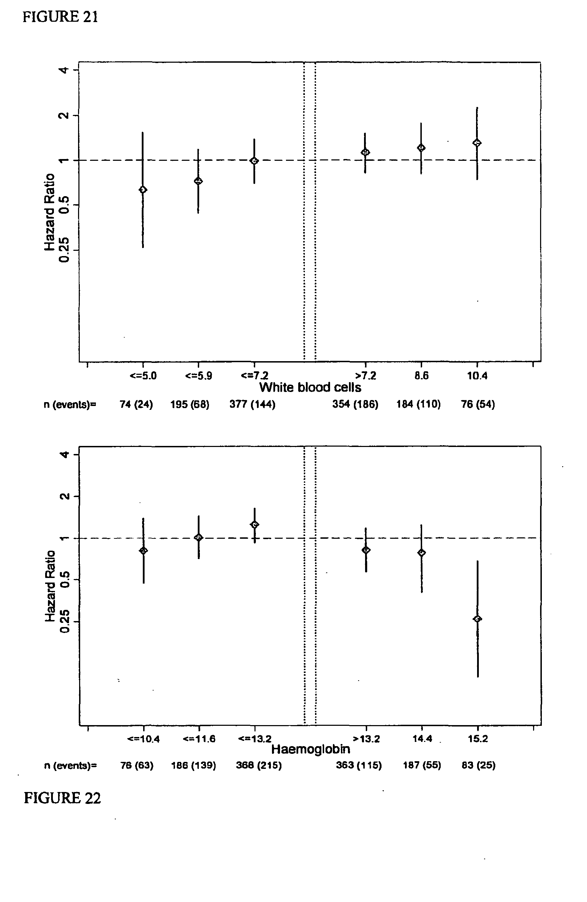

It can be seen from Table 4 and Figure 21, that the hazard ratio differed across subsets of patients define by WBC level. In patients with WBC ≤ 5.9, the hazard ratio was 0.72 (95% CI 0.44, 1.18). Conversely, in patients with WBC >7.2, the hazard ratio was 1.12 (95% CI 0.82, 1.52).

**Table 4: Adjusted hazard ratio and 95% CI for subsets defined by baseline levels of WBC**

| percentile | wbc | n (events) | Hazard ratio | 95% CI | p-value |
| --- | --- | --- | --- | --- | --- |
| 10% | ≤5.0 | 74 (24) | 0.63 | (0.26,1.53) | 0.308 |
| 25% | ≤5.9 | 195 (68) | 0.72 | (0.44,1.18) | 0.194 |
| 50% | ≤7.2 | 377 (144) | 0.99 | (0.70,1.38) | 0.942 |
| 50% | >7.2 | 354(186) | 1.12 | (0.82,1.52) | 0.469 |
| 75% | ≥8.6 | 184(110) | 1.2 | (0.81,1.78) | 0.357 |
| 90% | ≥10.4 | 76 (54) | 1.3 | (0.74,2.27) | 0.355 |

**Haemoglobin**

It can be seen from Table 5 and Figure 22, that the hazard ratio differed across subsets of patients defined by haemoglobin level. In patients with haemoglobin ≤ 13.2, the hazard ratio was 1.24 (95% CI 0.93, 1.64). Conversely, in patients with haemoglobin >15.2, the hazard ratio was 0.26 (95% CI 0.10, 0.68).

**Table 5: Adjusted hazard ratio and 95% CI for subsets defined by baseline levels of haemoglobin**

| percentile | haemoglobin | n (events) | Hazard ratio | 95% CI | p-value |
|---|---|---|---|---|---|
| 10% | ≤10.4 | 76 (63) | 0.81 | (0.47,1.38) | 0.430 |
| 25% | ≤11.6 | 186 (139) | 1.01 | (0.71,1.44) | 0.936 |
| 50% | ≤13.2 | 368 (215) | 1.24 | (0.93,1.64) | 0.141 |
| 50% | >13.2 | 363 (115) | 0.82 | (0.57,1.18) | 0.292 |
| 75% | ≥14.4 | 187 (55) | 0.78 | (0.40,1.23) | 0.219 |
| 90% | ≥15.2 | 83 (25) | 0.26 | (0.10,0.68) | 0.006 |

**Conclusions**

These data suggest that there is differential risk-benefit to patient survival dependent on the relative levels of certain biomarkers. These data can be used to plan treatment strategies to maximize the survival advantage of 5T4 targeted immunotherapies including identifying patient characteristics that would predict this type of response through assessment of baseline status.

Various features will also now be described with reference to the following numbered paragraphs:

1. A method for determining a prognosis for benefit for a cancer patient receiving immunotherapy treatment involving (a) measuring a level of corrected calcium in a sample from the cancer patient, and (b) comparing the level of corrected calcium in the sample to a reference level of corrected calcium, wherein a lower level of corrected calcium in the sample correlates with increased benefit to the patient.

2. A method for determining a prognosis for benefit for a cancer patient receiving immunotherapy treatment involving (a) measuring a level of corrected calcium in a sample from the cancer patient, and (b) classifying the patient as belonging to either a first or second group of patients, wherein the first group of patients having low levels of corrected calcium is classified as having an increased likelihood of benefit than the second group of patients having high levels of corrected calcium.

3. A method for monitoring the effectiveness of a course of treatment for a patient with cancer involving (a) determining a level of a corrected calcium in a sample from the cancer patient prior to immunotherapy treatment, and (b) determining the level of corrected calcium in a sample from the patient after treatment, whereby comparison of the corrected calcium level prior to treatment with the corrected calcium level after treatment indicates the effectiveness of the treatment.

4. A method of predicting the responsiveness of a patient or patient population with cancer to treatment with immunotherapy, or for selecting patients or patient populations that will respond to immunotherapy comprising comparing the differential levels of corrected calcium.

5. A method for determining a prognosis for benefit for a cancer patient receiving immunotherapy treatment involving (a) measuring a level of platelets in a sample from the cancer patient, and (b) comparing the level of platelets in the sample to a reference level of platelets, wherein a lower level of platelets in the sample correlates with increased benefit to the patient.

6. A method for determining a prognosis for benefit for a cancer patient receiving immunotherapy treatment involving (a) measuring a level of platelets in a sample from the cancer patient, and (b) classifying the patient as belonging to either a first or second group of patients, wherein the first group of patients having low levels of platelets is classified as having an increased likelihood of benefit than the second group of patients having high levels of platelets.

7. A method for monitoring the effectiveness of a course of treatment for a patient with cancer involving (a) determining a level of platelets in a sample from the cancer patient prior to immunotherapy treatment, and (b) determining the level of platelets in a sample from the patient after treatment, whereby comparison of the platelet level prior to treatment with the platelet level after treatment indicates the effectiveness of the treatment.

8. A method of predicting the responsiveness of a patient or patient population with cancer to treatment with immunotherapy, or for selecting patients or patient populations that will respond to immunotherapy, comprising comparing the differential levels of platelets.

9. A method for determining a prognosis for benefit for a cancer patient receiving immunotherapy treatment involving (a) measuring a level of haemoglobin in a sample from the cancer patient, and (b) comparing the level of haemoglobin in the sample to a reference level of haemoglobin, wherein a higher level of haemoglobin in the sample correlates with increased benefit to the patient.

10. A method for determining a prognosis for benefit for a cancer patient receiving immunotherapy treatment involving (a) measuring a level of haemoglobin in a sample from the cancer patient, and (b) classifying the patient as belonging to either a first or second group of patients, wherein the first group of patients having high levels of platelets is classified as having an increased likelihood of benefit than the second group of patients having low levels of haemoglobin.

11. A method for monitoring the effectiveness of a course of treatment for a patient with cancer involving (a) determining a level of haemoglobin in a sample from the cancer patient prior to immunotherapy treatment, and (b) determining the level of haemoglobin in a sample from the patient after treatment, whereby comparison of the haemoglobin level prior to treatment with the haemoglobin level after treatment indicates the effectiveness of the treatment.

12. A method of predicting the responsiveness of a patient or patient population with cancer to treatment with immunotherapy, or for selecting patients or patient populations that will respond to immunotherapy, comprising comparing the differential levels of haemoglobin.

13. A method for determining a prognosis for benefit for a cancer patient receiving immunotherapy treatment involving (a) measuring a level of creatinine in a sample from the cancer patient, and (b) comparing the level of creatinine in the sample to a reference level of creatinine, wherein a higher level of creatinine in the sample correlates with increased benefit to the patient.

14. A method for determining a prognosis for benefit for a cancer patient receiving immunotherapy treatment involving (a) measuring a level of creatinine in a sample from the cancer patient, and (b) classifying the patient as belonging to either a first or second group of patients, wherein the first group of patients having high levels of platelets is classified as having an increased likelihood of benefit than the second group of patients having low levels of creatinine.

15. A method for monitoring the effectiveness of a course of treatment for a patient with cancer involving (a) determining a level of creatinine in a sample from the cancer patient prior to immunotherapy treatment, and (b) determining the level of creatinine in a sample from the patient after treatment, whereby comparison of the creatinine level prior to treatment with the creatinine level after treatment indicates the effectiveness of the treatment.

16. A method of predicting the responsiveness of a patient or patient population with cancer to treatment with immunotherapy, or for selecting patients or patient populations that will respond to immunotherapy, comprising comparing the differential levels of creatinine.

17. A method for determining a prognosis for benefit for a cancer patient receiving immunotherapy treatment involving (a) measuring levels of one or more of corrected calcium, platelets, haemoglobin and creatinine and (b) comparing the level of corrected calcium, platelets, haemoglobin and creatinine in the sample to a reference level of corrected calcium, platelets, haemoglobin and creatinine, as appropriate, wherein a low level of corrected calcium or a low level of platelets, and/or a high level of haemoglobin or a high level of creatinine in said sample correlates with increased benefit to said patient.

18. A method for determining a prognosis for benefit for a cancer patient receiving immunotherapy treatment involving (a) measuring levels of corrected calcium, platelets, haemoglobin and/or creatinine and one or more factors selected from the group consisting of : chloride, eosinophils, haematocrit, sodium, basophils, serum calcium, monocytes, neutrophils and WBCs and (b) comparing the level of corrected calcium, platelets, haemoglobin and/or creatinine and the one or more selected biomarkers in the sample to a reference level of corrected calcium, platelets, haemoglobin and/or creatinine and the one or more selected biomarkers, wherein a low level of corrected calcium and/or platelets, a high level of haemoglobin and/or creatinine, and a high level of any of chloride, eosinophils, haematocrit, sodium and/or a low level of any basophils, serum calcium, monocytes, neutrophils and WBCs in said sample correlates with increased benefit to said patient.

19. The method according to any one of the preceding paragraphs, wherein the cancer is invasive carcinoma of the Ampulla of Vater, breast, colon, endometrium, pancreas, or stomach; a squamous carcinoma of the bladder, cervix, lung or oesophagus; a tubulovillous adenoma of the colon; a malignant mixed Mullerian tumour of the endometrium; a clear cell carcinoma of the kidney; a lung cancer (large cell undifferentiated, giant cell carcinoma, broncho-alveolar carcinoma, metastatic leiomyosarcoma); an ovarian cancer (a Brenner tumour, cystadenocarcinoma, solid teratoma); a cancer of the testis (seminoma, mature cystic teratoma); a soft tissue fibrosarcoma; a teratoma (anaplastic germ cell tumours); or a trophoblast cancer (choriocarcimoma (e.g. in uterus, lung or brain), tumour of placental site, hydatidiform mole).

20. The method according to any one of the preceding paragraphs. wherein the cancer is renal or colorectal cancer.

21. The method according to any one of the preceding paragraphs. wherein the immunotherapy comprises use of 5T4.

22. The method according to paragraph. 21 wherein the immunotherapy comprises use of a viral vector expressing 5T4.

23. The method according to paragraph. 22 wherein the immunotherapy comprises use of a Modified Vaccinia Ankara viral vector expressing 5T4.

24. The method according to paragraph.23 wherein the immunotherapy comprises use of a Modified Vaccinia Ankara viral vector expressing the human 5T4 gene under regulatory control of a modified mH5 promoter (TroVax®).

**Claims**

1. A method for determining a prognosis for benefit for a cancer patient receiving immunotherapy treatment, wherein the immunotherapy treatment comprises use of 5T4, involving (a) measuring a level of platelets and haemoglobin in a sample from the cancer patient, and (b) comparing the level of platelets in the sample to a reference level of platelets and comparing the level of haemoglobin in the sample to a reference level of haemoglobin, wherein a lower level of platelets and higher level of haemoglobin in the sample correlates with increased benefit to the patient.

2. A method of claim 1 wherein (a) further comprises measuring a level of monocytes in a sample from the cancer patient, and (b) further comprises comparing the level of monocytes in the sample to a reference level of monocytes, wherein a lower level of monocytes in the sample correlates with increased benefit to the patient.

3. A method of claim 1 or claim 2 wherein (a) further comprises measuring a level of white blood cells (WBCs) in a sample from the cancer patient, and (b) further comprises comparing the level of WBCs in the sample to a reference level of WBCs, wherein a lower level of WBCs in the sample correlates with increased benefit to the patient.

4. A method for determining a prognosis for benefit for a cancer patient receiving immunotherapy treatment, wherein the immunotherapy treatment comprises use of 5T4, involving (a) measuring a level of platelets and haemoglobin in a sample from the cancer patient, and (b) classifying the patient as belonging to either a first or second group of patients, wherein the first group of patients having lower levels of platelets than a reference level of platelets and higher levels of haemoglobin than a reference level of haemoglobin is classified as having an increased likelihood of benefit than the second group of patients having higher levels of platelets and lower levels of haemoglobin.

5. A method of claim 4 wherein (a) further comprises measuring a level of monocytes in a sample from the cancer patient and (b) further comprises classifying the patients where the first group of patients having lower levels of monocytes than a reference level of monocytes is classified as having an increased likelihood of benefit than the second group of patients having higher levels of monocytes.

6. A method of claim 4 or claim 5 wherein (a) further comprises measuring a level of WBCs in a sample from the cancer patient and (b) further comprises classifying the patients where the first group of patients having lower levels of WBCs than a reference level of WBCs is classified as having an increased likelihood of benefit than the second group of patients having higher levels of WBCs.

7. A method of predicting the responsiveness of a patient or patient population with cancer to treatment with immunotherapy, or for selecting patients or patient populations that will respond to immunotherapy, comprising comparing the differential levels of platelets and differential levels of haemoglobin and wherein the immunotherapy treatment comprises use of 5T4.

8. A method of claim 7 further comprising comparing the differential levels of monocytes.

9. A method of claim 7 or claim 8 further comprising comparing the differential levels of WBCs.

10. A method of claim 1 for determining a prognosis for benefit for a cancer patient receiving immunotherapy treatment involving (a) measuring levels of platelets, and haemoglobin, and/or monocytes and/or WBCs and one or more biomarkers selected from the group consisting of: corrected calcium, creatinine, chloride, eosinophils, haematocrit, sodium, basophils, serum calcium, and neutrophils and (b) comparing the level of platelets, and haemoglobin, and/or monocytes and/or WBCs and the one or more selected biomarkers in the sample to a reference level of platelets, haemoglobin, monocytes and/or WBCs and the one or more selected biomarkers, wherein a low level of platelets, monocytes and/or WBCs, a high level of haemoglobin, and a high level of any of creatinine, chloride, eosinophils, haematocrit, sodium and/or a low level of any corrected calcium, basophils, serum calcium, and neutrophils in said sample correlates with increased benefit to said patient.

11. The method according to any one of the preceding claims wherein the cancer is invasive carcinoma of the Ampulla of Vater, breast, colon, endometrium, pancreas, or stomach; a squamous carcinoma of the bladder, cervix, lung or oesophagus; a tubulovillous adenoma of the colon; a malignant mixed Mullerian tumour of the endometrium; a clear cell carcinoma of the kidney; a lung cancer; an ovarian cancer; a cancer of the testis; a soft tissue fibrosarcoma; a teratoma; or a trophoblast cancer.

12. The method according to any one of the preceding claims wherein the cancer is renal, prostate, breast, ovarian or colorectal cancer.

13. The method according to any one of the preceding claims wherein the immunotherapy treatment comprises use of a poxvirus vector.

14. The method according to any one of the preceding claims wherein the immunotherapy treatment comprises use of a viral vector expressing 5T4.

15. The method according to claim 14 wherein the immunotherapy treatment comprises use of a Modified Vaccinia Ankara viral vector expressing 5T4.

16. The method according to claim 15 wherein the immunotherapy treatment comprises use of a Modified Vaccinia Ankara viral vector expressing the human 5T4 gene under regulatory control of a mH5 promoter.


**Patentansprüche**

1. Verfahren zur Bestimmung einer Prognose zugunsten eines Krebspatienten, der eine immuntherapeutische Behandlung erhält, worin die immuntherapeutische Behandlung die Verwendung von 5T4, umfasst, umfassend (a) Messung einer Konzentration von Thrombozyten und Hämoglobin in einer Probe des Krebspatienten, und (b) Vergleich der Konzentration von Thrombozyten in der Probe mit einer Referenzkonzentration von Thrombozyten und Vergleich der Konzentration von Hämoglobin in der Probe mit einer Referenzkonzentration von Hämoglobin, worin eine niedrigere Konzentration von Thrombozyten und eine höhere Konzentration von Hämoglobin in der Probe mit erhöhtem Nutzen für den Patienten korreliert.

2. Verfahren nach Anspruch 1, worin (a) ferner die Messung einer Konzentration von Monozyten in einer Probe des Krebspatienten umfasst, und (b) ferner den Vergleich der Konzentration von Monozyten in der Probe mit einer Referenzkonzentration von Monozyten umfasst, worin eine niedrigere Konzentration von Monozyten in der Probe mit erhöhtem Nutzen für den Patienten korreliert.

3. Verfahren nach Anspruch 1 oder Anspruch 2, worin (a) ferner die Messung einer Konzentration von Leukozyten (weißen Blutkörperchen - WBK) in einer Probe des Krebspatienten umfasst, und (b) ferner den Vergleich der Konzentration von Leukozyten in der Probe mit einer Referenzkonzentration von Leukozyten umfasst, worin eine niedrigere Konzentration von Leukozyten in der Probe mit erhöhtem Nutzen für den Patienten korreliert.

4. Verfahren zur Bestimmung einer Prognose zugunsten eines Krebspatienten, der eine immuntherapeutische Behandlung erhält, worin die immuntherapeutische Behandlung die Verwendung von 5T4, umfasst, umfassend (a) Messung einer Konzentration von Thrombozyten und Hämoglobin in einer Probe des Krebspatienten, und (b) Klassifizierung des Patienten als Patient, der entweder zu einer ersten oder einer zweiten Gruppe von Patienten gehört, worin die erste Gruppe von Patienten, die niedrigere Konzentrationen von Thrombozyten als eine Referenzkonzentration von Thrombozyten und höhere Konzentrationen von Hämoglobin als eine Referenzkonzentration von Hämoglobin aufweist, als Gruppe klassifiziert wird, die mit höherer Wahrscheinlichkeit profitiert als die zweite Gruppe von Patienten, die höhere Konzentrationen von Thrombozyten und niedrigere Hämoglobinkonzentrationen aufweist.

5. Verfahren nach Anspruch 4, worin (a) ferner die Messung einer Konzentration von Monozyten in einer Probe des Krebspatienten umfasst, und (b) ferner die Klassifizierung der Patienten umfasst, worin die erste Gruppe von Patienten, die niedrigere Konzentrationen von Monozyten als eine Referenzkonzentration von Monozyten aufweist, als Gruppe klassifiziert wird, die mit höherer Wahrscheinlichkeit profitiert als die zweite Gruppe von Patienten, die höhere Konzentrationen von Monozyten aufweist.

6. Verfahren nach Anspruch 4 oder Anspruch 5, worin (a) ferner die Messung einer Konzentration von Leukozyten in einer Probe des Krebspatienten umfasst, und (b) ferner die Klassifizierung der Patienten umfasst, worin die erste Gruppe von Patienten, die niedrigere Konzentrationen von Leukozyten als eine Referenzkonzentration von Leukozyten aufweist, als Gruppe klassifiziert wird, die mit höherer Wahrscheinlichkeit profitiert als die zweite Gruppe von Patienten, die höhere Konzentrationen von Leukozyten aufweist.

7. Verfahren zur Vorhersage des Ansprechens eines Patienten oder einer Patientenpopulation mit Krebs auf eine immuntherapeutische Behandlung, oder zur Auswahl von Patienten oder Patientenpopulationen, die auf Immuntherapie ansprechen, umfassend den Vergleich der differenziellen Konzentrationen von Thrombozyten und der differenziellen Konzentrationen von Hämoglobin, und worin die immuntherapeutische Behandlung die Verwendung von 5T4 umfasst.

8. Verfahren nach Anspruch 7, ferner umfassend den Vergleich der differenziellen Konzentrationen von Monozyten.

9. Verfahren nach Anspruch 7 oder Anspruch 8, ferner umfassend den Vergleich der differenziellen Konzentrationen von Leukozyten.

10. Verfahren nach Anspruch 1 zur Bestimmung einer Prognose zugunsten eines Krebspatienten, der eine immuntherapeutische Behandlung erhält, umfassend (a) Messung von Konzentrationen von Thrombozyten, und Hämoglobin, und/oder Monozyten und/oder Leukozyten und einem oder mehreren Biomarkern, ausgewählt aus der Gruppe, die besteht aus: korrigiertem Calcium, Creatinin, Chlorid, Eosinophilen, Hämatokrit, Natrium, Basophilen, Serumcalcium und Neutrophilen, und (b) Vergleich der Konzentration von Thrombozyten, und Hämoglobin, und/oder Monozyten und/oder Leukozyten und dem einen oder den mehreren ausgewählten Biomarkern in der Probe mit einer Referenzkonzentration von Thrombozyten, Hämoglobin, Monozyten und/oder Leukozyten und dem einen oder den mehreren ausgewählten Biomarkern, worin eine niedrige Konzentration von Thrombozyten, Monozyten und/oder Leukozyten, eine hohe Konzentration von Hämoglobin, und eine hohe Konzentration eines beliebigen von Creatinin, Chlorid, Eosinophilen, Hämatokrit, Natrium und/oder eine niedrige Konzentration eines beliebigen von korrigiertem Calcium, Basophilen, Serumcalcium und Neutrophilen in der Probe mit erhöhtem Nutzen für den Patienten korreliert.

11. Verfahren nach einem der vorhergehenden Ansprüche, worin der Krebs ein invasives Karzinom der Vater-Ampulle, der Brust, des Kolons, des Endometriums, der Bauchspeicheldrüse oder des Magens; ein Plattenepithelkarzinom der Harnblase, des Gebärmutterhalses, der Lunge oder der Speiseröhre; ein tubulovillöses Adenom des Kolons; ein maligner Müllerscher Mischtumor des Endometriums; ein Klarzellkarzinom der Niere; ein Lungenkrebs; ein Ovarialkrebs; ein Hodenkrebs; ein Weichteilfibrosarkom; ein Teratom; oder ein Trophoblasttumor ist.

12. Verfahren nach einem der vorhergehenden Ansprüche, worin der Krebs Nieren-, Prostata-, Brust-, Ovarial- oder Kolorektalkrebs ist.

13. Verfahren nach einem der vorhergehenden Ansprüche, worin die immuntherapeutische Behandlung die Verwendung eines Pockenvirus-Vektors umfasst.

14. Verfahren nach einem der vorhergehenden Ansprüche, worin die immuntherapeutische Behandlung die Verwendung eines 5T4 exprimierenden viralen Vektors umfasst.

15. Verfahren nach Anspruch 14, worin die immuntherapeutische Behandlung die Verwendung eines 5T4 exprimierenden viralen Vektors des Modified-Vaccinia-Ankara-Virus umfasst.

16. Verfahren nach Anspruch 15, worin die immuntherapeutische Behandlung die Verwendung eines 5T4 exprimierenden viralen Vektors des Modified-Vaccinia-Ankara-Virus unter regulatorischer Kontrolle eines mH5-Promoters umfasst.

**Revendications**

1. Procédé de détermination d'un pronostic de bénéfices pour un patient cancéreux recevant un traitement d'immunothérapie, le traitement d'immunothérapie comprenant l'utilisation de 5T4, mettant en ouvre (a) la mesure d'un taux de plaquettes et d'hémoglobine dans un échantillon du patient cancéreux, et (b) la comparaison du taux de plaquettes dans l'échantillon à un taux de référence de plaquettes la comparaison du taux d'hémoglobine dans l'échantillon à un taux de référence d'hémoglobine, dans lequel un taux inférieur de plaquettes et un taux supérieur d'hémoglobine dans l'échantillon est corrélé à un bénéfice accru pour le patient.

2. Procédé de la revendication 1 dans lequel (a) comprend en outre la masure d'un taux de monocytes dans un échantillon du patient cancéreux, et (b) comprend en outre la comparaison du taux de monocytes dans l'échantillon à un taux de référence de monocytes, dans lequel un taux inférieur de monocytes dans l'échantillon est corrélé à un bénéfice accru pour le patient.

3. Procédé de la revendication 1 ou la revendication 2 dans lequel (a) comprend en outre la mesure d'un taux de leucocytes dans un échantillon du patient cancéreux, et (b) comprend en outre la comparaison du taux de leucocytes dans l'échantillon à un taux de référence de leucocytes, un taux inférieur de leucocytes dans l'échantillon étant corrélé à un bénéfice accru pour le patient.

4. Procédé de détermination d'un pronostic de bénéfices pour un patient cancéreux recevant un traitement d'immunothérapie, le traitement d'immunothérapie comprenant l'utilisation de 5T4, mettant en oeuvre (a) la mesure d'un taux de plaquettes et d'hémoglobine dans un échantillon du patient cancéreux, et (b) la classification du patient comme appartenant à un premier ou deuxième groupe de patients, où le premier groupe de patients ayant des taux de plaquettes plus faibles qu'un taux de référence de plaquettes et des taux d'hémoglobine plus élevés qu'un taux de référence d'hémoglobine est classé comme ayant une probabilité accrue de bénéfice par rapport au deuxième groupe de patients ayant des taux de plaquettes plus élevées et des taux d'hémoglobine moins élevés.

5. Procédé de la revendication 4 dans lequel (a) comprend en outre la mesure d'un taux de monocytes dans un échantillon du patient cancéreux et (b) comprend en outre la classification des patients où le premier groupe de patients ayant des taux de monocytes inférieurs à un taux de référence de monocytes est classé comme ayant une probabilité accrue de bénéfice par rapport au deuxième groupe de patients ayant des taux de monocytes plus élevés.

6. Procédé de la revendication 4 ou la revendication 5 dans lequel (a) comprend en outre la mesure d'un taux de leucocytes dans un échantillon du patient cancéreux et (b) comprend en outre la classification des patients où le premier groupe de patients ayant des taux de leucocytes inférieurs à un taux de référence de leucocytes est classé comme ayant une probabilité de bénéfice plus élevé que le deuxième coup de patients ayant des taux de leucocytes plus élevés.

7. Procédé de prédiction de la réponse d'un patient ou d'une population de patients atteints de cancer à un traitement par immunothérapie, ou pour sélectionner des patients ou des populations de patients qui répondent à une immunothérapie, comprenant la comparaison des taux différentiels de plaquettes et des taux différentiels d'hémoglobine et le traitement d'immunothérapie comprenant l'utilisation de 5T4.

8. Procédé de la revendication 7 comprenant en outre la comparaison des taux différentiels de monocytes.

9. Procédé de la revendication 7 ou la revendication 8 comprenant en outre la comparaison des taux différentiels de leucocytes.

10. Procédé de la revendication 1 pour déterminer un pronostic de bénéfice pour un patient cancéreux recevant un traitement d'immunothérapie mettant en oeuvre (a) la mesure de taux de plaquettes, et d'hémoglobine, et/ou de monocytes et/ou de leucocytes et d'un ou plusieurs biomarqueurs choisi dans le groupe constitué de : calcium corrigé, créatinine, chlorure, éosinophiles, hématocrite, sodium, basophiles, calcium sérique, et neutrophiles et (b) comparaison du taux de plaquettes, et d'hémoglobine, et/ou de monocytes et/ou de leucocytes et des un ou plusieurs biomarqueurs à un taux de référence de plaquettes, hémoglobine, monocytes et/ou leucocytes et des un ou plusieurs biomarqueurs sélectionnés, où un taux faible de plaquettes, monocytes et/ou leucocytes, un taux élevé d'hémoglobine, et un taux élevé de l'un quelconque des créatinine, chlorure, éosinophiles, hématocrite, sodium et/ou un taux faible de l'un quelconque des calcium corrigé, basophiles, calcium sérique et neutrophiles dans ledit échantillon est corrélé à un bénéfice accru pour ledit patient.

11. Procédé selon l'une quelconque des revendications précédentes dans lequel le cancer est un carcinome invasif de l'ampoule de Vater, du sein, du côlon, de l'endomètre, du pancréas ou de l'estomac ; un carcinome à cellules squameuses de la vessie, du col de l'utérus, du poumon ou de l'oesophage ; un adénome tubulovilleux du côlon ; une tumeur maligne mullérienne mixte de l'endomètre ; un carcinome à cellules claires du rein ; un cancer du poumon ; un cancer de l'ovaire ; un cancer des testicules ; un fibrosarcome des tissus mous ; un tératome ; ou un cancer trophoblastique.

**12.** Procédé selon l'une quelconque des revendications précédentes dans lequel le cancer est un cancer rénal, de la prostate, du sein, de l'ovaire ou colo-rectal.

**13.** Procédé selon l'une quelconque des revendications précédentes dans lequel le traitement d'immunothérapie comprend l'utilisation d'un vecteur à poxvirus.

**14.** Procédé selon l'une quelconque des revendications précédentes dans lequel le traitement d'immunothérapie comprend l'utilisation d'un vecteur viral exprimant 5T4.

**15.** Procédé selon la revendication 14 dans lequel le traitement d'immunothérapie comprend l'utilisation d'un vecteur à virus de la vaccine Ankara modifié exprimant 5T4.

**16.** Procédé selon la revendication 15 dans lequel le traitement d'immunothérapie comprend l'utilisation d'un vecteur à virus de la vaccine Ankara modifié exprimant le gène 5T4 humain sous le contrôle régulateur d'un promoteur mH5.

# Fig 1: TRIST Survival Analysis*
## Effect of Baseline Corrected Calcium on Patient Survival

**Baseline Corrected Calcium ≤ Median (≤ 9.3mg/dL)**

**Baseline Corrected Calcium >Median (>9.3mg/dL)**

|  | ≤Median Corrected Ca (Baseline) | |
|---|---|---|
|  | TroVax | Placebo |
| Sample size | 183 | 186 |
| Median OS | 562 | NR |
| Log-Rank | 0.24 | |

|  | >Median Corrected Ca ( Baseline) | |
|---|---|---|
|  | TroVax | Placebo |
| Sample size | 177 | 177 |
| Median OS | 308 | 476 |
| Log-Rank | 0.006 | |

bioMedica

* Survival Cut-Off August 11th 2008

# Fig 2: TRIST Survival Analysis*

## Effect of Baseline Corrected Calcium on Patient Survival

EP 2 386 062 B1

* Survival Cut-Off August 11th 2008

bioMedica

• Corrected Calcium levels in TRIST patients range from 6.8 to 15.4 mg/dL.
• Median Corrected Calcium levels in TRIST was 9.3 mg/dL

— = TroVax
— = Placebo

# Fig 3: TRIST Survival Analysis
## Effect of Baseline Corrected Calcium on Patient Survival

Corrected Calcium ≤8.8mg/dL

TroVax =39
Placebo = 38
P = 0.058

Corrected Calcium ≤8.7mg/dL

TroVax =25
Placebo = 21
P = 0.005

Corrected Calcium ≤8.6mg/dL

TroVax =15
Placebo = 14
P = 0.02

* Survival Cut-Off August 11th 2008
• Corrected Calcium levels in TRIST patients range from 6.8 to 15.4 mg/dL.
• Median Corrected Calcium levels in TRIST was 9.3 mg/dL

bioMedica

— = TroVax
— = Placebo

EP 2 386 062 B1

# Fig 4: TRIST Survival Analysis*
## Effect of Baseline Platelets on Patient Survival

**Platelets: > Median (287 x 10⁹ /L) at Baseline**

**Platelets: ≤ Median (287 x 10⁹ /L) at Baseline**

|  | >Median Platelets at Baseline | |
|---|---|---|
|  | TroVax | Placebo |
| Sample size | 184 | 175 |
| Median OS | 303 | 476 |
| Log-Rank | 0.016 | |

|  | ≤Median Platelets at Baseline | |
|---|---|---|
|  | TroVax | Placebo |
| Sample size | 175 | 185 |
| Median OS | 562 | NR |
| Log-Rank | 0.38 | |

bioMedica

* Survival Cut-Off August 11th 2008

EP 2 386 062 B1

# Fig 5: TRIST Survival Analysis
## Effect of Baseline Platelets on Patient Survival

Platelets ≤ 500 x 10⁹/L — TroVax = 328, Placebo = 324

Platelets ≤ 400 x 10⁹/L — TroVax = 287, Placebo = 283

Platelets ≤ 300 x 10⁹/L — TroVax = 195, Placebo = 199

Platelets ≤ 250 x 10⁹/L — TroVax = 119, Placebo = 122, P = 0.1

Platelets ≤ 225 x 10⁹/L — TroVax = 82, Placebo = 75, P = 0.046

Platelets ≤ 215 x 10⁹/L — TroVax = 68, Placebo = 58, P = 0.015

bioMedica

Platelet levels in TRIST patients range from 114 to 1074 x 10⁹ /L
Median Platelet levels in TRIST patients are 281 x 10⁹ /L
Normal Platelet levels are quoted as 130 – 400 x 10⁹ /L

— = TroVax
— = Placebo

EP 2 386 062 B1

# Fig 6: TRIST Survival Analysis*

## Effect of Haemoglobin on Survival of TroVax and Placebo-Treated Patients

**All Patients**

**>100g/dL Haemoglobin**

TroVax = 340
Placebo = 336

**>125g/dL Haemoglobin**

TroVax = 256
Placebo = 267

**>130g/dL Haemoglobin**

TroVax = 205
Placebo = 206

**>140g/dL Haemoglobin**

TroVax = 130
Placebo = 124
P=0.1

**>145g/dL Haemoglobin**

TroVax = 96
Placebo = 90
P=0.06

\* Survival Data to December 2008

bioMedica

Haemoglobin levels in TRIST patients range from 65 to 190g/dL
Median Haemoglobin levels in TRIST patients are 132g/dL
Normal Haemoglobin levels are quoted as approximately 118 to 168g/dL

— = TroVax
— = Placebo

EP 2 386 062 B1

# Fig 7: TRIST Survival Analysis*

## Effect of Baseline Creatinine on Survival of TroVax and Placebo-Treated Patients

Creatinine ≥ 90 µmol/L — TroVax = 305, Placebo = 310

Creatinine ≥ 100 µmol/L — TroVax = 250, Placebo = 255

Creatinine ≥ 110 µmol/L — TroVax = 139, Placebo = 200

Creatinine ≥ 120 µmol/L — TroVax = 127, Placebo = 133

Creatinine ≥ 130 µmol/L — TroVax = 84, Placebo = 84, P = 0.03

Creatinine ≥ 145 µmol/L — TroVax = 40, Placebo = 41, P = 0.027

* Survival Data censored at 11th August 2008

Creatinine levels in TRIST patients range from 58 to 268 µmol/L
Median Creatinine levels in TRIST patients are 111µmol/L
Normal Creatinine levels are quoted as 44 – 124 µmol /L

bioMedica

— = TroVax
— = Placebo

EP 2 386 062 B1

# Fig 8: TRIST Survival Analysis*

## Effect of Baseline Calcium, Creatinine, Haemoglobin and Platelets on Patient Survival

| Group | TroVax | Placebo |
|---|---|---|
| Number | 185 | 198 |
| Median OS | 562 | NR |
| Log-Rank | 0.005 | |

| Baseline Variable | Sub-Set Thresholds |
|---|---|
| Corrected Calcium | ≤9.1mg/dL |
| Creatinine | ≥145µmol/L |
| Haemoglobin | ≥153g/dL |
| Platelets | ≤232GI/L |

**— = TroVax**
**— = Placebo**

* Survival Data censored at 11th August 2008

bioMedica

EP 2 386 062 B1

# Fig 9: TRIST Survival Analysis*

## Effect of Baseline Calcium, Creatinine, Haemoglobin and Platelets on Patient Survival

### Zero Baseline Inclusion Factors

| Group | TroVax | Placebo |
|---|---|---|
| Number | 180 | 170 |
| Median OS | 298 | NR |
| Log-Rank | <0.0001 | |

### ≥1 Baseline Inclusion Factor(s)

| Group | TroVax | Placebo |
|---|---|---|
| Number | 185 | 198 |
| Median OS | 562 | NR |
| Log-Rank | 0.005 | |

bioMedica

* Survival Data censored at 11th August 2008

EP 2 386 062 B1

# Fig 10: TRIST Survival Analysis*

## Effect of Baseline Calcium, Creatinine, Haemoglobin and Platelets on Patient Survival

Survival probability (%) vs Time (Days)
- = TroVax
- = Placebo

| "Favourable" Baseline Variables | Placebo | TroVax |
|---|---|---|
| One | 135 | 112 |
| Two | 38 | 45 |
| Three | 10 | 15 |
| Four | 1 | 1 |

| "Favourable" Baseline Variables | Placebo (Nos) | TroVax (Nos) |
|---|---|---|
| Ca | 65 | 48 |
| Cr | 19 | 17 |
| Hb | 11 | 13 |
| Pl | 40 | 34 |
| Ca / Cr | 10 | 5 |
| Ca / Hb | 3 | 7 |
| Ca / Pl | 23 | 31 |
| Cr / Hb | 2 | 2 |
| Cr / Pl | 2 | 7 |
| Hb / Pl | 12 | 5 |
| Ca /Cr / Hb | 1 | 0 |
| Ca / Cr / Pl | 4 | 4 |
| Ca / Hb / Pl | 4 | 10 |
| Cr / Hb / Pl | 1 | 1 |
| Ca /Cr / Hb / Pl | 1 | 1 |

bioMedica

* Survival Data censored at 11th August 2008

Key: Ca = Corrected Calcium; Cr = Creatinine; Hb = Hamoglobin; Pl = Platelets

# Fig 11: Phase II Survival Analysis (CRC Patients;4 Trials) Effect of Baseline Platelets on Patient Survival

| Group | >Median >275.5 x $10^9$/L | ≤ Median ≤275.5 x $10^9$/L |
|---|---|---|
| Number | 36 | 36 |
| Median OS | 14.5 | 28.4 |
| Log-Rank | 0.0019 | |

Legend (on graph):
— = >Median
— = ≤ Median

Axis labels: Survival probability (%) vs Time (Days)

bioMedica

Median Platelet levels in Phase II CRC patients are 275.5 x $10^9$ /L
Median Platelet levels in TRIST patients are 281 x $10^9$ /L
Normal Platelet levels are quoted as 130 – 400 x $10^9$ /L

EP 2 386 062 B1

# Fig 12: Phase II Survival Analysis (CRC Patients; 4 trials) Effect of Baseline Platelets on Patient Survival

| Group | >232 x 10$^9$/L | ≤ 232 x 10$^9$/L |
|---|---|---|
| Number | 51 | 22 |
| Median OS | 15.6 | 32.3 |
| Log-Rank | 0.034 | |

Graph legend: = >232x10$^9$/L ; = ≤ 232x10$^9$/L

Y-axis: Survival probability (%)
X-axis: Time (Months)

bioMedica

Median Platelet levels in Phase II CRC patients are 275.5 x 10$^9$ /L
Median Platelet levels in TRIST patients are 281 x 10$^9$ /L
Normal Platelet levels are quoted as 130 – 400 x 10$^9$ /L

EP 2 386 062 B1

# Fig 13: Phase II Survival Analysis (4 x CRC, 2 x RCC Trials) Effect of Baseline Platelets on Patient Survival

|  | >Median | ≤Median |
|---|---|---|
| Sample size | 51 | 51 |
| Median OS | 15.8 | 32.3 |
| Log-Rank | 0.0012 | |

Median Platelet levels in Phase II CRC and RCC patients are 273 x $10^9$ /L
Median Platelet levels in TRIST patients are 281 x $10^9$ /L
Normal Platelet levels are quoted as 130 – 400 x $10^9$ /L

bioMedics

EP 2 386 062 B1

# Fig 14: TRIST Exploratory Analyses

## Influence of Baseline Platelet Levels on Patient Survival

**Above Normal Range**  —  **Normal Range**  —  **Lower Normal Range**

- Lower baseline platelet levels are associated with improved survival in favour of MVA-5T4 vs placebo

# Fig 15: Phase II Survival Analysis (CRC Patients; 4 trials) Effect of Baseline Haemoglobin on Patient Survival

| Group | >Median (>129g/dL) | ≤ Median (≤129g/dL) |
|---|---|---|
| Number | 35 | 37 |
| Median OS | 23 | 15.6 |
| Log-Rank | 0.058 | |

— = >Median
— = ≤ Median

Survival probability (%)

Time (Months)

bioMedics

Median Haemoglobin levels in Phase II CRC patients are 129g/dL
Median Haemoglobin levels in TRIST patients are 132g/dL
Normal Haemoglobin levels are quoted as approximately 118 to 168g/dL

EP 2 386 062 B1

# Fig 16:Phase II Survival Analysis (CRC Patients; 4 trials)
## Effect of Baseline Creatinine on Patient Survival

| Group | >Median (>90µmol/L) | ≤ Median (≤90 µmol/L) |
|---|---|---|
| Number | 36 | 37 |
| Median OS | 17.6 | 17.9 |
| Log-Rank | 0.14 | |

Legend:
— = >Median
— = ≤ Median

Median Creatinine levels in Phase II CRC patients are 90 µmol/L
Median Creatinine levels in TRIST patients are 111µmol/L
Normal Creatinine levels are quoted as approximately 44 to 124µmol/L

bioMedica

EP 2 386 062 B1

# Fig 17:TRIST Exploratory Analyses
## Influence of Baseline Monocyte Levels on Patient Survival

**Above Normal Range**

— = MVA-5T4
— = Placebo

Survival probability (%) — Time (Days)

**Normal Range**

— = MVA-5T4
— = Placebo

Survival probability (%) — Time (Days)

**Lower Normal Range**

— = MVA-5T4
— = Placebo

Survival probability (%) — Time (Days)

- Lower baseline monocyte levels are associated with improved survival in favour of MVA-5T4 vs placebo

bioMedica

# Fig 18: TRIST Exploratory Analyses
## Patients with Normal Haematology Show Survival Benefit Trend

**All Patients (n=703)**

**Patients with Normal Haematology\* (n=368)**
\* Normal levels of monocytes, platelets and haemoglobin

Adjusted Hazard Ratio = 0.96
(95% CI 0.77, 1.21; p = 0.731)

Adjusted Hazard Ratio = 0.73
(95%CI 0.49, 1.09; p = 0.129)

- "Normal" baseline levels of monocytes, platelets and haemoglobin associated with reduced hazard ratio in favour of MVA-5T4

bioMedica

FIGURE 19

FIGURE 20

FIGURE 21

FIGURE 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7148035 B

- WO 64027005400 A

**Non-patent literature cited in the description**

- **CHOUERIRI et al.** *Cancer,* 2007, vol. 110 (3), 543-550
- **FUSEK et al.** *World J Gastroenterol,* 2004, vol. 10 (13), 1890-1892
- **PADRIK, P. et al.** *Med. Oncol.,* 2003, vol. 20, 325-34
- Cancer Vaccines and immunotherapy. Cambridge University Press, 2000
- Cancer Immunology. Kluwer Academic Publishers, 2001
- Basic Cancer Medicine. **MARKMAN.** Saunders. 1997
- **MOTZER.** Prognostic factors for survival of patients with stage IV renal cell carcinoma: Memorial Sloan-Kettering Cancer Center experience. *Clin Cancer Res,* 2004, vol. 10, 6302S-3S
- **NEGRIER et al.** Recombinant human interleukin-2, recombinant human interferon alfa-2a, or both, in metastatic renal-cell carcinoma. *N Engl J Med,* 1998, vol. 338, 1272-8
- **LAN KKG ; DEMETS DL.** Discrete sequential boundaries for clinical trials. *Biometrika,* 1983, vol. 70, 659-663

- **LI G ; SHIH WJ ; XIE T ; LU J.** A sample size adjustment procedure for clinical trials based on conditional power. *Biostatistics,* 2002, vol. 3, 277-287
- **HARROP R ; CONNOLLY N ; REDCHENKO I ; VALLE J ; SAUNDERS M ; RYAN MG ; MYERS KA ; DRURY N ; KINGSMAN SM ; HAWKINS RE.** *Clin Cancer Res.,* 01 June 2006, vol. 12, 3416-24
- **ELKORD E ; DANGOOR A ; DRURY NL ; HARROP R ; BURT DJ ; DRIJFHOUT JW ; HAMER C ; ANDREWS D ; NAYLOR S ; SHERLOCK D.** *J Immunother,* November 2008, vol. 31 (9), 820-9
- **HARROP R ; DRURY N ; SHINGLER VV ; CHIKOTI P ; REDCHENKO I ; CARROLL MW ; KINGSMAN SM ; NAYLOR S ; GRIFFITHS R ; STEVEN N.** *Cancer Immunol Immunother,* July 2008, vol. 57 (7), 977-86
- **HARROP R ; DRURY N ; SHINGLER VV ; CHIKOTI P ; REDCHENKO I ; CARROLL MW ; KINGSMAN SM ; NAYLOR S ; MELCHER A ; NICHOLLS J.** *Clin Cancer Res.,* 01 August 2007, vol. 13, 4487-94